# EUROPEAN PATENT APPLICATION

(11) **EP 4 741 415 A2**
(43) Date of publication of application: **13.05.2026**
(21) Application number: 25224005.6
(22) Date of filing: 16.07.2021
(51) Int. Cl.: C07K 14/19

(54) **CHIMERIC MYD88 RECEPTORS FOR REDIRECTING IMMUNOSUPPRESSIVE SIGNALING AND RELATED COMPOSITIONS AND METHODS**

(30) Priority: 17.07.2020 US 202063053529 P
(62) Divisional of application: 21752401.6
(71) Applicant: Simurx, Inc., Pasadena, CA 91107 (US); Children's Hospital Los Angeles, Los Angeles, CA 90027-0034 (US)
(72) Inventor: ASGHARZADEH, Shahab, Pasadena, 91107 (US); MOGHIMI, Babak, Pasadena, 91107 (US); HADJIDANIEL, Michael, Pasadena, 91107 (US); SHIRINBAK, Soheila, Pasadena, 91107 (US)
(74) Representative: Carpmaels & Ransford LLP

(57) **Abstract**

The present disclosure relates in some aspects to chimeric signaling receptors containing an extracellular domain capable of binding a molecule, such as an immunosuppressive cytokine, and a MyD88-containing intracellular domain capable of engaging a signaling pathway to activate an immune cell. In some aspects, the disclosure further relates to engineered cells, such as T cells, and compositions comprising the chimeric signaling receptors or engineered cells, and methods and uses thereof. In some embodiments, the cells may further express a genetically engineered recombinant antigen receptor directed against an antigen, such as a chimeric antigen receptor (CAR) or recombinant T cell receptor (TCR) or, in some cases, secrete a recombinant molecule, for example, a bispecific antibody.

## Description

### Cross-Reference to Related Applications

This application claims priority from U.S. provisional application No. 63/053,529, filed July 17, 2020, entitled "CHIMERIC MYD88 RECEPTORS FOR REDIRECTING IMMUNOSUPPRESSIVESIGNALING AND RELATED COMPOSITIONS AND METHODS," the contents of which are incorporated by reference in their entirety.

### Incorporation by Reference of Sequence Listing

The present application is being filed along with a Sequence Listing in electronic format. The Sequence Listing is provided as a file entitled 165282000140SeqList.txt, created July 10, 2021, which is 228,223 bytes in size. The information in the electronic format of the Sequence Listing is incorporated by reference in its entirety.

### Field

The present disclosure relates in some aspects to chimeric signaling receptors containing an extracellular domain capable of binding a molecule, such as an immunosuppressive cytokine, and a MyD88-containing intracellular domain capable of engaging a signaling pathway to activate an immune cell. In some aspects, the disclosure further relates to engineered cells, such as T cells, and compositions comprising the chimeric signaling receptors or engineered cells, and methods and uses thereof. In some embodiments, the cells may further express a genetically engineered recombinant antigen receptor directed against an antigen, such as a chimeric antigen receptor (CAR) or recombinant T cell receptor (TCR) or, in some cases, secrete a recombinant molecule, for example, a bispecific antibody.

### Background

Cell therapy strategies for treatment of diseases or conditions, such as cancers or tumors, include engineering immune cells to express genetically engineered recombinant antigen receptors, such as chimeric antigen receptors (CARs) or recombinant T cell receptors (TCRs), and administering compositions containing such cells to subjects. Improved strategies are needed to increase efficacy of the treatments, for example, by preventing and/or reversing immunosuppressive effects of cytokine signaling in tumor microenvironments, to improve the persistence, survival, and cytotoxicity of engineered cells. Provided are compositions, cells, and methods that meet such needs.

### Summary

Provided herein are chimeric signaling receptor constructs that are able to redirect immunosuppressive signals by inducing a positive signal and T cell activation in response to an immunosuppressive cytokine. In provided aspects, the immunosuppressive cytokine is TGFβ and the chimeric signaling receptor contains an extracellular domain of a TGFβ receptor (TGFβR) or a portion thereof that binds TGFβ and MyD88 signaling domain(s) to mediate the positive signal in the cells upon binding of TGFβ to the extracellular domain of the chimeric signaling receptor. Thus, the provided chimeric signaling receptor can provide a positive signal to T cells even when the cells are present in an immunosuppressive environment, such as the TME, thereby improving the T cell activity, proliferation, persistence, or survival.

In some embodiments, the chimeric receptor signaling receptor provided herein contains: (a) an extracellular domain of a TGFβ receptor (TGFβR) or a portion thereof that binds TGFβ; (b) a transmembrane domain; (c) a first truncated MyD88 polypeptide that lacks the full-length TIR domain of full-length MyD88; and (d) a second truncated MyD88 domain that lacks the full-length TIR domain of full-length MyD88. In some embodiments, the TGFβR is a TGFβR2.

In some embodiments, the extracellular domain or the portion thereof contains: (i) the sequence of amino acids set forth in SEQ ID NO: 20; (ii) a sequence of amino acids that exhibits at least or about 85%, at least or about 90%, at least or about 92%, at least or about 95%, at least or about 97% sequence identity to the sequence set forth in SEQ ID NO: 20; or (iii) a portion of (i) or (ii) that binds TGFβ. In some embodiments, the extracellular domain or the portion thereof is set forth in SEQ ID NO:20.

In some embodiments, the transmembrane domain contains the native transmembrane domain of the TGFβR. In some embodiments, the transmembrane domain contains the sequence of amino acids set forth in SEQ ID NO: 22 or a sequence of amino acids that exhibits at least or about 85%, at least or about 90%, at least or about 92%, at least or about 95%, at least or about 97% sequence identity to the sequence set forth in SEQ ID NO: 22. In some embodiments, the transmembrane domain is set forth in SEQ ID NO:22.

In some embodiments, the transmembrane domain is a heterologous transmembrane domain from a transmembrane protein other than the TGFβR.

In some embodiments, the transmembrane domain and the first truncated MyD88 polypeptide are directly linked. In some embodiments, the transmembrane domain and the first truncated MyD88 polypeptide are indirectly linked by a linker. In some embodiments, the linker is or comprises a peptide linker. In some embodiments, the linker is or contains a partial sequence of N-terminal contiguous amino acids of the cytoplasmic domain of the TGFβR that is a non-functional portion, wherein the non-functional portion is not a functional inhibitory signaling domain capable of mediating inhibitory signaling. In some embodiments, the intracellular (or cytoplasmic) domain that serves as a linker between the transmembrane domain and the first truncated MyD88 polypeptide is not capable of recruiting an inhibitory adaptor molecule. In some embodiments, the intracellular (or cytoplasmic) domain that serves as a linker between the transmembrane domain and the first truncated MyD88 polypeptide is not capable of recruiting a SMAD. In some embodiments, the linker is amino acids 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, or 1-25 of the TGFβR2 cytoplasmic domain set forth in SEQ ID NO:129. In some embodiments, the linker is set forth by SEQ ID NO: 24.

Provided herein are chimeric signaling receptor containing (i) a portion of a TGFβ receptor (TGFβR) containing the extracellular domain and the transmembrane domain of a TGFβR, wherein the portion is less than the full-length TGFβR and lacks a functional inhibitory signaling domain of the full-length TGFβR; and (ii) an intracellular domain containing a first truncated MyD88 polypeptide that lacks the full-length TIR domain of full-length MyD88, and a second truncated MyD88 domain that lacks the full-length TIR domain of full-length MyD88. In some embodiments, the TGFβR is a TGFβR2. In some embodiments, the portion of the TGFβR contains the sequence of amino acids set forth in SEQ ID NO: 3 or a sequence of amino acids that exhibits at least or about 85%, at least or about 90%, at least or about 92%, at least or about 95%, at least or about 97% sequence identity to the sequence set forth in SEQ ID NO: 3. In some embodiments, the portion of the TGFβR is set forth in SEQ ID NO:3. In some embodiments, the portion of the TGFβR and the intracellular domain are directly linked. In some embodiments, the portion of the TGFβR and the intracellular domain are indirectly linked by a peptide linker. In some embodiments, the first truncated MyD88 polypeptide and the second MyD88 polypeptide each contain the death domain (DD), the intermediate domain (ID) and a portion of the full-length TIR domain of MyD88.

In some embodiments, the first truncated MyD88 polypeptide and the second MyD88 polypeptide each independently is a sequence of amino acids selected from the group consisting of amino acids 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179 or 2-180 of the full-length MyD88. In some embodiments, the first truncated MyD88 polypeptide and the second MyD88 polypeptide each independently is the sequence set forth by SEQ ID NO:128. In some embodiments, the first truncated MyD88 polypeptide and the second truncated polypeptide each independently is a sequence of amino acids 2-171 or 2-172 of the full-length MyD88, optionally of SEQ ID NO:128. In some embodiments, the first truncated MyD88 polypeptide is set forth in SEQ ID NO: 2. In some embodiments, the second truncated MyD88 polypeptide is set forth in SEQ ID NO: 2. In some embodiments, the first truncated MyD88 polypeptide and the second truncated MyD88 polypeptide are the same.

In some embodiments, the first MyD88 polypeptide and the second MyD88 polypeptide are connected by a peptide linker. In some embodiments, the peptide linker is (G4S)n (SEQ ID NO: 47), wherein n is an integer between 1 to 4, inclusive. In some embodiments, the peptide linker is set forth in SEQ ID NO: 47 ((GGGGS)n), where n is an integer between 1 and 4, inclusive, optionally wherein the peptide linker is selected from the group consisting of SEQ ID NO:48 (GGGGS), SEQ ID NO: 85 (GGGGSGGGGS), SEQ ID NO: 49 (GGGGSGGGGSGGGGS), and SEQ ID NO: 50 (GGGGSGGGGSGGGGSGGGGS). In some embodiments, the peptide linker is selected from the group consisting of SEQ ID NO:48 (GGGGS), SEQ ID NO: 85 (GGGGSGGGGS), SEQ ID NO: 49 (GGGGSGGGGSGGGGS), and SEQ ID NO: 50 (GGGGSGGGGSGGGGSGGGGS).

In some embodiments, the chimeric signaling receptor is set forth in SEQ ID NO:93 or a sequence of amino acids that exhibits at least at or about 85%, at least at or about 90%, at least at or about 92%, at least at or about 95%, or at least at or about 97% sequence identity to the sequence set forth in SEQ ID NO:93. In some embodiments, the chimeric signaling receptor is set forth in SEQ ID NO:93. In some embodiments, the chimeric signaling receptor is set forth in SEQ ID NO:94 or a sequence of amino acids that exhibits at least at or about 85%, at least at or about 90%, at least at or about 92%, at least at or about 95%, or at least at or about 97% sequence identity to the sequence set forth in SEQ ID NO:94. In some embodiments, the chimeric signaling receptor is set forth in SEQ ID NO:94.

In some embodiments, when the chimeric signaling receptor is expressed from a cell, binding of TGFβ to the extracellular domain induces dimerization of the first and second MyD88 polypeptide. In some embodiments, when the chimeric signaling receptor is expressed from a cell, binding of TGFβ to the extracellular domain recruits an IRAK4 to the first MyD88 polypeptide and the second MyD88 polypeptide. In some embodiments, when the chimeric signaling receptor is expressed from a cell, binding of TGFβ to the extracellular domain results in phosphorylation of IRAK4.

Provided herein are polynucleotides containing a sequence of nucleotides encoding a chimeric signaling receptor provided herein. In some embodiments, the sequence of nucleotides encoding the chimeric signaling receptor is a first sequence of nucleotides and the polynucleotide further comprises a second sequence of nucleotides encoding a recombinant molecule.

In some embodiments, the recombinant molecule is a recombinant antigen receptor. In some embodiments, the sequence of nucleotides encoding the chimeric signaling receptor is a first sequence of nucleotides and the polynucleotide further comprises a second sequence of nucleotides encoding a recombinant antigen receptor. In some embodiments, the recombinant antigen receptor is a recombinant T cell receptor (TCR) or is a chimeric antigen receptor (CAR). In some embodiments, the recombinant antigen receptor is a CAR. In some embodiments, the recombinant antigen receptor binds a tumor antigen. In some embodiments, the tumor antigen is a CD19. In some embodiments, the tumor antigen is a B7H3.

In some embodiments, the recombinant molecule is a secretable molecule, such as a bispecific antibody (e.g. BiTE) or a cytokine. In some embodiments, the sequence of nucleotides encoding the chimeric signaling receptor is a first sequence of nucleotides and the polynucleotide further comprises a second sequence of nucleotides encoding a recombinant secretable molecule. In some embodiments, the recombinant secretable molecule is a bispecific antibody, such as a bispecific T cell engager (BiTE). In some embodiments, the recombinant secretable molecule is a recombinant cytokine (e.g. IL-15 or IL-12).

In some embodiments, the first sequence of nucleotides encoding the chimeric signaling receptor and the second sequence of nucleotides are separated by a bicistronic element. In some embodiments, the bicistronic element is an IRES or is a cleavable peptide. In some embodiments, the cleavable peptide is selected from the group consisting of a P2A, a T2A and an F2A. In some embodiments, the cleavable peptide is a T2A. In some embodiments, the sequence(s) of nucleotides (e.g. the first and/or second sequence of nucleotides) is operably linked to a promoter. In some embodiments, the first sequence of nucleotides and the second sequence of nucleotides are each operably linked to a promoter. In some embodiments, the promoter is the same promoter. In some embodiments, the promoter is an EF1 promoter.

Also provided herein are vectors containing the polynucleotides provided herein. In some embodiments, the vector is a viral vector. In some embodiments, the viral vector is a retroviral vector. In some embodiments, the viral vector is a lentiviral vector.

Provided herein are methods of producing an engineered cell, the methods including introducing a polynucleotide provided herein or a vector provided herein into a cell under conditions for expression of the chimeric signaling receptor on the surface of the cell.

In some embodiments, the methods further include introducing a polynucleotide encoding a recombinant antigen receptor into the cell under conditions for expression of the recombinant antigen receptor on the surface of the cell. In some embodiments, the recombinant antigen receptor is a recombinant T cell receptor (TCR) or is a chimeric antigen receptor (CAR). In some embodiments, the recombinant antigen receptor is a CAR. In some embodiments, the recombinant antigen receptor binds a tumor antigen. In some embodiments, the tumor antigen is a CD19. In some embodiments, the tumor antigen is a B7H3.

In some embodiments, the methods further include introducing a polynucleotide encoding a recombinant molecule that is secretable, such as a bispecific antibody (e.g. BiTE) or cytokine, into the cell under conditions for expression and secretion of the recombinant molecule. In some embodiments, the methods further include introducing a polynucleotide encoding a bispecific antibody, such as a BiTE, into the cell under conditions for expression and, in some cases secretion, of the bispecific antibody (e.g. BiTE) from the cell. In some embodiments, the methods further include introducing a polynucleotide encoding a recombinant cytokine into the cell under conditions for expression and, in some cases secretion, of the cytokine (e.g. IL-15 or IL-12) from the cell. In some embodiments, the methods include introducing both a polynucleotide encoding a recombinant antigen receptor into the cell under conditions for expression of the recombinant antigen receptor on the surface of the cell and a polynucleotide encoding a recombinant molecule that is secretable, such as a cytokine or bispecific antibody (e.g. BiTE), into the cell under conditions for expression and, in some cases secretion, of the cytokine or bispecific antibody (e.g. BiTE) from the cell. Examples of bispecific antibodies, such as a BiTE include any as described herein. In some embodiments, the bispecific antibody is a BiTE that binds CD3 and GD2 (anti-CD3 and anti-GD2).

In some embodiments, the cell is a primary cell from a subject. In some embodiments, the cell is a T cell, a tumor-infiltrating cell (TIL), a B cell, a natural killer cell, or a macrophage. In some embodiments, the cell is a T cell, optionally a CD3+, CD4+, or CD8+ T cells. In some embodiments, the cell is a CD3+ T cell. In some embodiments, the cell is a CD8+ T cell. In some embodiments, the cell is a CD4+ T cell.

Provided herein is an engineered cell produced by a method provided herein.

Provided herein is an engineered cell containing a chimeric signaling receptor provided herein. In some embodiments, the engineered cell further comprises a recombinant antigen receptor. In some embodiments, the recombinant antigen receptor is a recombinant T cell receptor (TCR) or is a chimeric antigen receptor (CAR). In some embodiments, the recombinant antigen receptor binds a tumor antigen. In some embodiments, the tumor antigen is a CD19. In some embodiments, the tumor antigen is a B7H3. In some embodiments, the engineered cell further comprises a recombinant molecule that is secretable, such as a cytokine (e.g. IL-15 or IL-12) or a bispecific antibody (e.g. a BiTE). In some embodiments, the engineered cell further comprises a recombinant molecule that is secretable, such as a cytokine or bispecific antibody (e.g. BiTE), and a recombinant antigen receptor.

Provided herein is an engineered cell containing a polynucleotide provided herein. In some embodiments, the cell further contains a polynucleotide encoding a recombinant antigen receptor. In some embodiments, the recombinant antigen receptor is a recombinant T cell receptor (TCR) or is a chimeric antigen receptor (CAR). In some embodiments, the recombinant antigen receptor is a CAR. In some embodiments, the recombinant antigen receptor binds a tumor antigen. In some embodiments, the tumor antigen is a CD19. In some embodiments, the tumor antigen is a B7H3. In some embodiments, the engineered cell further comprises a polynucleotide encoding a recombinant molecule that is secretable, such as a cytokine (e.g. IL-15 or IL-12) or a bispecific antibody (e.g. a BiTE). In some embodiments, the engineered cell further comprises a polynucleotide encoding a a recombinant molecule that is secretable, such as a cytokine or bispecific antibody (e.g. BiTE), and a polynucleotide encoding a recombinant antigen receptor. The polynucleotides may be expressed as a single nucleic acid molecule, for example each separated by a bicistronic element or cleavable linker (e.g. T2A, PTA or F2A). Examples of bispecific antibodies, such as a BiTE include any as described herein. In some embodiments, the bispecific antibody is a BiTE that binds CD3 and GD2 (anti-CD3 and anti-GD2).

In some embodiments, the engineered cell is a primary cell from a subject. In some embodiments, the engineered cell is a T cell, a tumor infiltrating lymphocyte (TIL), a B cell, a natural killer (NK) cell, or a macrophage. In some embodiments, the engineered cell is a T cell, optionally a CD3+, CD4+, and/or CD8+ T cells. In some embodiments, the engineered cell is a CD3+ T cell. In some embodiments, the engineered cell is a CD8+ T cell. In some embodiments, the engineered cell is a CD4+ T cell.

Provided herein are pharmaceutical compositions containing the engineered cell provided herein. For instance, in some embodiments, the pharmaceutical composition contains a plurality of the engineered cell. In some embodiments, the pharmaceutical composition further includes a pharmaceutically acceptable excipient. In some embodiments, the pharmaceutical composition is sterile.

Provided herein are methods of treatment including administering an engineered cell provided herein or a pharmaceutical composition containing an engineered cell provided herein to a subject that has a cancer. The engineered cells express a provided chimeric signaling receptor. In some embodiments, the engineered cells additionally contain an antigen receptor targeted against an antigen of the cancer. In some embodiments, the engineered cells express a recombinant antigen receptor containing an antigen-binding domain that binds to a tumor antigen associated with the cancer. In some embodiments, the cells of the cancer express the tumor antigen. In some embodiments, the engineered cells additionally express, and in some cases are able to secrete, a recombinant molecule that is secretable, such as a cytokine (e.g. IL-15 or IL-12) or a bispecific antibody (e.g. a BiTE).

Also provided herein is use of any of the provided pharmaceutical compositions containing an engineered cell or the engineered cells for manufacture of a medicament for use in treating a subject that has a cancer. Also provided herein is any of the provided pharmaceutical compositions containing an engineered cell or the engineered cells for use in treating a subject that has a cancer. The engineered cells express a provided chimeric signaling receptor. In some of any embodiments, the engineered cell of the pharmaceutical composition also may comprise an antigen receptor targeted against an antigen of the cancer. In some embodiments, the engineered cells express a recombinant antigen receptor comprising an antigen-binding domain that binds to a tumor antigen associated with the cancer. In some embodiments, the engineered cells additionally express, and in some cases are able to secrete, a recombinant molecule that is secretable, such as a cytokine (e.g. IL-15 or IL-12) or a bispecific antibody (e.g. a BiTE).

In some embodiments, the recombinant antigen receptor is a chimeric antigen receptor (CAR) or a T cell receptor (TCR). In some embodiments, the recombinant antigen receptor is a CAR.

In some embodiments, the cancer is a hematologic cancer or is a solid tumor.

In some embodiments, the tumor antigen is a B7H3. In some embodiments, the cancer is a prostate cancer, melanoma, Head and neck squamous cell carcinoma (HNSCC), non-small cell lung cancer (NSCLC), urothelial cancer, ovarian cancer, neuroblastoma, rhabdomyosarcoma, osteosarcoma, Ewing sarcoma, Wilms' tumor. In some embodiments, the cancer is an ovarian carcinoma or a neuroblastoma.

In some embodiments, the tumor antigen is a CD19. In some embodiments, the cancer is a B cell cancer. In some embodiments, the cancer is a leukemia or a lymphoma. In some embodiments, the cancer is a metastatic lymphoma.

### Brief Description of the Drawings

**FIGS. 1A-1C** show diagrams depicting the native TGFβ signaling pathway **(****FIG. 1A****),** the native Toll-like receptor signaling pathway **(****FIG. 1B****),** and the chimeric TGFβ signaling receptor pathway **(****FIG. 1C****).**
**FIG. 2** shows exemplary chimeric TGFβ signaling receptor constructs and exemplary control chimeric signaling receptor constructs.
**FIG. 3A** shows expression levelsof exemplary chimeric TGFβ signaling receptor constructs in transduced Jurkat cells compared against untransduced (UT) Jurkat cells, as determined using flow cytometry to detect myc-tags. **FIG. 3B** shows flow cytometric analysis of exemplary CAR and exemplary chimeric TGFβ signaling receptor CTSR-2C (SEQ ID NO: 30) in Jurkat cells. **FIG. 3C** and **FIG. 3D** show expression of the exemplary CAR and the exemplary chimeric TGFβ signaling receptor CTSR-2C in primary human T cells, respectively.
**FIG. 4A** and **FIG. 4B** show protein expression using western blot analysis for phospho-IRAK4 (pIRAK4), phospho-SMAD2 (pSMAD2), SMAD2 (total protein), and an actin control, in untransduced (UT) Jurkat cells and Jurkat cells transduced with exemplary chimeric TGFβ signaling receptors,NULL-MYD88, TGFBR2-DNR, following incubation with different amounts of TGFβ for 2 hours.
**FIG. 5A** shows a CFDA proliferation assay of T cells engineered with an exemplary chimeric TGFβ signaling receptor (CTSR-2C; SEQ ID NO: 30) and an exemplary CAR (CTSR-2C+CAR) or the exemplary CAR alone (CAR), and incubated with TGFβ at various concentrations (1, 5, 10, and 20 ng/ml) after 6 days of exposure to target cells (Effector:Target ratio 1:1).
**FIG. 5B** shows proliferative activity of untransduced cells (UT), cells expressing both an exemplary chimeric TGFβ signaling receptor (SEQ ID NO: 30) and a CAR (CTSR-2C+CAR), and a CAR alone (CAR),as determined by a CFDA proliferation assay.
**FIG. 6A** and **FIG. 6B** show T cell count fold changes over time for cells transduced with exemplary chimeric TGFβ signaling receptors and a CAR or a CAR alone and untransduced controls (UT) in the presence of 10 and 50 ng/mL TGFβ, respectively.
**FIG. 7** shows the kinetics of cell killing by T cells transduced with exemplary chimeric TGFβ signaling receptors and a CAR or CAR alone under culturing conditions with different Effector to Target ratios (E:T) and TGFβ concentrations.
**FIG. 8** shows cytotoxic kinetics of T cells transduced with an exemplary chimeric TGFβ signaling receptor (SEQ ID NO: 30) and a CAR under culturing conditions with different E:T ratios and TGFβ concentrations using live cell imaging.
**FIG. 9A** (CTSR-2C (SEQ ID NO: 30) + CAR) and **FIG.9B** (CAR only) show normalized cell counts at different E:T ratios and TGFβ concentrations.
**FIG. 10A** and **FIG. 10B** show bioluminescence of orthotopic growth over 8 weeks of a human neuroblastoma tumor model and a pancreatic tumor model (Left - CFPAC cell lines, Right - CF10.05 cell lines), respectively, in response to treatment with untransduced cells (UT), a CAR alone, CTSR-2C alone, or a combination of a CAR with CTSR-2C (CTSR-2C+CAR). Star denotes euthanized animal (n = 3).
FIG. 11A and **FIG. 11B** show median and boxplot summary data of the presence of CAR+ and CD3 T cells in peripheral blood counts (counts/mm³ of blood), respectively, for tumor bearing mice and non-tumor (NT) bearing mice (saline injected) that received treatment with cells expressing an exemplary chimeric TGFβ signaling receptor (SEQ ID NO: 30) and a CAR (CTSR-2C+CAR), a CAR alone, or untransduced cells (UT).
**FIGS. 12A-12C** show bioluminescence images of metastatic neuroblastoma tumor growth at different times post tumor inoculation (top plots) and representative images from immunohistochemical analysis of liver tissue obtained from mice 7 days post treatment injection after staining for neuroblastoma specific marker PHOX2b (brown stain) and human CD3 (red stain) (bottom plots).
**FIG. 13** shows exemplary bicistronic polynucleotide constructs including an exemplary chimeric TGFβ signaling receptor and an exemplary CAR.
**FIG. 14** shows representative dot plots from flow cytometric analysis of T cells transduced with bicistronic polynucleotides, where the exemplary chimeric TGFβ signaling receptor was detected by myc-tag, and the CAR was detected by Protein L.
**FIG. 15** shows protein expression using western blot analysis for phospho-IRAK4 (pIRAK4), phospho-SMAD2 (pSMAD2), and Beta Actin (loading control) in Jurkat cells transduced with bicistronic polynucleotides or control polynucleotides following incubation with 0, 1, 5, 10, and 50 ng of TGFβ for 2 hours.
**FIG. 16A** shows the kinetics of cytotoxicity effects of T cells transduced with exemplary bicistronic polynucleotides, exemplary chimeric TGFβ signaling receptors, or CAR alone on CD19+ tumor cells at different Effector to Target ratios (E:T of 20, 10, 5, and 2).
**FIG. 16B** shows the kinetics of cytotoxicity effects of T cells transduced with exemplary bicistronic polynucleotides, exemplary chimeric TGFβ signaling receptors, or CAR alone on CD19+ tumor cells in the presence or absence of TGFβ (10 ng/mL).
**FIG. 17** shows bioluminescence images of tumor (lymphoma) growth over 8 weeks for indicated treatment groups.
**FIG. 18** shows the percentage of cells expressing 1, 2, or 3 exhaustion markers (PD1, TIM3, and LAG3) of exemplary chimeric TGFβ signaling receptor constructs and a CAR in transduced T cells compared against T cells transduced with CAR alone, or transduced with a CAR and TGFβR2-41bb construct after 72 hours of exposure to target cells (Effector:Target ratio 1:1) in the presence or absence of TGFβ (10 ng/mL), as determined by flow cytometry.
**FIG. 19** shows T cell count fold changes over time for T cells transduced with exemplary chimeric TGFβ signaling receptors and CAR, TGBFR2-DNR CAR, TGFBR2-41BB CAR, TGFBR2-DAP12 CAR, or a CAR alone for 7 days of exposure to target cells (Effector:Target ratio 1:1) followed by daily IL2 treatment (30U/ml) in the presence or absence of 10 ng of TGFβ (added every 48 hours) for 3 weeks.
**FIG. 20A** and **FIG. 20B** show the kinetics of cell killing by T cells transduced with exemplary chimeric TGFβ signaling receptors and a CAR, TGBFR2-DNR CAR, TGFBR2-41BB CAR, TGFBR2-DAP12 CAR, or a CAR cultured with target cells (Effector:Target ratio 1:1) for 1 week in the presence or absence of 10 ng/ml of TGFβ (added every 48 hours). **FIG. 20B** demonstrates killing activity of T cells obtained after the end of the experiment in **FIG. 20A****,** cultured with fresh target cells (Effector:Target ratio 1:1) for an additional week.

### Detailed Description

Provided herein are chimeric signaling receptors that contain an extracellular domain capable of binding an immunosuppressive molecule (e.g., immunosuppressive cytokine) present in an extracellular milieu and a MyD88-containing intracellular domain involved in signaling within immune cells. In some cases, the immunosuppressive molecule is a soluble molecule present in the extracellular milieu of the tumor microenvironment (TME). Engineered cells expressing the provided chimeric signaling receptors can be used in methods of adoptive cell therapy for redirecting immunosuppressive signals and improve responses of the cell therapy.

TMEs contain numerous signaling molecules capable of promoting tumor growth and tumor angiogenesis. Some signaling molecules in the TME also act to suppress immune responses, thereby promoting the survival of tumor cells. Non-limiting examples of immunosuppressive or anti-inflammatory cytokines include transforming growth factor-beta (TGFβ), interleukin 10 (IL10), interleukin 4 (IL4), and interleukin 1 receptor antagonist (IL1Ra). Immunosuppressive or anti-inflammatory cytokines may be secreted by cells of or surrounding the TME, where they may act, for example, to inhibit inflammatory responses to diseased or cancerous cells.

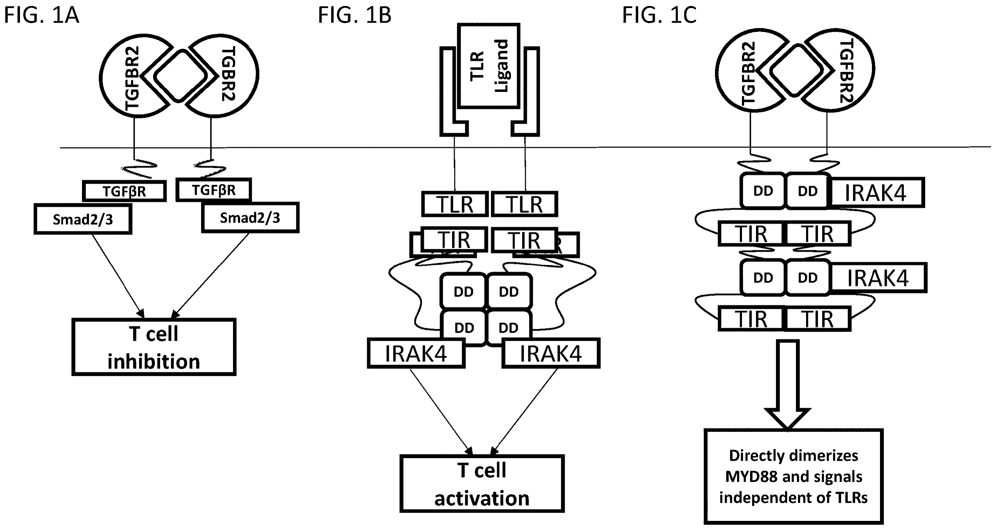

For example, transforming growth factor-beta (TGFβ), which is found in TMEs, is secreted by cancer cells and surrounding stromal cells (e.g., fibroblasts), resulting in, for example, cancer cell proliferation and angiogenesis. Binding of TGFβ to its cognate binding partner TGFβ receptor (e.g., TGFβR2) on immune cells, e.g., T cells, Tumor infiltrating Lymphocytes (TILs), Natural Killer (NK) cells, engages a signaling pathway (e.g., SMAD pathway) that results in cellular inhibition, e.g., T cell inhibition. TGFβ in the tumor TME is thus immunosuppressive, allowing cancer cells to escape a cytotoxic inflammatory response **(****FIG. 1A****).**

To circumvent the immunosuppressive effects of certain immunosuppressive cytokines, such as TGFβ, IL10, IL4, IL1Ra, in the TME, the chimeric signaling receptors provided herein are engineered to contain an extracellular binding domain that recognizes the immunosuppressive cytokine and an intracellular domain with MyD88 domains. In cells expressing the chimeric signaling receptors, the binding of the immunosuppressive cytokine to the chimeric signaling receptor reverses the T cell inhibitory activity of the immunosuppressive cytokine and instead mediates T cell activation. Thus, the provided chimeric signaling receptor can provide a positive signal to T cells even when the cells are present in an immunosuppressive environment, such as the TME, thereby improving the T cell activity, proliferation, persistence, or survival.

MyD88 is an adaptor signaling molecule that provides positive cell signaling to promote immune cell, e.g., T cell activation, and is normally engaged in the Toll-like receptor (TLR) signaling pathway (see, **FIG. 1B****).** MyD88 is involved in mediating activation of NF-κB and mitogen-activated protein kinase signaling cascades following ligation of interleukin-1 Receptor (IL-1R) or Toll-like receptor (TLR) by its ligands. As depicted in **FIG. 1B****,** normally, ligand binding to the IL-1R or TLR receptors promotes receptor oligomerization, resulting in recruitment of MyD88 via TIR domains and Myd88 dimerization. MyD88 then recruits interleukin-1 receptor-associated kinase 4 (IRAK4), resulting in IRAK4 dimerization and trans-autophosphorylation to initiate downstream signaling cascades leading to activation of transcription factors, such as NF-κB, production of inflammatory cytokines and generation of TH1 responses.

MyD88 (e.g. UniProt Accession No. Q99836; set forth in SEQ ID NO:128) contains an N-terminal death domain (DD, e.g. amino acids 19-109), an intermediate domain (ID; e.g. amino acids 110-155), and a COOH-terminal Toll/IL-1 receptor (TIR) domain (e.g. amino acids 159-296) that are involved in the interactions with the receptor TIR domains and interactions with IRAK4. Studies have shown that residues in the DD and ID are involved in the dimerization of MyD88 and recruitment of IRAK4 (Burns et al. 2003, J. Exp. Med., 197:263-268). A portion of residues (e.g. within or including the first 17 amino acids) in the TIR domain also may be involved in IRAK 4 recruitment, whereas the presence of a complete TIR domain is not required (Burns et al. 2003). It is understood that reference to amino acids, including to a specific sequence set forth as a SEQ ID NO used to describe domain organization (e.g. of a MyD88 polypeptide or a chimeric signaling receptor containing same) are for illustrative purposes and are not meant to limit the scope of the embodiments provided. It is understood that polypeptides and the description of domains thereof are theoretically derived based on homology analysis and alignments with similar molecules. Thus, the exact locus can vary, and is not necessarily the same for each protein. Hence, a specific domain, such as specific DD domain, ID domain or TIR domain of MyD88 can be several amino acids (such as one, two, three or four) longer or shorter.

In some embodiments, the intracellular domain, also referred to herein as a cytoplasmic domain, of the provided chimeric signaling receptor contains one or more, e.g. two, MyD88 polypeptide. Each of the one or more, e.g. two, MyD88 polypeptide can be a full-length MyD88 or a truncated portion of MyD88 that is able to dimerize, recruit IRAK-4, and/or facilitate phosphorylation (e.g. activation) of IRAK4, such as when the chimeric signaling receptor is expressed from a cell and engaged by the immunosuppressive cytokine. Hence, reference to a "truncated MyD88" or "truncated portion of MyD88", or variations thereof, means that the MyD88 is not full length and may lack a domain or part of a domain, but retains or exhibits one or more activities of full-length MyD88 to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine. In some embodiments, the truncated portion of MyD88 is the minimal part of the MyD88 polypeptide needed to dimerize, recruit IRAK4 and/or facilitate phosphorylation of IRAK4, such as when the chimeric signaling receptor is expressed from a cell and engaged by the immunosuppressive cytokine. In some embodiments, the truncated portion contains the DD, ID and, in some cases, lacks the full-length TIR domain. In some embodiments, the truncated portion contains the DD, ID and contains a partial portion of the TIR domain that lacks up to 140 contiguous C-terminal amino acid residues in the TIR domain, such as 124, 125, 126, 127, 128, 129, 130, 131, 132, 133, 134, 135, 136, 137, 138, 139 or 140 contiguous C-terminal amino acid residues of the TIR domain. It should be appreciated that the term MyD88 polypeptide as used herein encompasses full-length and truncated portions of a MyD88 polypeptide as described, unless stated otherwise.

In some embodiments, the one or more, e.g. two, MyD88 polypeptide comprised in the intracellular signaling domain is a full-length MyD88 polypeptide. In some embodiments, the one or more, e.g. two, MyD88 polypeptide comprised in the intracellular signaling domain is truncated portion of MyD88 sufficient to dimerize, recruit IRAK-4 and/or mediate IRAK-4 phosphorylation, such as when the chimeric signaling receptor is expressed from a cell and engaged by the immunosuppressive cytokine. In cells expressing the chimeric signaling receptors, the binding of the immunosuppressive cytokine to the chimeric signaling receptor is able to promote MyD88 dimerization and/or recruitment of IRAK-4, resulting in IRAK-4 phosphorylation and downstream signaling cascades **(****FIG. 1C****).** The ability of the MyD88 polypeptide to mediate or promote IRAK4 phosphorylation triggers IRAK4 activation to initiate a signaling cascade that results in activation of the immune cell, e.g., T cell.

In some embodiments, the intracellular domain of the chimeric signaling receptor is or comprises a MyD88 polypeptide. In some cases, the intracellular domain contains at least one, and typically two, MyD88 polypeptides. In some embodiments, the intracellular domain contains at least one MyD88 polypeptide. In some embodiments, the intracellular domain contains two MyD88 polypeptide. In particular embodiments, the two MyD88 polypeptides present in the intracellular domain are identical. In some embodiments, each of the two MyD88 polypeptides are a full-length MyD88. In some embodiments, each of the two MyD88 polypeptides is a MyD88 truncated portion that is able to dimerize, recruit IRAK-4 and/or mediate IRAK-4 phosphorylation, such as when the chimeric signaling receptor is expressed from a cell and engaged by the immunosuppressive cytokine. In some embodiments, for example when two MyD88 polypeptides are present, the MyD88 polypeptides are linked directly or indirectly, e.g., via a linker. In some embodiments, when two MyD88 polypeptides are present, the MyD88 polypeptides are linked via a linker. In some embodiments, the linker is (GGGGS)n (SEQ ID NO: 47), where n is an integer between 1 to 4, inclusive. In some embodiments, the linker is GGGGS (SEQ ID NO: 48).

The extracellular domain of the provided chimeric signaling receptors binds to a molecule, such as a cytokine, known to be prevalent in the TME and to cause immune suppression, and is linked (directly or indirectly via a linker) to the one or more, e.g. two, MyD88-containing intracellular domain via the transmembrane domain. An extracellular domain may also be referred to herein alternatively as an extracellular domain. In some embodiments, the cytokine to which the extracellular domain binds is an immunosuppressive or anti-inflammatory cytokine. In some embodiments, the cytokine is an immunosuppressant. In some embodiments, the cytokine suppresses immune cells. In some embodiments, the cytokine is transforming growth factor-beta (TGFβ). TGFβ is secreted in the TME by cancer cells and fibroblasts, and can result in immune suppression and angiogenesis. In some embodiments, the cytokine receptor binds TGFβ. Other examples of immunosuppressive cytokines targeted by a provided chimeric signaling receptor include, but are not limited to, IL10, IL4 or IL1Ra.

The chimeric signaling receptors provided herein reverse the immunosuppressive effects of the immunosuppressive cytokine, e.g. TGFβ, IL10, IL4, IL1Ra, in the TME on immune cells, allowing immune cells to mount an effective response against tumor cells. For example, as shown in the Examples herein, the chimeric signaling receptors engage signaling pathways that activate immune cells, while also reducing signaling through the native, e.g., suppressive, pathway. As shown herein, cells expressing chimeric signaling receptors with an intracellular domain containing two MyD88 polypeptides in particular show surprisingly effective redirection of immunosuppressive signaling as exemplified in the presence of TGFβ, including the ability to facilitate, instead of reduce, tumor cell killing in both in vitro and in vivo assays. Cells engineered to express the chimeric signaling receptors provided herein demonstrate improved persistence, survival, proliferation, and cytotoxicity in the presence of an environmental milieu containing the exemplary immunosuppressive cytokine TGFβ, as compared to cells, including those engineered to express a recombinant antigen receptor (e.g., CAR, TCR), lacking the chimeric signaling receptor described herein. The chimeric signaling receptors provided herein can be used to turn an immunosuppressive TME into an activating environment. In some embodiments, the chimeric signaling receptors provided herein can be used to enhance persistence, survival, proliferation and cytotoxicity of cells that express a recombinant antigen receptor (e.g. CAR) and/or that produce secretory proteins (such as BiTEs or cytokines, etc.).

In some embodiments, the chimeric signaling receptors described herein are expressed in immune cells, such as T cells, Tumor Infiltrating Lymphocytes (TILs), and Natural Killer (NK) cells, capable of tumor/cancer cell killing.

In some embodiments, the chimeric signaling receptor is provided in cells encoding a recombinant antigen receptor, such as a CAR or recombinant TCR, that combines a ligand-binding domain (e.g. antibody or antibody fragment) that provides specificity for a desired antigen (e.g., tumor antigen) with an activating intracellular domain portion, such as a T cell activating domain, providing a primary activation signal. Alternatively or additionally, in some aspects, the chimeric signaling receptor is provided in cells encoding a secretable recombinant molecule, such as a cytokine or a bispecific antibody (e.g. a BiTE). In some embodiments, the provided chimeric signaling receptors, when genetically engineered into immune cells can modulate immune cell, e.g., T cell, stimulation and/or activation, thereby resulting in genetically engineered cells with improved longevity, survival and/or persistence in vivo, such as for use in adoptive cell therapy methods. In some aspects, the engineered cell effects increased killing of a target cell expressing the target molecule compared to killing of cells not expressing the target antigen.

In some cases, the chimeric signaling receptors and recombinant antigen receptors can be expressed in T cells to produce genetically engineered T cells that, when administered to a subject, exhibit one or more properties that are improved compared to a reference cell composition that does not express the chimeric signaling receptor. Alternatively or additionally, in some aspects, the chimeric signaling receptors can be expressed in T cells encoding a secretable recombinant molecule, such as a cytokine or a bispecific antibody (e.g. a BiTE). In some instances, the increased persistence, activity, binding, and/or killing is greater than effected by a reference composition comprising an engineered cell not expressing the chimeric signaling receptor. Thus, in some cases, one or more properties of administered genetically engineered cells that can be improved or increased or greater compared to administered cells of a reference composition include increased activation, and increased survival and/or persistence.

In some embodiments, engineered cells containing the chimeric signaling receptor exhibit increased persistence and/or survival compared to similar cells not engineered or compared to similar cells comprising a recombinant antigen receptor but not the chimeric signaling receptor. In some embodiments, a genetically engineered cell with increased persistence exhibits better potency in a subject to which it is administered.

Also provided herein are methods, uses, and compositions of the chimeric signaling receptor, engineered cells contain chimeric signaling receptors, and engineered cells containing the chimeric signaling receptor and recombinant molecule, such as a recombinant antigen receptor, e.g., CAR, TCR, or a recombinant molecule that is secreted such as a cytokine or bispecific antibody, e.g. a BiTE. Methods of engineering, preparing, and producing chimeric signaling receptors and engineered cells containing the chimeric signaling receptor are also provided, including nucleic acids for engineering cells. Further provided are kits and article of manufacture.

All publications, including patent documents, scientific articles and databases, referred to in this application are incorporated by reference in their entirety for all purposes to the same extent as if each individual publication were individually incorporated by reference. If a definition set forth herein is contrary to or otherwise inconsistent with a definition set forth in the patents, applications, published applications and other publications that are herein incorporated by reference, the definition set forth herein prevails over the definition that is incorporated herein by reference.

The section headings used herein are for organizational purposes only and are not to be construed as limiting the subject matter described.

### I. CHIMERIC SIGNALING RECEPTORS

Provided herein are chimeric signaling receptors capable of engaging intracellular signaling pathways that induce immune cell activation upon binding of target molecules, e.g., cytokines such as immunosuppressive or anti-inflammatory cytokines, for example in a tumor microenvironment (TME). In some embodiments, the chimeric signaling receptors are engineered to contain an extracellular domain joined to an intracellular signaling domain, where the extracellular domain contains an antigen binding domain, e.g., from a receptor such as cytokine receptor, that binds to an immunosuppressive or anti-inflammatory cytokine, such as TGFβ, IL10, IL4, or IL1Ra, and the intracellular domain contains one or more, e.g. two, MyD88 adaptor molecule polypeptide. In some embodiments, the intracellular signaling domain contains two MyD88 polypeptides. In some embodiments, the one or more MyD88 polypeptide, e.g. two MyD88 polypeptide, may be a full-length MyD88. In some embodiments, the one or more MyD88 polypeptide, e.g. two MyD88 polypeptide, is a truncated portion that is able or sufficient to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine. In some embodiments, the one or more, e.g. two, MyD88 polypeptide has the sequence set forth in SEQ ID NO:2. In some embodiments, the chimeric receptor polypeptide has an intracellular domain containing two MyD88 polypeptides that each has the sequence set forth in SEQ ID NO:2.

In some embodiments, the extracellular domain binds to an immunosuppressive or anti-inflammatory cytokine, such as TGFβ, IL10, IL4, or IL1Ra. In some embodiments, the extracellular domain of the chimeric signaling receptor is any molecule that is able to bind, such as specifically bind, to an immunosuppressive cytokine. In some embodiments, the extracellular domain is or contains a portion of the native receptor of the immunosuppressive cytokine of a receptor, for example a portion of the native cytokine receptor that binds a cytokine present in a TME. For instance, the extracellular domain of the provided chimeric signaling receptor is or contains an extracellular domain of a naturally occurring cytokine receptor or cytokine binding fragment thereof that is able to bind, such as specifically bind, the immunosuppressive cytokine. In some embodiments, the extracellular domain of the chimeric signaling receptor is an antibody or an antigen binding fragment that is able to bind, such as specifically bind, the immunosuppressive cytokine. Non-limiting examples of antigen binding molecules include antibodies, antibody fragments including, for example, divalent antibody fragments such as a (Fab)2'-fragments and divalent single-chain Fv (scFv) fragments, and monovalent antibody fragments such as Fab fragments, Fv fragments, and scFv fragments, and antigen binding fragments of any of the foregoing.

In some embodiments, the cytokine is TGFβ and the extracellular domain of the chimeric signaling receptor binds, such as specifically binds, TGFβ. In some embodiments, the extracellular domain is or contains a portion of the native cytokine receptor that binds TGFβ, which can be a TGFβ receptor. In some embodiments, the extracellular domain of the provided chimeric signaling receptor is or contains an extracellular domain or binding fragment thereof of a TGFβ receptor (TGFβR), e.g., a TGFβR2 or TGFβR1. In some embodiments, the extracellular domain is the extracellular domain of a TGFβR2.

In some embodiments, the cytokine is IL10 and the extracellular domain of the chimeric signaling receptor binds, such as specifically binds, IL-10. In some embodiments, the extracellular domain is or contains a portion of the native cytokine receptor that binds IL10, which can be the IL10R. In some embodiments, the extracellular domain of the provided chimeric signaling receptor is or contains an extracellular domain or binding fragment thereof of IL-10R.

In some embodiments, the cytokine is IL4 and the extracellular domain of the chimeric signaling receptor binds, such as specifically binds, IL-4. In some embodiments, the extracellular domain is or contains a portion of the native cytokine receptor that binds IL4, which can be the IL4R. In some embodiments, the extracellular domain of the provided chimeric signaling receptor is or contains an extracellular domain or binding fragment thereof of IL4R.

In some embodiments, the cytokine is IL1Ra and the extracellular domain of the chimeric signaling receptor binds, such as specifically binds, IL-1Ra. In some embodiments, the extracellular domain of the provided chimeric signaling receptor is or contain an antibody or antigen-binding fragment, e.g., scFv, directed against IL-1Ra.

The chimeric signaling receptors provided herein may include additional domains, such as transmembrane domains and/or linkers, to maintain functionality of the chimeric signaling receptor and the components thereof, and/or to allow expression of the chimeric signaling receptors on a cell surface, e.g., engineered cell surface.

In some embodiments, the intracellular domain containing the one or more, e.g. two, MyD88 polypeptide (e.g. SEQ ID NO:2) of the chimeric signaling receptor is joined to the extracellular domain through the transmembrane domain. In some embodiments, the transmembrane domain of the provided chimeric signaling receptor is linked directly to one or both of the extracellular domain and the intracellular domain containing the one or more, e.g. two, MyD88 polypeptide (e.g. SEQ ID NO:2). In some embodiments, the transmembrane domain of the provided chimeric signaling receptor is linked indirectly via a linker to one or both of the extracellular domain and the intracellular domain containing the one or more, e.g. two, MyD88 polypeptide (e.g. SEQ ID NO:2). In some embodiments, the transmembrane domain of the provided chimeric signaling receptor is linked directly to the extracellular domain and is linked indirectly via a linker to the intracellular domain containing the one or more, e.g. two, MyD88 polypeptide (e.g. SEQ ID NO:2). In some embodiments, the transmembrane domain of the provided chimeric signaling receptor is linked indirectly via a linker to the extracellular domain and is linked directly to the intracellular domain containing the one or more, e.g. two, MyD88 polypeptide (e.g. SEQ ID NO:2). In some embodiments, the linker may be a naturally occurring sequence of the cytokine receptor from which the extracellular domain or the transmembrane domain is derived. In some embodiments, the linker may be a synthetic linker. In some embodiments, the linker is a peptide linker. For instance, in some embodiments, the linker is (GGGGS)n (SEQ ID: 47), where n is an integer between 1 to 4, inclusive. In some embodiments, the linker is (GGGGS)₂ (SEQ ID NO: 85).

In some embodiments, the transmembrane domain is heterologous to or not naturally part of the native cytokine receptor. The heterologous transmembrane domain may be any as described.

In some embodiments, the extracellular domain and the transmembrane domain are both from the native cytokine receptor that binds the immunosuppressive cytokine, e.g. TGFβ, IL10, or IL4. For instance, the transmembrane domain and the extracellular domain can be from the same cytokine receptor, in which the transmembrane domain is the naturally occurring transmembrane domain of the cytokine receptor. In some embodiments, the extracellular domain is an extracellular domain of a TGFβR, e.g., a TGFβR2 or TGFβR1, and the transmembrane domain is a TGFβR transmembrane domain, e.g., a TGFβR2 or TGFβR1 transmembrane domain, that is linked directly to the respective TGFβR extracellular domain. In some embodiments, the transmembrane domain is a TGFβR2 linked directly to a TGFβR2 extracellular domain. In some embodiments, the extracellular domain is an extracellular domain of an IL10R and the transmembrane domain is an IL10R transmembrane domain that is linked directly to the IL10R extracellular domain. In some embodiments, the extracellular domain is an extracellular domain of an IL4R and the transmembrane domain is an IL4R transmembrane domain that is linked directly to the IL4R extracellular domain.

In some embodiments, the one or more MyD88 polypeptide of the intracellular domain of the chimeric signaling receptor is joined to the transmembrane domain through a linker. In some embodiments, the linker may be a naturally occurring sequence that is or includes a partial sequence of the intracellular domain of the cytokine receptor from which the extracellular domain or the transmembrane domain is derived. In some embodiments, the linker may be a synthetic linker. In some embodiments, the linker is a peptide linker. For instance, in some embodiments, the linker is (GGGGS)n, where n is an integer between 1 to 4, inclusive (SEQ ID NO: 47). In some embodiments, the linker is (GGGGS)₂ (SEQ ID NO: 85).

In some embodiments, the extracellular domain, the transmembrane domain and a partial sequence of the intracellular domain linking the one or more, e.g. two, MyD88 polypeptide to the transmembrane domain in the provided chimeric signaling receptor are contiguous sequences of amino acids from the native cytokine receptor that binds the immunosuppressive cytokine, e.g. TGFβ, IL10, or IL4. In some embodiments, the chimeric signaling receptor binds TGFβ and contains an extracellular domain that is the extracellular domain of a TGFβR, e.g., a TGFβR2 or TGFβR1, a transmembrane domain that is the transmembrane domain of the respective TGFβR, e.g., a TGFβR2 or TGFβR1, and an intracellular domain containing at least a contiguous N-terminal portion of the intracellular domain of the respective TGFβR, e.g., a TGFβR2 or TGFβR1 linked to the one or more, e.g. two, MyD88 polypeptide (e.g. SEQ ID NO:2). In some embodiments, the chimeric signaling receptor binds IL10 and contains an extracellular domain that is the extracellular domain of a IL10R, a transmembrane domain that is the transmembrane domain of the respective IL10R, and an intracellular domain containing at least a contiguous N-terminal portion of the intracellular domain of the IL10R linked to the one or more, e.g. two, MyD88 polypeptide (e.g. SEQ ID NO:2). In some embodiments, the chimeric signaling receptor binds IL4 and contains an extracellular domain that is the extracellular domain of a IL4R, a transmembrane domain that is the transmembrane domain of the IL4R, and an intracellular domain containing at least a contiguous N-terminal portion of the intracellular domain of the IL4R linked to the one or more, e.g. two, MyD88 polypeptide (e.g. SEQ ID NO:2).

In some embodiments, the chimeric signaling receptor contains a contiguous amino acid portion of the native cytokine receptor that binds the immunosuppressive cytokine, e.g. TGFβ, IL10, or IL4, in which the contiguous amino acid portion includes at least the extracellular domain and transmembrane domain of the native cytokine receptor, but in which a portion of the intracellular signaling domain of the native cytokine receptor is replaced by the one or more, e.g. two, MyD88 polypeptides (SEQ ID NO:2). In some embodiments, the intracellular signaling domain of the chimeric signaling receptor retains at least a partial sequence of the intracellular signaling domain of the native cytokine receptor. In some embodiments, the partial sequence is between 1 and 50 amino acid residues of the N-terminal portion of the intracellular signaling domain of the native cytokine receptor. In some embodiments, the partial sequence is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acid residues of the N-terminal portion of the intracellular signaling domain of the native cytokine receptor. Typically, the partial sequence of the intracellular signaling domain of the native cytokine receptor is a non-functional portion that does not confer inhibitory signaling activity. For instance, the partial sequence of the intracellular signaling domain of the native cytokine receptor is not a functional inhibitory signaling domain such that it is not capable of being phosphorylated (activated) and/or of recruiting an inhibitory adaptor molecule such as a SMAD (e.g. SMAD2 or SMAD3) in response to ligand binding to the chimeric signaling receptor.

In some embodiments, the chimeric signaling receptors described herein contain, from N- to C-terminus: an extracellular domain that binds to the immunosuppressive cytokine; a transmembrane domain; a linker sequence, e.g. as described herein; and an intracellular signaling domain comprising two MyD88 domains linked in tandem, e.g. though a linker as described herein. The chimeric signaling receptors provided herein are engineered such that binding of the cytokine, e.g., TGFβ, IL10, IL4, IL1Ra, by the extracellular domain induces dimerization of the MyD88 polypeptide. For instance, upon binding of the immunosuppressive cytokine, e.g. TGFβ, IL10, IL4, IL1Ra, two chimeric receptor signaling receptors can form a dimer at the surface of the cell via interactions between the MyD88 domain (see e.g. FIG. 1C). In some embodiments, dimerization of the MyD88 polypeptide produces an oligomeric complex comprising the MyD88 polypeptides and IRAK4 that has been recruited to the MyD88 adaptor. Thus, in some embodiments, binding of the immunosuppressive or anti-inflammatory cytokine by the extracellular domain induces MyD88-mediated signaling (e.g., intracellular signaling). In some embodiments, the MyD88-mediated signaling induces immune cell, e.g., T cell, activation.

In some embodiments, for purposes of aiding in the detection or quantification of chimeric signaling receptor expression, the chimeric signaling receptor can include a detectable moiety or marker. In some embodiments, the detectable marker is a myc-tag (SEQ ID NO: 101). In some embodiments, the detectable marker is a polyhistidine tag (SEQ ID NO: 103). In some embodiments, the detectable marker is located at the N-terminus of the extracellular domain of the chimeric signaling receptor.

In some embodiments, the chimeric signaling receptor includes a signal peptide to facilitate localization to the cell membrane for expression on the cell surface. In some embodiments, the signal peptide is present in a precursor chimeric signaling receptor protein and is cleaved to form a the mature chimeric signaling receptor. In some embodiments, the signal peptide is a CD8α signal peptide. In some embodiments, the signal peptide is or comprises the amino acid sequence set forth by SEQ ID NO: 102.

In the subsections below, exemplary domains and sequences of the provided chimeric signaling receptors are described.

### A. Extracellular Domain

In some embodiments, the chimeric signaling receptor includes an extracellular domain that binds, such as specifically binds, to an immunosuppressive or anti-inflammatory cytokine. In some embodiments, the immunosuppressive or anti-inflammatory cytokine is any one of TGFβ, IL10, IL4, or IL1Ra. In some embodiments, the extracellular domain of the chimeric signaling receptor is or includes an extracellular portion of a cognate receptor or a binding portion that binds the respective immunosuppressive or anti-inflammatory cytokine, such as an extracellular portion of a cognate receptor that binds one of TGFβ, IL10, IL4, or IL1Ra. In some embodiments, the extracellular domain of the chimeric signaling receptor is or includes an antibody or antigen-binding fragment thereof, e.g. single chain variable fragment (scFv) or single domain antibody (sdAb) that binds to the immunosuppressive or anti-inflammatory cytokine, such as that binds one of TGFβ, IL10, IL4, or IL1Ra.

In some embodiments, the immunosuppressive or anti-inflammatory cytokine is TGFβ. In some embodiments, the chimeric signaling receptor includes an extracellular domain that binds, such as specifically binds, to TGFβ. In some embodiments, the extracellular domain that binds TGFβ is an extracellular domain of a TGFβ receptor (TGFβR) or a portion thereof that binds TGFβ. In some embodiments, the TGFβ receptor (TGFβR) or a portion thereof that binds TGFβ is a TGFβ receptor 1 (TGFβR1). In some embodiments, the TGFβR or portion thereof that binds TGFβ is a TGFβ receptor 2 (TGFβR2).

In some embodiments, the extracellular domain contains an extracellular domain of TGFβR2, or a portion thereof that binds TGFβ. TGFβR2 has a structural organization and sequence as described in UniProt P37173. In some embodiments, the extracellular domain of the chimeric signaling receptor comprises or is the sequence set forth by SEQ ID NO: 20. In some embodiments, the extracellular domain of the chimeric signaling receptor comprises or is a sequence of amino acid that is or exhibits at least or about 85%, at least or about 90%, at least or about 92%, at least or about 95%, at least or about 97% sequence identity to the sequence set forth in SEQ ID NO: 20 that binds to TGFβ. In some embodiments, the extracellular domain of the chimeric signaling receptor is or comprises a portion of the amino acid sequence of SEQ ID NO: 20 that binds to TGFβ. In some embodiments, the extracellular domain of the chimeric signaling receptor is or comprises a portion of an amino acid sequence that exhibits at least or about 85%, 90%, 92%, 95%, or 97% sequence identity to SEQ ID NO: 20 and that binds to TGFβ. In some embodiments, the extracellular domain of the chimeric signaling receptor is encoded by the sequence set forth by SEQ ID NO: 21, or a sequence of amino acid that exhibits at least or about 85%, at least or about 90%, at least or about 92%, at least or about 95%, at least or about 97% sequence identity to the sequence set forth in SEQ ID NO: 21 in which the encoded sequence binds to TGFβ.

In some embodiments, the extracellular domain that binds TGFβ is an extracellular domain of a TGFβR1 or a portion thereof that binds TGFβ. TGFβR1 has a structural organization and sequence as described in UniProt P36897. In some embodiments, the extracellular domain of the chimeric signaling receptor comprises or is the sequence set forth by SEQ ID NO: 4. In some embodiments, the extracellular domain of the chimeric signaling receptor comprises or is a sequence of amino acid that is or exhibits at least or about 85%, at least or about 90%, at least or about 92%, at least or about 95%, at least or about 97% sequence identity to the sequence set forth in SEQ ID NO: 4. In some embodiments, the extracellular domain of the chimeric signaling receptor is or comprises a portion of the amino acid sequence of SEQ ID NO: 4 that binds to TGFβ. In some embodiments, the extracellular domain of the chimeric signaling receptor is or comprises a portion of an amino acid sequence that exhibits at least or about 85%, 90%, 92%, 95%, or 97% sequence identity to SEQ ID NO: 4 and that binds to TGFβ. In some embodiments, the extracellular domain of the chimeric signaling receptor is a protein encoded by the sequence set forth by SEQ ID NO: 5. In some embodiments, the extracellular domain of the chimeric signaling receptor is a protein encoded by a nucleic acid sequence that exhibits at least or about 85%, at least or about 90%, at least or about 92%, at least or about 95%, at least or about 97% sequence identity to the sequence set forth in SEQ ID NO: 5, in which the encoded sequence binds to TGFβ. In some embodiments, the extracellular domain of the chimeric signaling receptor is a protein encoded by a portion of the nucleic acid sequence of SEQ ID NO: 5, in which the encoded portion binds to TGFβ. In some embodiments, the extracellular domain of the chimeric signaling receptor is a protein encoded by a portion of a nucleic acid sequence that exhibits at least or about 85%, 90%, 92%, 95%, or 97% sequence identity to SEQ ID NO: 5, in which the encoded sequence binds to TGFβ.

In some embodiments, the immunosuppressive or anti-inflammatory cytokine is IL10. In some embodiments, the chimeric signaling receptor includes an extracellular domain that binds, such as specifically binds, to IL10. In some embodiments, the extracellular domain that binds IL10 is an extracellular domain of an IL10 receptor (IL10R) or a portion thereof that binds IL10. The IL10 receptor is a type II cytokine receptor and is a tetramer of two IL10Ra subunits and two IL10Rb subunits. Binding to IL-10 is mediated by the high-affinity receptor IL10Ra (also called IL-10R1), but this intermediate complex normally must subsequently recruit the low affinity IL10Rb (also called IL-10R2) chain before cell signaling can occur.

In provided embodiments, the extracellular domain of the chimeric signaling receptor includes an extracellular domain that binds, such as specifically binds, to IL10. In some embodiments, the extracellular domain that binds IL10 is an extracellular domain of an IL-10Ra or a portion thereof that binds IL10. IL10Ra has a structural organization and sequence as described in UniProt Q13651. IL-10Ra has a sequence that contains an extracellular domain (e.g. SEQ ID NO:119), a transmembrane domain (e.g. SEQ ID NO:120) and a intracellular cytoplasmic domain (e.g. SEQ ID NO:121). In some embodiments, the extracellular domain of the chimeric signaling receptor comprises or is the sequence set forth by SEQ ID NO: 119. In some embodiments, the extracellular domain of the chimeric signaling receptor comprises or is a sequence of amino acid that is or exhibits at least or about 85%, at least or about 90%, at least or about 92%, at least or about 95%, at least or about 97% sequence identity to the sequence set forth in SEQ ID NO: 119. In some embodiments, the extracellular domain of the chimeric signaling receptor is or comprises a portion of the amino acid sequence of SEQ ID NO: 119 that binds to IL10. In some embodiments, the extracellular domain of the chimeric signaling receptor is or comprises a portion of an amino acid sequence that exhibits at least or about 85%, 90%, 92%, 95%, or 97% sequence identity to SEQ ID NO: 119 and that binds to IL10.

In some embodiments, the extracellular domain that binds IL10 is an extracellular domain of an IL-10Rb or a portion thereof that binds IL10. In some embodiments, the extracellular domain that binds IL10 is an extracellular domain that additionally is able to recruit an IL-10Rb or a portion thereof that binds IL10. IL10Rb has a structural organization and sequence as described in UniProt Q08334. IL-10Rb has a sequence that contains an extracellular domain (e.g. SEQ ID NO:122), a transmembrane domain (e.g. SEQ ID NO:123) and a intracellular cytoplasmic domain (e.g. SEQ ID NO:124). In some embodiments, the extracellular domain of the chimeric signaling receptor comprises or is the sequence set forth by SEQ ID NO: 122. In some embodiments, the extracellular domain of the chimeric signaling receptor comprises or is a sequence of amino acid that is or exhibits at least or about 85%, at least or about 90%, at least or about 92%, at least or about 95%, at least or about 97% sequence identity to the sequence set forth in SEQ ID NO: 122. In some embodiments, the extracellular domain of the chimeric signaling receptor is or comprises a portion of the amino acid sequence of SEQ ID NO: 122 that binds to IL10. In some embodiments, the extracellular domain of the chimeric signaling receptor is or comprises a portion of an amino acid sequence that exhibits at least or about 85%, 90%, 92%, 95%, or 97% sequence identity to SEQ ID NO: 122 and that binds to IL10.

In some embodiments, the immunosuppressive or anti-inflammatory cytokine is IL4. In some embodiments, the chimeric signaling receptor includes an extracellular domain that binds, such as specifically binds, to IL4. In some embodiments, the extracellular domain that binds IL4 is an extracellular domain of an IL4 receptor (IL4R) or a portion thereof that binds IL4. In some embodiments, the extracellular domain contains an extracellular domain of IL4R, or a portion thereof that binds IL4. IL4R has a structural organization and sequence as described in UniProt P24394. IL-4R has a sequence that contains an extracellular domain (e.g. SEQ ID NO:125), a transmembrane domain (e.g. SEQ ID NO:126) and an intracellular cytoplasmic domain (e.g. SEQ ID NO:127). In some embodiments, the extracellular domain of the chimeric signaling receptor comprises or is the sequence set forth by SEQ ID NO: 125. In some embodiments, the extracellular domain of the chimeric signaling receptor comprises or is a sequence of amino acid that is or exhibits at least or about 85%, at least or about 90%, at least or about 92%, at least or about 95%, at least or about 97% sequence identity to the sequence set forth in SEQ ID NO: 125. In some embodiments, the extracellular domain of the chimeric signaling receptor is or comprises a portion of the amino acid sequence of SEQ ID NO: 125 that binds to IL4. In some embodiments, the extracellular domain of the chimeric signaling receptor is or comprises a portion of an amino acid sequence that exhibits at least or about 85%, 90%, 92%, 95%, or 97% sequence identity to SEQ ID NO: 125 and that binds to IL4.

In some embodiments, the immunosuppressive or anti-inflammatory cytokine is IL1Ra. In some embodiments, the chimeric signaling receptor includes an extracellular domain that binds, such as specifically binds, to IL1Ra. In some embodiments, the chimeric signaling receptor includes an extracellular domain that is an antibody or antigen-binding fragment that binds to IL1Ra. In some embodiments, the extracellular domain is a scFv directed against IL1Ra. In some embodiments, the extracellular domain is an sdAb directed against IL1Ra.

### B. Transmembrane Domain

In some embodiments, the chimeric signaling receptor includes a transmembrane domain between the extracellular domain and the intracellular signaling domain containing the one or more, e.g. two, MyD88 polypeptides. In some embodiments, the transmembrane domain results in an encoded protein for cell surface expression on a cell. In some embodiments, the transmembrane domain is linked directly to the extracellular domain (e.g. C-terminus of the extracellular domain). In some embodiments, the transmembrane domain is linked indirectly to the extracellular domain (e.g. C-terminus of the extracellular domain) via one or more linkers or spacers. In some embodiments, the transmembrane domain is linked directly to the intracellular signaling domain (e.g. N-terminus of the intracellular signaling domain). In some embodiments, the transmembrane domain is linked indirectly to the intracellular signaling domain (e.g. N-terminus of the intracellular signaling domain) via one or more linkers or spacers. In some embodiments, the transmembrane domain contains predominantly hydrophobic amino acid residues, such as leucine and valine.

In some embodiments, the transmembrane domain is derived from a natural source. For examples, a natural transmembrane domain may be derived from any membrane-bound or transmembrane protein. In some embodiments, a transmembrane anchor domain can be used to ensure that the chimeric signaling receptor will be expressed on the surface of the engineered cell, such as engineered T cell. Conveniently, this could be from a particular native cytokine receptor from which the extracellular domain of the chimeric receptor signaling receptor is derived. In some embodiments, where the extracellular domain is an extracellular domain of a native cytokine receptor that binds to the immunosuppressive cytokine as described above, the transmembrane domain of the chimeric signaling receptor is a transmembrane domain from the native cytokine receptor or is a portion thereof sufficient to span a lipid bilayer of a cell or mediate expression of the chimeric signaling receptor on the surface of a cell. It is understood that reference to a portion of a transmembrane domain in chimeric signaling receptors provided herein is a functional portion that confers activity of the transmembrane domain to mediate expression of the chimeric receptor in the lipid bilayer of a cell. In some embodiments, the transmembrane domain is the full transmembrane domain of the native cytokine receptor. In other embodiments, the transmembrane domain is heterologous to or not naturally associated with the extracellular domain.

In some embodiments, the chimeric signaling receptor includes a transmembrane domain that is or is derived from a TGFβR. In some embodiments, the transmembrane domain is a TGFβR2 transmembrane domain. In some embodiments, the TGFβR2 transmembrane domain is or includes the sequence of amino acids of SEQ ID NO: 22. In some embodiments, the transmembrane domain is or includes a sequence of amino acids that exhibits at least or about 85%, at least or about 90%, at least or about 92%, at least or about 95%, at least or about 97% sequence identity to the sequence set forth in SEQ ID NO: 22. In some embodiments, the TGFβR2 transmembrane domain is encoded by the sequence of nucleotides set forth in SEQ ID NO:23. In some such embodiments, the extracellular domain of the chimeric receptor signaling domain is an extracellular domain from TGFβR2, such as described above.

In some embodiments, the transmembrane domain is a TGFβR1 transmembrane domain. In some embodiments, the TGFβR1 transmembrane domain is or includes the sequence of amino acids of SEQ ID NO: 6. In some embodiments, the transmembrane domain is or includes a sequence of amino acids that exhibits at least or about 85%, at least or about 90%, at least or about 92%, at least or about 95%, at least or about 97% sequence identity to the sequence set forth in SEQ ID NO: 6. In some embodiments, the TGFβR1 transmembrane domain is encoded by the sequence of nucleotides set forth in SEQ ID NO:7. In some such embodiments, the extracellular domain of the chimeric receptor signaling domain is an extracellular domain from TGFβR1, such as described above.

In some embodiments, the transmembrane domain is an IL10Ra transmembrane domain. In some embodiments, the IL10Ra transmembrane domain is or includes the sequence set forth by SEQ ID NO: 120. In some embodiments, the transmembrane domain is or includes a sequence of amino acids that exhibits at least or about 85%, at least or about 90%, at least or about 92%, at least or about 95%, at least or about 97% sequence identity to the sequence set forth in SEQ ID NO: 120. In some such embodiments, the extracellular domain of the chimeric receptor signaling domain is an extracellular domain from IL10Ra, such as described above.

In some embodiments, the transmembrane domain is IL10Rb transmembrane domain. In some embodiments, the IL10Rb transmembrane is or includes the sequence set forth by SEQ ID NO: 123. In some embodiments, the transmembrane domain is or includes a sequence of amino acids that exhibits at least or about 85%, at least or about 90%, at least or about 92%, at least or about 95%, at least or about 97% sequence identity to the sequence set forth in SEQ ID NO: 123. In some such embodiments, the extracellular domain of the chimeric receptor signaling domain is an extracellular domain from IL10Rb, such as described above.

In some embodiments, the transmembrane domain is an IL4R transmembrane domain. In some embodiments, the IL4R transmembrane domain is or comprises a sequence set forth by SEQ ID NO: 126. In some embodiments, the transmembrane domain is or comprises a sequence of amino acids that exhibits at least or about 85%, at least or about 90%, at least or about 92%, at least or about 95%, at least or about 97% sequence identity to the sequence set forth in SEQ ID NO: 126. In some such embodiments, the extracellular domain of the chimeric receptor signaling domain is an extracellular domain from IL4R, such as described above.

In some embodiments, the transmembrane domain is a heterologous transmembrane domain, such as any of a number of known transmembrane domains from native cell surface receptors. In some embodiments, where the extracellular domain is an extracellular domain of a native cytokine receptor that binds to the immunosuppressive cytokine as described above, the transmembrane domain is a non-native transmembrane domain that is not the transmembrane domain of the cytokine receptor. In some embodiments, the transmembrane domain is derived from a transmembrane domain from another receptor polypeptide that is a membrane-bound or is a transmembrane protein. In some embodiments, a transmembrane anchor domain from another protein on T cells can be used. Non-limiting examples of transmembrane domains derived from a natural source include those derived from (e.g., include at least the transmembrane domain or a portion thereof) the alpha, beta or zeta chain of the T-cell receptor, CD28, CD3 epsilon, CD45, CD4, CD5, CD8, CD9, CD 16, CD22, CD33, CD37, CD64, CD80, CD86, CD 134, CD137, CD 154.

In some embodiments, the transmembrane is derived from a synthetic source. For example, a synthetic transmembrane domain may be engineered to include predominantly hydrophobic residues, e.g., leucine and valine. In some embodiments, a synthetic transmembrane domain may be engineered to include a triplet of phenylalanine, tryptophan and valine at one or both ends of the synthetic transmembrane domain.

### C. Intracellular Signaling Domain, e.g. MyD88 Intracellular Signaling Domain

The provided chimeric signaling receptors have an intracellular signaling domain that contains one or more, e.g. two, MyD88 polypeptide that engages an immune cell activating pathway. In some embodiments, the extracellular domain of the chimeric signaling receptor that binds to the immunosuppressive cytokine is linked, e.g. via one or more transmembrane domain, to the intracellular signaling domain. Thus, in some embodiments, the extracellular domain that may normally engage an immunosuppressive pathway in the native cytokine receptor is linked to one or more, e.g. two, MyD88 polypeptide to instead engage an immune cell activating pathway.

In some embodiments, the intracellular domain comprising one or more MyD88 polypeptides that are capable of inducing interleukin-1 receptor-associated kinase signaling and/or binding or recruitment or phosphorylation of IRAK, e.g., IRAK4. In some embodiments, the intracellular domain comprises two MyD88 polypeptides. In some embodiments, the intracellular domain comprising two MyD88 polypeptides in tandem.

As described above, MyD88 is an adaptor signaling molecule that provides positive cell signaling to promote T cell activation, and is part of the Toll-like receptor (TLR) signaling pathway (see, **FIG. 1B****).** In the TLR pathway, a MyD88-dependent response occurs upon dimerization of TLRs, which recruits the MyD88 adaptor protein. MyD88 in turn recruits protein kinases IRAKs, including IRAK4, leading to their phosphorylation. Phosphorylated IRAKs may induce TRAF-6 signaling, thus resulting in T cell activation.

In some cases, the intracellular domain is or comprises one or more MyD88 polypeptide, e.g. two MyD88 polypeptides, that each includes: (1) a death domain (DD) or a portion thereof (e.g. amino acids 19-109, such as portion 54-109 of SEQ ID NO:128); (2) an intermediate domain (ID) or a portion thereof (e.g. amino acids 110-155 of SEQ ID NO:128); and (3) a termination immediate domain (TIR) or a portion thereof (e.g. amino acids 159-213 of SEQ ID NO:128). In some embodiments, each of the one or more MyD88 polypeptide sequence contains an N-terminal methionine. For instance, an N-terminal methionine may be required for start of translation. As such N-terminal methionines are commonly cleaved co- or post-translationally, such that the mature protein sequences disclosed herein are also contemplated as lacking the N-terminal methionine. In some embodiments, each of the one or more MyD88 polypeptide sequence lacks an N-terminal methionine.

In any of the provided embodiments, the intracellular domain contains two MyD88 polypeptides that each have the same MyD88 polypeptide sequence. In some embodiments, each of the MyD88 polypeptide sequences is a full-length MyD88 or is a functional variant thereof (e.g. set forth in SEQ ID NO:128). In some embodiments, each of the MyD88 polypeptide is a truncated portion of MyD88.

In some embodiments, the MyD88 polypeptide has the sequence of amino acids set forth in SEQ ID NO:128. In some embodiments, the MyD88 polypeptide is a functional variant of the sequence set forth in SEQ ID NO:128 sufficient to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine. In some embodiments, the functional variant has a sequence of amino acids that exhibits at least at or about 85%, at least at or about 90%, at least at or about 92%, at least at or about 95%, or at least at or about 98% sequence identity to the sequence of amino acids set forth in SEQ ID NO:128.

In some embodiments, the MyD88 polypeptide is a truncated portion of MyD88. In some embodiments, the MyD88 polypeptide is a truncated portion of the sequence set forth in SEQ ID NO:128. In some embodiments, the truncated portion is a contiguous sequence of amino acids of MyD88 that is sufficient to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine. In some embodiments, the truncated portion is a contiguous sequence of amino acids of MyD88 that includes the DD, ID, and a portion of a TIR domain. In some embodiments, the truncated portion is a contiguous sequence of amino acids of MyD88 that is between 100 amino acids and 194 amino acids in length, such as is between 150 and 180 amino acids in length. In some embodiments, the truncated portion is a contiguous sequence of amino acids of MyD88 that is 150, 151, 152, 153, 154, 155, 156, 157, 158, 159, 160, 161, 162, 163, 164, 165, 166, 167, 168, 169, 170, 171, 172, 173, 174, 175, 176, 178, 179 or 180 amino acids in length. In some embodiments, the truncated portion of MyD88 lacks the full TIR domain. In some embodiments, the truncated portion of MyD88 lacks between 120 and 138 of the contiguous C-terminal amino acids of the full-length TIR domain. In some embodiments, the truncated portion of MyD88 lacks 120, 121, 122, 123, 124, 125, 126, 127, 128, 129, 130, 131, 132, 133, 134, 135, 136, 137 or 138 of the contiguous C-terminal amino acids of the full-length TIR domain. In some embodiments, the MyD88 polypeptide contains a portion of the TIR domain that is or comprises at least a portion of the amino acid sequence of SEQ ID NO: 14. In some embodiments, the MyD88 polypeptide contains a portion of the TIR domain set forth in SEQ ID NO: 14.

In some embodiments, the MyD88 polypeptide is a truncated portion of MyD88 that includes the sequence set forth as amino acids 1-155 of the full-length MyD88, e.g. set forth in SEQ ID NO:128. In some embodiments, the MyD88 polypeptide is a truncated portion of MyD88 set forth as amino acids 1-155, 1-156, 1-157, 1-158, 1-159, 1-160, 1-161, 1-162, 1-163, 1-164, 1-165, 1-166, 1-167, 1-168, 1-169, 1-170, 1-171, 1-172, 1-173, 1-174, 1-175, 1-176, 1-177, 1-178, 1-179 or 1-180 of the full-length MyD88, e.g. set forth in SEQ ID NO:128. In some embodiments, the truncated portion of MyD88 set forth as amino acids 1-171 of full-length MyD88, e.g. set forth in SEQ ID NO:128. In some embodiments, the truncated portion of MyD88 set forth as amino acids 1-172 of full-length MyD88, e.g. set forth in SEQ ID NO:128. In some embodiments, the sequence may be a functional variant that exhibits at least at or about 85%, at least at or about 90%, at least at or about 92%, at least at or about 95%, or at least at or about 98% sequence identity to the sequence of amino acids of any of the foregoing and is sufficient to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine.

In some embodiments, the truncated portion of MyD88 includes the sequence set forth as amino acids 2-155 of the full-length MyD88, e.g. set forth in SEQ ID NO:128. In some embodiments, the truncated portion of MyD88 is set forth as amino acids 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179 or 2-180 of the full-length MyD88, e.g. set forth in SEQ ID NO:128. In some embodiments, the truncated portion of MyD88 is set forth as amino acids 2-171 of full-length MyD88, e.g. set forth in SEQ ID NO:128. In some embodiments, the truncated portion of MyD88 is set forth as amino acids 2-172 of full-length MyD88, e.g. set forth in SEQ ID NO:128. In some embodiments, the sequence may be a functional variant that exhibits at least at or about 85%, at least at or about 90%, at least at or about 92%, at least at or about 95%, or at least at or about 98% sequence identity to the sequence of amino acids of any of the foregoing and is sufficient to dimerize, recruit IRAK-4, and/or facilitate phosphorylation (e.g. activation) of IRAK4, such as when the chimeric signaling receptor is expressed from a cell and engaged by a ligand, e.g. immunosuppressive cytokine.

In some embodiments, the MyD88 polypeptide includes a DD, an ID, and a portion of a TIR domain. In some embodiments, the MyD88 polypeptide includes a DD, an ID, and a truncated TIR domain. In some embodiments, the MyD88 polypeptide includes a DD, an ID, and a full-length TIR domain. In some embodiments, the DD is or comprises the amino acid sequence of SEQ ID NO: 10. In some embodiments, the DD is encoded by the sequence of nucleotides set forth in SEQ ID NO: 11. In some embodiments, the ID is or comprises the amino acid sequence of SEQ ID NO: 12. In some embodiments, the ID is encoded by the sequence of nucleotides set forth in SEQ ID NO:13. In some embodiments, the TIR is or comprises at least a portion of the amino acid sequence of SEQ ID NO: 14. In some embodiments, the at least a portion of the amino acid sequence is encoded by a contiguous portion of a sequence of nucleotides set forth in SEQ ID NO:15. In some embodiments, the TIR is or comprises at least a portion of an amino acid sequence that exhibits at least or about 85%, 90%, 92%, 95%, or 97% sequence identity to SEQ ID NO: 14. In some embodiments, the TIR is a portion of the TIR domain set forth in SEQ ID NO: 14. In some embodiments, the T portion of the TIR domain is encoded by the sequence of nucleotides set forth in SEQ ID NO:15.

In some embodiments, the MyD88 polypeptide is or comprises the sequence of SEQ ID NO: 2. In some embodiments, the MyD88 polypeptide consists of or consists essentially of sequence of SEQ ID NO: 2. In some embodiments, the MyD88 polypeptide is or comprises a sequence that exhibits at least or about 85%, 90%, 92%, 95%, or 97% sequence identity to the sequence of SEQ ID NO: 2. In some embodiments, the MyD88 polypeptide consists of or consists essentially of a sequence that exhibits at least or about 85%, 90%, 92%, 95%, or 97% sequence identity to the sequence of SEQ ID NO: 2.

In some cases, for example when the intracellular domain includes two MyD88 polypeptides, the MyD88 polypeptides are arranged in tandem. For example, in some embodiments, the intracellular domain contains a first MyD88 polypeptide as described herein, and a second MyD88 polypeptide as described herein, where the first MyD88 polypeptide and second MyD88 polypeptide are linked directly or indirectly. In some embodiments, the first and second MyD88 polypeptide are linked directly. For example, in some embodiments, the N-terminus of the second MyD88 polypeptide is directly linked to the C-terminus of the first MyD88 polypeptide. Alternatively, in some embodiments, the first and second MyD88 polypeptides are connected through a linker. For example, in some embodiments, the N-terminus of the second MyD88 polypeptide is linked to the C-terminus of the first polypeptide by a linker. In some embodiments, the linker is a glycine/serine linker. In some embodiments, the linker sequence is the amino acid sequence set forth by SEQ ID NO: 47 ((GGGGS)n). In some embodiments, the linker sequence is the amino acid sequence set forth by SEQ ID NO: 47 ((GGGGS)n), where n is an integer between 1 and 4, inclusive. In some embodiments, the linker sequence is the amino acid sequence set forth by SEQ ID NO: 47 ((GGGGS)n), where n is an integer between 1 and 4, inclusive. In some embodiments, the linker sequence is the amino acid sequence set forth in SEQ ID NO:48 (GGGGS). In some embodiments, the linker sequence is the amino acid sequence set forth by SEQ ID NO: 85 (GGGGSGGGGS). In some embodiments, the linker sequence is the amino acid sequence set forth by SEQ ID NO: 49 (GGGGSGGGGSGGGGS). In some embodiments, the linker sequence is the amino acid sequence set forth by SEQ ID NO: 50 (GGGGSGGGGSGGGGSGGGGS).

In some embodiments, the first and second MyD88 polypeptides are identical. The sequence of the first and second MyD88 polypeptide can include any sequence of a MyD88 polypeptide as described herein. In some embodiments, each of the first and second MyD88 polypeptide is the same truncated portion of MyD88. In some embodiments, the first and second MyD88 polypeptides each comprise the sequence of amino acids set for by SEQ ID NO: 2. In some embodiments, the first and second MyD88 polypeptides each consist or consist essentially of the sequence of amino acids set for by SEQ ID NO: 2. In some embodiments, the first and second MyD88 polypeptides each comprise the same sequence that is a sequence that exhibits at least or about 85%, 90%, 92%, 95%, or 97% sequence identity to the sequence of SEQ ID NO: 2. In some embodiments, the first and second MyD88 polypeptides each consist or consist essentially of the same sequence that is a sequence that exhibits at least or about 85%, 90%, 92%, 95%, or 97% sequence identity to the sequence of SEQ ID NO: 2.

In some embodiments, the first and second MyD88 polypeptides are different. The sequence of the first and second MyD88 polypeptide can independently include any sequence of a MyD88 polypeptide as described herein. In some embodiments, each of the first and second MyD88 polypeptide is a truncated portion of MyD88. In some embodiments, one of the first or second MyD88 polypeptides is or comprises the sequence of amino acids set for by SEQ ID NO: 2, and the other of the first or second MyD88 polypeptide is or comprises a sequence that exhibits at least or about 85%, 90%, 92%, 95%, or 97% sequence identity to the sequence of SEQ ID NO: 2.

In some embodiments, the one or more MyD88 polypeptide, e.g. two MyD88 polypeptide, is linked to the transmembrane domain of the chimeric signaling receptor. In some embodiments, the one or more MyD88 polypeptide is linked indirectly to the transmembrane domain. For example, in some cases the N-terminus of the MyD88 polypeptide is linked directly to the C-terminus of the transmembrane domain. In some embodiments, the one or more MyD88 polypeptide of the intracellular domain of the chimeric signaling receptor is joined to the transmembrane domain through a linker. Thus, in some embodiments, the MyD88 polypeptide is linked indirectly to the transmembrane domain through a linker. For example, the N-terminus of one of the one or more MyD88 polypeptide, e.g. first MyD88 polypeptide, is linked to the C-terminus of the transmembrane domain by a linker sequence.

In some embodiments, the linker joining the one or more MyD88 polypeptide, e.g. two MyD88 polypeptide, to the transmembrane domain is a peptide linker. The linker may be 2-25 amino acids in length, such as 2, 3, 4, 5, 6, 7, 8, 9, 10, 11, 12, 13, 14, 15, 16, 17, 18, 19, 20, 21, 22, 23, 24, or 25 amino acids. In some embodiments, the linker sequence is the amino acid sequence set forth by SEQ ID NO: 47 ((GGGGS)n), where n is an integer between 1 and 4, inclusive. In some embodiments, the linker sequence is the amino acid sequence set forth in SEQ ID NO:48 (GGGGS). In some embodiments, the linker sequence is the amino acid sequence set forth by SEQ ID NO: 85 (GGGGSGGGGS). In some embodiments, the linker sequence is the amino acid sequence set forth by SEQ ID NO: 49 (GGGGSGGGGSGGGGS). In some embodiments, the linker sequence is the amino acid sequence set forth by SEQ ID NO: 50 (GGGGSGGGGSGGGGSGGGGS).

In some embodiments, the linker joining the one or more MyD88, e.g. two MyD88 polypeptide, to the transmembrane domain may be a naturally occurring sequence that is or includes a partial sequence of the intracellular domain of the cytokine receptor from which the extracellular domain or the transmembrane domain is derived. In some embodiments, the partial sequence of the intracellular domain of the cytokine receptor is a portion of the cytoplasmic domain, for example a non-functional portion of the cytoplasmic domain that does not confer inhibitory signaling activity. For instance, the partial sequence of the intracellular signaling domain of the native cytokine receptor is not a functional inhibitory signaling domain capable of mediating inhibitory signaling, such that it is not capable of being phosphorylated (activated) and/or of recruiting an inhibitory adaptor molecule such as a SMAD (e.g. SMAD2 or SMAD3) in response to ligand binding to the chimeric signaling receptor. In some embodiments, the partial sequence includes between 2 and 50, such as between 2 and 25, of the N-terminal amino acids of the cytoplasmic domain of the cytokine receptor.

In some embodiments, the linker joining the one or more MyD88 polypeptide, e.g. two MyD88, to the transmembrane domain is a partial sequence of the intracellular domain of TGFβR2 that is is a non-functioning portion of the cytoplasmic domain of TGFβR2, . In some embodiments, the partial sequence of the TGFβR2 cytoplasmic domain includes between 2 and 25 N-terminal acids of the TGFβR2 cytoplasmic domain, e.g. between 2 and 25 of the N-terminal amino acids of the TGFβR2 cytoplasmic domain set forth in SEQ ID NO:129. In some embodiments, the partial sequence of the TGFβR2 cytoplasmic domain is amino acids 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, or 1-25 of the TGFβR2 cytoplasmic domain, e.g. set forth in SEQ ID NO:129. In some embodiments, the partial sequence of the TGFβR2 cytoplasmic domain is or comprises the sequence of amino acids of SEQ ID NO: 24. In some embodiments, the partial sequence of the TGFβR2 cytoplasmic domain is or comprises the sequence of amino acids that exhibits at least or about 85%, 90%, 92%, 95%, or 97% sequence identity to SEQ ID NO: 24. In some embodiments, the partial sequence of the TGFβR2 cytoplasmic domain is set forth in SEQ ID NO: 24. In some embodiments, the partial sequence of the TGFβR2 cytoplasmic domain is encoded by the sequence of nucleotides set forth in SEQ ID NO:25. In some such embodiments, the extracellular domain of the chimeric receptor signaling domain is an extracellular domain from TGFβR2, such as described above. In some such embodiments, the transmembrane domain of the chimeric receptor signaling domain is an extracellular domain from TGFβR2, such as described above.

In some embodiments, the linker joining the one or more MyD88 polypeptide, e.g. two MyD88, to the transmembrane domain is a partial sequence of the intracellular domain of TGFβR1, such as a portion of the cytoplasmic domain that is a non-functioning portion of TGFβR1. In some embodiments, the partial sequence of the TGFβR1 cytoplasmic domain includes between 2 and 25 of the N-terminal contiguous amino acids of the TGFβR1 cytoplasmic domain, e.g. TGFβR1 cytoplasmic domain set forth in SEQ ID NO:130. In some embodiments, the partial sequence of the TGFβR1 cytoplasmic domain is amino acids 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, or 1-25 of the TGFβR1 cytoplasmic domain, e.g. full set forth in SEQ ID NO:130. In some embodiments, the partial sequence of the TGFβR1 cytoplasmic (intracellular) domain is or comprises the sequence of amino acids of SEQ ID NO: 8. In some embodiments, the partial sequence of the TGFβR1 cytoplasmic domain is or comprises the sequence of amino acids that exhibits at least or about 85%, 90%, 92%, 95%, or 97% sequence identity to SEQ ID NO: 1. In some embodiments, the partial sequence of the TGFβR1 cytoplasmic domain is set forth in SEQ ID NO: 8. In some embodiments, the partial sequence of the TGFβR1 cytoplasmic domain is encoded by the sequence of nucleotides set forth in SEQ ID NO:9. In some such embodiments, the extracellular domain of the chimeric receptor signaling domain is an extracellular domain from TGFβR1, such as described above. In some such embodiments, the transmembrane domain of the chimeric receptor signaling domain is an extracellular domain from TGFβR1, such as described above.

In some embodiments, the intracellular domain of the chimeric signaling receptor includes a partial sequence of the intracellular domain of IL10Ra, such as a portion of the cytoplasmic domain that is a non-functioning portion of IL10Ra, joining the one or more MyD88 polypeptide, e.g. two MyD88, to the transmembrane domain. In some embodiments, the partial sequence of the IL10Ra cytoplasmic domain is a portion of the cytoplasmic domain that includes 2-25 N-terminal contiguous amino acids of the full IL10Ra cytoplasmic domain, e.g. full IL10Ra cytoplasmic domain set forth in SEQ ID NO:121. In some embodiments, the partial sequence of the IL10Ra cytoplasmic domain is 2, 3, 4, 5, 6, 7, 8, 9, 10, 11, 12, 13, 14, 15, 16, 17, 18, 19, 20, 21, 22, 23, 24, or 25 N-terminal contiguous amino acids of the full IL10Ra cytoplasmic domain, e.g. full set forth in SEQ ID NO:121. In some such embodiments, the extracellular domain of the chimeric receptor signaling domain is an extracellular domain from IL10Ra, such as described above. In some such embodiments, the transmembrane domain of the chimeric receptor signaling domain is an extracellular domain from IL10Ra, such as described above.

In some embodiments, the intracellular domain of the chimeric signaling receptor includes a partial sequence of the intracellular domain of IL10Rb, such as a portion of the cytoplasmic domain that is a non-functioning portion of IL10Rb, joining the one or more MyD88 polypeptide, e.g. two MyD88, to the transmembrane domain. In some embodiments, the partial sequence of the IL10Rb cytoplasmic domain is a portion of the cytoplasmic domain that includes 2-25 N-terminal contiguous amino acids of the full IL10Rb cytoplasmic domain, e.g. full IL10Rb cytoplasmic domain set forth in SEQ ID NO:124. In some embodiments, the partial sequence of the IL10Rb cytoplasmic domain is 2, 3, 4, 5, 6, 7, 8, 9, 10, 11, 12, 13, 14, 15, 16, 17, 18, 19, 20, 21, 22, 23, 24, or 25 N-terminal contiguous amino acids of the full IL10Ra cytoplasmic domain, e.g. full set forth in SEQ ID NO:124. In some such embodiments, the extracellular domain of the chimeric receptor signaling domain is an extracellular domain from IL10Rb, such as described above. In some such embodiments, the transmembrane domain of the chimeric receptor signaling domain is an extracellular domain from IL10Rb, such as described above.

In some embodiments, the intracellular domain of the chimeric signaling receptor includes a partial sequence of the intracellular domain of IL4R, such as a portion of the cytoplasmic domain that is a non-functioning portion of IL4R, joining the one or more MyD88 polypeptide, e.g. two MyD88, to the transmembrane domain. In some embodiments, the partial sequence of the IL4R cytoplasmic domain is a portion of the cytoplasmic domain that includes 2-25 N-terminal contiguous amino acids of the full IL4R cytoplasmic domain, e.g. full IL4R cytoplasmic domain set forth in SEQ ID NO:127. In some embodiments, the partial sequence of the IL4R cytoplasmic domain is 2, 3, 4, 5, 6, 7, 8, 9, 10, 11, 12, 13, 14, 15, 16, 17, 18, 19, 20, 21, 22, 23, 24, or 25 N-terminal contiguous amino acids of the full IL10Ra cytoplasmic domain, e.g. full set forth in SEQ ID NO:127. In some such embodiments, the extracellular domain of the chimeric receptor signaling domain is an extracellular domain from IL4R, such as described above. In some such embodiments, the transmembrane domain of the chimeric receptor signaling domain is an extracellular domain from IL4R, such as described above.

In some embodiments, the linker sequence contains a cytoplasmic domain as described herein and a glycine/serine linker sequence such as described herein.

### D. Exemplary chimeric signaling receptor polypeptides

The chimeric signaling receptors include an extracellular domain, transmembrane domain, and an intracellular domain containing one or more MyD88 polypeptides as described herein, such as in Sections IA-IC. In some embodiments, the chimeric signaling receptors provided herein include from N- to C-terminus an extracellular domain capable of binding immunosuppressive or anti-inflammatory cytokines, as described in Section I-A, a transmembrane domain as described in Section I-B , and an intracellular domain as described in Section I-C. Any of the domains may be joined via a linker as described. In some embodiments, the chimeric signaling receptors provided herein include from N- to C-terminus an extracellular domain capable of binding immunosuppressive or anti-inflammatory cytokines, as described in Section I-A, a transmembrane domain as described in Section I-B, a linker sequence as described herein, and an intracellular domain as described in Section I-C.

In some embodiments, the chimeric signaling receptor contains a contiguous sequence of a native cytokine receptor of an immunosuppressive cytokine, e.g. TGFβ, IL10 or IL4, that contains the extracellular domain for binding the immunosuppressive cytokine and the transmembrane domain of the cytokine receptor, but in which at least a functional inhibitory signaling domain of the intracellular signaling domain of the cytokine receptor is replaced by an intracellular signaling domain containing one or more MyD88 polypeptide, e.g. two MyD88 polypeptides in tandem. In some embodiments, the intracellular domain of the chimeric signaling receptor can contain a partial sequence of the intracellular signaling domain of the native cytokine receptor, for example as a linker sequence between the transmembrane domain and the one or more MyD88 polypeptide, so long as the partial sequence is not a functional inhibitory signaling domain and is not capable of being phosphorylated (activated) and/or of recruiting an inhibitory adaptor molecule such as a SMAD (e.g. SMAD2 or SMAD3) in response to ligand binding to the chimeric signaling receptor. In some embodiments, the cytokine receptor is a TGFβR1 and the contiguous sequence of amino acids of the cytokine receptor that lacks a functional inhibitory signaling domain is set forth in SEQ ID NO:1. In some embodiments, the cytokine receptor is a TGFβR2 and the contiguous sequence of amino acids of the cytokine receptor that lacks a functional inhibitory signaling domain is set forth in SEQ ID NO:3.

In some embodiments, the one or more MyD88 polypeptides of the intracellular signaling domain includes at least one MyD88 domain having the sequence set forth in SEQ ID NO:2. In some embodiments, the one or more MyD88 polypeptides of the intracellular domain are two MyD88 domains joined in tandem. In some embodiments, the two MyD88 domains are directly linked. In some embodiments, the two MyD88 domains are linked though a linker as described herein. In some embodiments, the two MyD88 domains linked in tandem each individually have the sequence set forth in SEQ ID NO:2. The linker between each MyD88 domain can be any as described.

In some embodiments, the chimeric signaling receptor binds to TGFβ. In some embodiments, the chimeric signaling receptors provided herein include from N- to C-terminus an extracellular domain capable of binding to TGFβ, as described in Section I-A, a transmembrane domain as described in Section I-B, and an intracellular domain as described in Section I-C. In some embodiments, the chimeric signaling receptors provided herein include from N- to C-terminus an extracellular domain capable of binding to TGFβ, as described in Section I-A, a transmembrane domain as described in Section I-B, a linker sequence as described herein, and an intracellular domain as described in Section I-C.

In some embodiments, the chimeric signaling receptor comprises a signal peptide to facilitate localization to the cell membrane for expression on the cell surface. In some embodiments, the signal peptide is present in a precursor chimeric signaling receptor protein and is cleaved to form a the mature chimeric signaling receptor. In some embodiments, the signal peptide is a CD8α signal peptide. In some embodiments, the signal peptide is or comprises the amino acid sequence set forth by SEQ ID NO: 102.

In some embodiments, the chimeric signaling receptors described herein contain, from N- to C-terminus: an extracellular domain that is the extracellular domain of a TGFβR, or a portion thereof that binds TGFβ; a transmembrane domain that is a TGFβR transmembrane domain or a portion thereof; a linker sequence as described herein; and an intracellular signaling domain containing at least one MyD88 domain, such as any as described. In some embodiments, the MyD88 domain as the sequence set forth in SEQ ID NO:2. In some embodiments, the chimeric signaling receptors described herein contain, from N- to C-terminus: an extracellular domain that is the extracellular domain of a TGFβR, or a portion thereof that binds TGFβ; a transmembrane domain that is a TGFβR transmembrane domain or a portion thereof; a linker sequence as described herein; and an intracellular signaling domain comprising two MyD88 domains in tandem, which each individually is any as described. In some embodiments, the two MyD88 domains are directly linked. In some embodiments, the two MyD88 domains are linked though a linker as described herein. In some embodiments, the two MyD88 domains linked in tandem each individually have the sequence set forth in SEQ ID NO:2. The linker between each MyD88 domain can be any as described.

In some embodiments, the chimeric signaling receptor comprises from N- to C-terminus: a transmembrane domain that is an extracellular domain of TGFβR1, or a portion thereof that binds TGFβ; a transmembrane domain that is a TGFβR1 transmembrane domain or a portion thereof; a linker sequence as described herein; and an intracellular signaling domain containing at least one MyD88 domain, such as any as described. In some embodiments, the MyD88 domain as the sequence set forth in SEQ ID NO:2.

In some embodiments, the chimeric signaling receptor described herein contains the sequence of amino acids set forth in SEQ ID NO:1 joined to the sequence of amino acids set forth in SEQ ID NO:2. In some embodiments, the sequence set forth in SEQ ID NO:1 and the sequence set forth in SEQ ID NO:2 are directly linked. In some embodiments, the chimeric signaling receptor is or comprises the sequence of amino acids set forth by SEQ ID NO: 90. In some embodiments, the chimeric signaling receptor is or comprises a sequence of amino acids that exhibits at least or about 85%, 90%, 92%, 95%, or 97% sequence identity to sequence of SEQ ID NO: 90. In some embodiments, the chimeric signaling receptor includes a signal peptide (e.g. SEQ ID NO:102), which, in some cases can be cleaved when expressed from a cell. In some embodiments, the chimeric signaling receptor is or comprises the sequence of amino acids set forth by SEQ ID NO: 16. In some embodiments, the chimeric signaling receptor is or comprises a sequence of amino acids that exhibits at least or about 85%, 90%, 92%, 95%, or 97% sequence identity to sequence of SEQ ID NO: 16. In some embodiments, the chimeric signaling receptor is encoded by the sequence of nucleotides set forth by SEQ ID NO: 17. In some embodiments, the chimeric signaling receptor is encoded by a sequence of nucleotides that exhibits at least or about 85%, 90%, 92%, 95%, or 97% sequence identity to sequence of SEQ ID NO: 17.

In some embodiments, the chimeric signaling receptor is or comprises the sequence of amino acids set forth by SEQ ID NO: 91. In some embodiments, the chimeric signaling receptor is or comprises a sequence of amino acids that exhibits at least or about 85%, 90%, 92%, 95%, or 97% sequence identity to sequence of SEQ ID NO: 91. In some embodiments, the chimeric signaling receptor includes a signal peptide (e.g. SEQ ID NO:102), which, in some cases can be cleaved when expressed from a cell. In some embodiments, the chimeric signaling receptor is or comprises the sequence of amino acids set forth by SEQ ID NO: 18. In some embodiments, the chimeric signaling receptor is or comprises a sequence of amino acids that exhibits at least or about 85%, 90%, 92%, 95%, or 97% sequence identity to sequence of SEQ ID NO: 18. In some embodiments, the chimeric signaling receptor is encoded by the sequence of nucleotides set forth by SEQ ID NO: 19. In some embodiments, the chimeric signaling receptor is encoded by a sequence of nucleotides that exhibits at least or about 85%, 90%, 92%, 95%, or 97% sequence identity to sequence of SEQ ID NO: 19.

In some embodiments, the chimeric signaling receptor comprises from N- to C-terminus: a transmembrane domain that is an extracellular domain of TGFβR1, or a portion thereof that binds TGFβ; a transmembrane domain that is a TGFβR1 transmembrane domain or a portion thereof; a linker sequence as described herein; and an intracellular signaling domain containing two MyD88 domains in tandem, which each individually is any as described. In some embodiments, the two MyD88 domains are directly linked. In some embodiments, the two MyD88 domains are linked though a linker as described herein. In some embodiments, the two MyD88 domains linked in tandem each individually have the sequence set forth in SEQ ID NO:2. The linker between each MyD88 domain can be any as described.

In some embodiments, the chimeric signaling receptor comprises from N- to C-terminus: a transmembrane domain that is an extracellular domain of TGFβR2, or a portion thereof that binds TGFβ; a transmembrane domain that is a TGFβR2 transmembrane domain or a portion thereof; a linker sequence as described herein; and an intracellular signaling domain containing at least one MyD88 domains such as any as described. In some embodiments, the MyD88 domain as the sequence set forth in SEQ ID NO:2.

In some embodiments, the chimeric signaling receptor described herein contains the sequence of amino acids set forth in SEQ ID NO:3 joined to the sequence of amino acids set forth in SEQ ID NO:2. In some embodiments, the sequence set forth in SEQ ID NO:3 and the sequence set forth in SEQ ID NO:2 are directly linked. In some embodiments, the chimeric signaling receptor is or comprises the sequence of amino acids set forth by SEQ ID NO: 92. In some embodiments, the chimeric signaling receptor is or comprises a sequence of amino acids that exhibits at least or about 85%, 90%, 92%, 95%, or 97% sequence identity to sequence of SEQ ID NO: 92. In some embodiments, the chimeric signaling receptor includes a signal peptide (e.g. SEQ ID NO:102), which, in some cases can be cleaved when expressed from a cell. In some embodiments, the chimeric signaling receptor is or comprises the sequence of amino acids set forth by SEQ ID NO: 26. In some embodiments, the chimeric signaling receptor is or comprises a sequence of amino acids that exhibits at least or about 85%, 90%, 92%, 95%, or 97% sequence identity to sequence of SEQ ID NO: 26. In some embodiments, the chimeric signaling receptor is encoded by the sequence of nucleotides set forth by SEQ ID NO: 27. In some embodiments, the chimeric signaling receptor is encoded by a sequence of nucleotides that exhibits at least or about 85%, 90%, 92%, 95%, or 97% sequence identity to sequence of SEQ ID NO: 27.

In some embodiments, the chimeric signaling receptor comprises from N- to C-terminus: a transmembrane domain that is an extracellular domain of TGFβR2, or a portion thereof that binds TGFβ; a transmembrane domain that is a TGFβR2 transmembrane domain or a portion thereof; a linker sequence as described herein; and an intracellular signaling domain containing two MyD88 domains in tandem, which each individually is any as described. In some embodiments, the two MyD88 domains are directly linked. In some embodiments, the two MyD88 domains are linked though a linker as described herein. In some embodiments, the two MyD88 domains linked in tandem each individually have the sequence set forth in SEQ ID NO:2. The linker between each MyD88 domain can be any as described.

In some embodiments, the chimeric signaling receptor is or comprises the sequence of amino acids set forth by SEQ ID NO: 93. In some embodiments, the chimeric signaling receptor is or comprises a sequence of amino acids that exhibits at least or about 85%, 90%, 92%, 95%, or 97% sequence identity to sequence of SEQ ID NO: 93. In some embodiments, the chimeric signaling receptor includes a signal peptide (e.g. SEQ ID NO:102), which, in some cases can be cleaved when expressed from a cell. In some embodiments, the chimeric signaling receptor is or comprises the sequence of amino acids set forth by SEQ ID NO: 28. In some embodiments, the chimeric signaling receptor is or comprises a sequence of amino acids that exhibits at least or about 85%, 90%, 92%, 95%, or 97% sequence identity to sequence of SEQ ID NO: 28. In some embodiments, the chimeric signaling receptor is encoded by the sequence of nucleotides set forth by SEQ ID NO: 29. In some embodiments, the chimeric signaling receptor is encoded by a sequence of nucleotides that exhibits at least or about 85%, 90%, 92%, 95%, or 97% sequence identity to sequence of SEQ ID NO: 29.

In some embodiments, the chimeric signaling receptor is or comprises the sequence of amino acids set forth by SEQ ID NO: 94. In some embodiments, the chimeric signaling receptor is or comprises a sequence of amino acids that exhibits at least or about 85%, 90%, 92%, 95%, or 97% sequence identity to sequence of SEQ ID NO: 94. In some embodiments, the chimeric signaling receptor includes a signal peptide (e.g. SEQ ID NO:102), which, in some cases can be cleaved when expressed from a cell. In some embodiments, the chimeric signaling receptor is or comprises the sequence of amino acids set forth by SEQ ID NO: 30. In some embodiments, the chimeric signaling receptor is or comprises a sequence of amino acids that exhibits at least or about 85%, 90%, 92%, 95%, or 97% sequence identity to sequence of SEQ ID NO: 30. In some embodiments, the chimeric signaling receptor is encoded by the sequence of nucleotides set forth by SEQ ID NO: 31. In some embodiments, the chimeric signaling receptor is encoded by a sequence of nucleotides that exhibits at least or about 85%, 90%, 92%, 95%, or 97% sequence identity to sequence of SEQ ID NO: 31.

In some embodiments, for purposes of aiding in the detection or quantification of chimeric signaling receptor expression, the chimeric signaling receptor further comprises a detectable moiety or marker. In some embodiments, the detectable marker is a myc-tag (SEQ ID NO: 101). In some embodiments, the detectable marker is a polyhistidine tag (SEQ ID NO: 103). In some embodiments, the detectable marker is located at the N-terminus of the extracellular domain of the chimeric signaling receptor.

In some cases, the chimeric signaling receptors provided herein contain an cleavable peptide or ribosomal skip element, such as an IRES or T2A at the N-terminus or C-terminus of the chimeric signaling receptor polypeptide. In some embodiments, inclusion of the cleavable peptide or ribosomal skip element allows for expression of two or more polypeptides from a single polypeptide. See, for example, Section II below. In some embodiments, the cleavable peptide is a T2A. In some embodiments, the T2A is or comprises the amino acid sequence set forth by SEQ ID NO: 61. In some embodiments, the T2A is or comprises the amino acid sequence set forth by SEQ ID NO: 62. In some embodiments, the T2A is or comprises the amino acid sequence set forth by SEQ ID NO: 113. In some embodiments, the T2A is or comprises the amino acid sequence set forth by SEQ ID NO: 114.

In some embodiments, the chimeric signaling receptor is or comprises the sequence of amino acids set forth by SEQ ID NO: 98. In some embodiments, the chimeric signaling receptor is or comprises a sequence of amino acids that exhibits at least or about 85%, 90%, 92%, 95%, or 97% sequence identity to sequence of SEQ ID NO: 98.

In some embodiments, the chimeric signaling receptor is or comprises the sequence of amino acids set forth by SEQ ID NO: 99. In some embodiments, the chimeric signaling receptor is or comprises a sequence of amino acids that exhibits at least or about 85%, 90%, 92%, 95%, or 97% sequence identity to sequence of SEQ ID NO: 99.

In some embodiments, the chimeric signaling receptor is or comprises the sequence of amino acids set forth by SEQ ID NO: 39. In some embodiments, the chimeric signaling receptor is or comprises a sequence of amino acids that exhibits at least or about 85%, 90%, 92%, 95%, or 97% sequence identity to sequence of SEQ ID NO: 39.

In some embodiments, the chimeric signaling receptor is or comprises the sequence of amino acids set forth by SEQ ID NO: 41. In some embodiments, the chimeric signaling receptor is or comprises a sequence of amino acids that exhibits at least or about 85%, 90%, 92%, 95%, or 97% sequence identity to sequence of SEQ ID NO: 41.

### II. NUCLEIC ACIDS AND VECTORS

Also provided are polynucleotides (nucleic acid molecules) encoding the chimeric signaling receptors and vectors for genetically engineering cells to express such polypeptides and receptors.

### A. Polynucleotides

In some embodiments, provided are polynucleotides that encode any of the chimeric signaling receptors provided herein. In some aspects, the polynucleotide contains a single nucleic acid sequence, such as a nucleic acid sequence encoding the chimeric signaling receptor. As described above, for example in Section I-D, in some cases, the polynucleotide contains a signal sequence that encodes a signal peptide. For example, the signal sequence may encode a signal peptide derived from CD8, such as a CD8α signal sequence. In some embodiments, the CD8α signal peptide is or comprises the sequence set forth in SEQ ID NO: 102, or a sequence that has at least 95% sequence identity to such a sequence.

In some embodiments, the polynucleotide contains a first nucleic acid sequence encoding the chimeric signaling receptor and a second nucleic acid sequence encoding another recombinant molecule, such as a secretable recombinant molecule, for example a cytokine (e.g. IL-15 or IL-12) or bispecific antibody (e.g. BiTE). In some embodiments, the polynucleotide contains a first nucleic acid sequence encoding the chimeric signaling receptor and a second nucleic acid sequence encoding a recombinant antigen receptor. In some aspects, the recombinant antigen receptor is or contains a chimeric antigen receptor (CAR). In some aspects, the recombinant antigen receptor is or contains a T cell receptor (TCR), e.g., a transgenic TCR. Exemplary recombinant antigen receptors are described in Section III. In some embodiments, the polynucleotide contains a first nucleic acid sequence encoding the chimeric signaling receptor and a second nucleic acid sequence encoding another recombinant molecule that is secretable, such as a bispecific antibody or a cytokine. In some aspects, the bispecific antibody is a BiTE. Exemplary recombinant secretable molecules, including cytokines and BiTEs, are described in Section IV.

In some embodiments, the polynucleotide encoding the chimeric signaling receptor contains at least one promoter that is operatively linked to control expression of the chimeric signaling receptor. In some embodiments, the polynucleotide contains two, three, or more promoters operatively linked to control expression of the chimeric signaling receptor.

In some embodiments, for example when the polynucleotide contains two or more nucleic acid coding sequences, such as a sequence encoding a chimeric signaling receptor and a sequence encoding another recombinant molecule, such as a recombinant antigen receptor, e.g., CAR or a recombinant TCR, cytokine, or a bispecific antibody, e.g. a BiTE, at least one promoter is operatively linked to control expression of the two or more nucleic acid sequences. In some embodiments, the polynucleotide contains two, three, or more promoters operatively linked to control expression of the chimeric signaling receptor and/or the recombinant antigen receptor.

In some embodiments, expression of the chimeric signaling receptor is inducible or conditional. In some embodiments, expression of the chimeric signaling receptor and recombinant antigen receptor is inducible or conditional. Thus, in some aspects, the polynucleotide encoding the chimeric signaling receptor and/or recombinant antigen receptor contains a conditional promoter, enhancer, or transactivator. In some such aspects, the conditional promoter, enhancer, or transactivator is an inducible promoter, enhancer, or transactivator or a repressible promoter, enhancer, or transactivator. For example, in some embodiments, an inducible or conditional promoter can be used to restrict expression of the chimeric signaling receptor and/or recombinant antigen receptor to a specific microenvironment, such as a tumor microenvironment. In some embodiments, the inducible or conditional promoter is active in the presence of one or more conditions in the tumor microenvironment, such as hypoxia, low glucose, acidic pH, and/or oxidative stress. In other embodiments, expression driven by the inducible or conditional promoter is regulated by exposure to an exogenous agent, such as heat, radiation, or drug.

In some embodiments, the polynucleotide encoding the chimeric signaling receptor is operably linked to an EF1α promoter. In some embodiments, the polynucleotide encoding the chimeric signaling receptor and recombinant antigen receptor are operably linked to a single EF1α promoter. In some embodiments, the polynucleotide encoding the chimeric signaling receptor and secretable recombinant molecule, such as a cytokine or bispecific antibody (e.g. BiTE), are operably linked to a single EF1α promoter. In some embodiments, the EF1α promoter is or comprises the nucleic acid sequence set forth by SEQ ID NO: 112.

In cases where the polynucleotide contains more than one nucleic acid sequence encoding a protein, e.g., a first nucleic acid sequence encoding a chimeric signaling receptor and a second nucleic acid sequence encoding another recombinant molecule, such as a recombinant antigen receptor (e.g., CAR, TCR) or bispecific antibody (e.g. BiTE) or vice versa, the polynucleotide may further include a nucleic acid sequence encoding a peptide between the first and second nucleic acid sequences. In some cases, the nucleic acid positioned between the first and second nucleic acid sequence encodes a peptide that separates the translation products of the first and second nucleic acid sequences during or after translation. In some embodiments, the peptide contains an internal ribosome entry site (IRES), a self-cleaving peptide, or a peptide that causes ribosome skipping, such as a T2A peptide. In some embodiments, inclusion of the cleavable peptide or ribosomal skip element allows for expression of two or more polypeptides from a single polypeptide. In some embodiments, the peptide is a self-cleaving peptide that is a T2A peptide. In some embodiments, the T2A is or comprises the amino acid sequence set forth by SEQ ID NO: 61. In some embodiments, the T2A is or comprises the amino acid sequence set forth by SEQ ID NO: 62. In some embodiments, the T2A is or comprises the amino acid sequence set forth by SEQ ID NO: 113. In some embodiments, the T2A is or comprises the amino acid sequence set forth by SEQ ID NO: 114.

In some embodiments, the polynucleotide encodes a chimeric signaling receptor and a CAR directed against or specific to an antigen (e.g. tumor antigen, such as CD19 or B7H3), in which the nucleic acid encoding the chimeric signaling receptor and the nucleic acid encoding the CAR are separated by a T2A self-cleaving peptide.

In some embodiments, the polynucleotide is or comprises a sequence of nucleotides that encodes the sequence set forth by SEQ ID NO: 104. In some embodiments, the polynucleotide is or comprises a sequence of nucleotides that encodes a sequence that exhibits at least or about 85%, 90%, 92%, 95%, or 97% sequence identity to sequence of SEQ ID NO: 104.

In some embodiments, the polynucleotide is or comprises the sequence of nucleotides that encodes the sequence set forth by SEQ ID NO: 105. In some embodiments, the polynucleotide is or comprises a sequence of nucleotides that encodes a sequence that exhibits at least or about 85%, 90%, 92%, 95%, or 97% sequence identity to sequence of SEQ ID NO: 105.

In some embodiments, the polynucleotide is or comprises the sequence of nucleotides that encodes the sequence set forth by SEQ ID NO: 106. In some embodiments, the polynucleotide is or comprises a sequence of nucleotides that encodes a sequence that exhibits at least or about 85%, 90%, 92%, 95%, or 97% sequence identity to sequence of SEQ ID NO: 106.

In some embodiments, the polynucleotide is or comprises the sequence of nucleotides that encodes the sequence set forth by SEQ ID NO: 107. In some embodiments, the polynucleotide is or comprises a sequence of nucleotides that encodes a sequence that exhibits at least or about 85%, 90%, 92%, 95%, or 97% sequence identity to sequence of SEQ ID NO: 107.

In some embodiments, the polynucleotide encoding the chimeric signaling receptor is introduced into a composition containing cultured cells, such as by retroviral transduction, transfection, or transformation. In some embodiments, the polynucleotide encoding the chimeric signaling receptor and recombinant antigen receptor is introduced into a composition containing cultured cells, such as by retroviral transduction, transfection, or transformation.

In some embodiments, the polynucleotide (nucleic acid molecule) provided herein encodes a chimeric signaling receptor as described herein. In some embodiments, the polynucleotide provided herein encodes a chimeric signaling receptor and a recombinant antigen receptor, e.g., CAR, TCR. In addition, in some embodiments, a polynucleotide encoding a recombinant antigen receptor, e.g., CAR or TCR, is provided herein.

### B. Vectors

Also provided are vectors or constructs containing the polynucleotide, such as nucleic acid molecules as described herein. In some embodiments, the vectors or constructs contain one or more promoters operatively linked to the nucleic acid molecule encoding the chimeric signaling receptor to drive expression thereof. In some embodiments, the promoter is operatively linked to one or more than one nucleic acid molecule, e.g., the nucleic acid molecule encoding the chimeric signaling receptor and a nucleic acid molecule encoding a recombinant antigen receptor, e.g., CAR, recombinant TCR as described below.

In some embodiments, the vector is a viral vector. In some embodiments the viral vector is a retroviral vector. In some embodiments, the retroviral vector is a lentiviral vector. In some embodiments, the retroviral vector is a gammaretroviral vector. In some embodiments, the viral vector containing the polynucleotide encoding the chimeric signaling is introduced into a composition containing cultured cells, such as by retroviral transduction (e.g. lentiviral transduction). In some embodiments, the polynucleotide encoding the chimeric signaling receptor and another recombinant molecule, such as a recombinant antigen receptor and/or secretable recombinant molecule (e.g. bispecific antibody or cytokine) is introduced into a composition containing cultured cells, such as by retroviral transduction (e.g. lentiviral transduction).

In some embodiments, the vector or construct includes a single promoter that drives the expression of one or more nucleic acid molecules of the polynucleotide. In some embodiments, such promoters can be multicistronic (bicistronic or tricistronic, see e.g., U.S. Patent No. 6,060,273). For example, in some embodiments, transcription units can be engineered as a bicistronic unit containing an IRES (internal ribosome entry site), which allows coexpression of gene products (e.g. encoding a chimeric signaling receptor and a recombinant antigen receptor) by a message from a single promoter. In some embodiments, the vectors provided herein are bicistronic, allowing the vector to contain and express two nucleic acid sequences. In some cases, the two nucleic acid sequences of the bicistronic vector are a chimeric signaling receptor and a CAR. In some embodiments, the vectors provided herein are tricistronic, allowing the vector to contain and express three nucleic acid sequences. In some cases, the three nucleic acid sequences of the tricistronic vector are a chimeric signaling receptor and nucleic acid molecules encoding the alpha and beta chains of a recombinant TCR.

In some embodiments, a single promoter directs expression of an RNA that contains, in a single open reading frame (ORF), two or three genes (e.g. encoding the chimeric signaling receptor and encoding a recombinant antigen receptor) separated from one another by sequences encoding a self-cleavage peptide (e.g., 2A sequences) or a protease recognition site (e.g., furin). The ORF thus encodes a single polypeptide, which, either during (in the case of 2A) or after translation, is processed into the individual proteins. In some cases, the peptide, such as T2A, can cause the ribosome to skip (ribosome skipping) synthesis of a peptide bond at the C-terminus of a 2A element, leading to separation between the end of the 2A sequence and the next peptide downstream (see, for example, de Felipe. Genetic Vaccines and Ther. 2:13 (2004) and deFelipe et al. Traffic 5:616-626 (2004)). Many 2A elements are known in the art. Examples of 2A sequences that can be used in the methods and nucleic acids disclosed herein include, without limitation, 2A sequences from the foot-and-mouth disease virus (F2A, e.g., SEQ ID NO: 66), equine rhinitis A virus (E2A, e.g., SEQ ID NO: 65), Thosea asigna virus (T2A, e.g., SEQ ID NO: 61, 62, 113, or 114), and porcine teschovirus-1 (P2A, e.g., SEQ ID NO: 63 or 64) as described in U.S. Patent Publication No. 20070116690.

In some embodiments, engineered cells (e.g. T cells) introduced with the chimeric signaling receptor are selected for cells that are positive for surface expression of the chimeric signaling receptor. In some embodiments, surface expression can be assessed by detecting or selecting for cells using a reagent (e.g. antibody or other immunoaffinity reagent) specific to the extracellular binding domain of the chimeric signaling receptor. For instance, surface expression can be assessed by detecting or selecting for cells specific for TGFBR.

In some embodiments in which cells are also introduced with a nucleic acid encoding a recombinant antigen receptor (e.g. CAR or TCR), cells may be selected for cells that are positive for surface expression of the recombinant antigen receptor. In some embodiments, cells may be selected that are positive for both the chimeric signaling receptor and the recombinant antigen receptor. In some embodiments, surface expression can be assessed by detecting or selecting cells using a reagent (e.g. antibody or other immunoaffinity reagent) specific to the extracellular domain of the recombinant antigen receptor. For instance, such reagents include, for example, a target antigen specific to the CAR, an anti-idiotypic antibody specific to the antigen-binding domain of the CAR, or a reagent that binds to a portion of an immunoglobulin, such as protein L or other anti-immunoglobulin antibody. In other aspects for detection of a recombinant or engineered T cell receptor (TCR) may be by staining with an MHC tetramer or with an anti-TCR alpha or beta chain antibody.

A number of well-known methods for assessing expression level of surface markers or proteins may be used, such as detection by affinity-based methods, e.g., immunoaffinity-based methods, e.g., in the context of surface markers, such as by flow cytometry. In some embodiments, the label is a fluorophore and the methods for detection or identification of surface markers (e.g. chimeric signaling receptor or recombinant antigen receptor) is by flow cytometry. In some embodiments, different labels are used for each of the different markers by multicolor flow cytometry.

In some embodiments, a composition containing engineered cells, such as engineered T cells, is produced in which a plurality of the cells express the chimeric signaling receptor. In some embodiments, at least at or about 50%, at least at or about 60%, at least at or about 70%, a least at or about 80%, at least at or about 90% or at least at or about 95% of the cells in the composition express the chimeric signaling receptor. In some embodiments, a composition containing engineered cells, such as engineered T cells, is produced in which a plurality of cells express the chimeric signaling receptor and a recombinant antigen receptor (e.g. CAR or TCR). In some embodiments, at least at or about 50%, at least at or about 60%, at least at or about 70%, a least at or about 80%, at least at or about 90% or at least at or about 95% of the cells in the composition express the chimeric signaling receptor and the recombinant antigen receptor (e.g. CAR or TCR). In particular embodiments, at least at or about 80% of the cells in the composition express the chimeric signaling receptor and the recombinant antigen receptor (e.g. CAR or TCR). In some embodiments, the cells are T cells. In some of any embodiments, the engineered cells may also produce, such as a secrete, another recombinant molecule, such as a cytokine (e.g. IL-12 or IL-15) or a bispecific antibody (e.g. a BiTE).

### III. Recombinant Antigen Receptor

As described herein, in some cases, a chimeric signaling receptor provided herein is co-expressed in a cell, e.g. T cell, with a recombinant antigen receptor. In some embodiments, the recombinant antigen receptor is a chimeric antigen receptor (CAR). In some embodiments, the recombinant antigen receptor is a T cell receptor.

### A. Chimeric Antigen Receptor

In some embodiments, the recombinant antigen receptor is a chimeric antigen receptor (CAR). In some embodiments, the chimeric signaling receptor and the CAR are expressed in a cell engineered, e.g., with a polynucleotide or vector described herein, to express the chimeric signaling receptor and the CAR on the cell surface. In some embodiments, the chimeric signaling receptor and the CAR are contained in two separate polynucleotides, for example as describes in Section II-A. In some embodiments, the chimeric signaling receptor and the CAR are contained in a single polynucleotide, for example as describes in Section II-A.

The CARs provided herein are genetically engineered receptors including an extracellular antigen binding domain and one or more intracellular signaling domains, such that the chimeric antigen receptor mimics activation through an antigen receptor complex, such as a TCR complex. In some embodiments, the CAR further contains a transmembrane domain and/or intracellular domain, in some embodiments, the extracellular domain is linked to the intracellular signaling domain through the transmembrane domain and/or intracellular domain. The CAR is typically engineered such that the domains, e.g., extracellular antigen binding domain, transmembrane domain, intracellular domain, and intracellular signaling domain function to produce a signal akin to a signal received via a naturally occurring antigen receptor, including, for example, a costimulatory receptor.

In some embodiments, the CAR is engineered to bind, e.g., specifically bind, via the extracellular antigen binding domain contained in the CAR to an antigen. The antigen may be referred to herein as a target antigen.

Exemplary target antigens include, but are not limited to, αvβ6 integrin (avb6 integrin), B cell maturation antigen (BCMA), B7-H6, B7H3, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD123, CD138, CD171, epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, fetal acetylcholine receptor, ganglioside GD2, O-acetylated GD2 (OGD2), ganglioside GD3, glycoprotein 100 (gp100), Her2/neu (receptor tyrosine kinase erbB2), Her3 (erb-B3), Her4 (erb-B4), erbB dimers, Human high molecular weight-melanoma-associated antigen (HMW-MAA), hepatitis B surface antigen, Human leukocyte antigen A1 (HLA-AI), Human leukocyte antigen A2 (HLA-A2), IL-22 receptor alpha(IL-22Ra), IL-13 receptor alpha 2 (IL-13Ra2), kinase insert domain receptor (kdr), kappa light chain, L1 cell adhesion molecule (L1CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, mesothelin, c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), vascular endothelial growth factor receptor (VEGFR), vascular endothelial growth factor receptor 2 (VEGFR2), Wilms Tumor 1 (WT-1), a pathogen-specific antigen, or an antigen associated with a universal tag, and/or biotinylated molecules, and/or molecules expressed by HIV, HCV, HBV or other pathogens.

In some embodiments, the target antigen is selected from the group consisting of: CD3, NKp46, CD5, CD19; CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRviii); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAca-Ser/Thr)); prostate-specific membrane antigen (PSMA); Receptor tyrosine kinase-like orphan receptor 1 (ROR1); FmsLike Tyrosine Kinase 3 (FLT3); Tumor-associated glycoprotein 72 (TAG72); CD38; CD44v6; a glycosylated CD43 epitope expressed on acute leukemia or lymphoma but not on hematopoietic progenitors, a glycosylated CD43 epitope expressed on non-hematopoietic cancers, Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-llRa); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAlX); Proteasome (Prosome, Macropain) Subunit, Beta Type, 9 (LMP2); glycoprotein 100 (gpl00); oncogene fusion protein consisting of breakpoint cluster region (BCR) and Abelson murine leukemia viral oncogene homolog 1 (Abl) (bcr- abl); tyrosinase; ephrin type-A receptor 2 (EphA2); Fucosyl GM1; sialyl Lewis adhesion molecule (sLe); ganglioside GM3 (aNeu5Ac(2-3)bDClalp(1-4)bDGlcp(1-1)Cer); transglutaminase 5 (TGS5); high molecular weight-melanomaassociated antigen (HMWMAA); o-acetyl-GD2 ganglioside (OAcGD2); tumor endothelial marker 1; (TEM1/CD248); tumor endothelial marker 7-related (TEM7R); claudin 6 (CLDN6); thyroid stimulating hormone receptor (TSHR); G protein coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ES0-1); Cancer/testis antigen 2 (LAGE-1a); Melanoma- associated antigen 1 (MAGE-A1); ETS translocation-variant gene 6, located on chromosome 12p (ETV6-AML); sperm protein 17 (SPA17); X Antigen Family, Member 1A (XAGEl); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCT A-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl- transferase V (NA17); paired box protein Pax-3 (PAX3); Androgen receptor; Cyclin B1; v- myc avian myelocytomatosis viral oncogene neuroblastoma derived homolog (MYCN); Ras Homolog Family Member C (RhoC); Tyrosinase-related protein 2 (TRP-2); Cytochrome P450 1B 1 (CYPlB 1 ); CCCTC-Binding Factor (Zinc Finger Protein)-Like (BORIS or Brother of the Regulator oflmprinted Sites), Squamous Cell Carcinoma Antigen Recognized By T Cells 3 (SART3); Paired box protein Pax-5 (PAX5); proacrosin binding protein sp32 (OY-TESl); lymphocyte-specific protein tyrosine kinase (LCK); A kinase anchor protein 4 (AKAP-4); synovial sarcoma, X breakpoint 2 (SSX2); Receptor for Advanced Glycation End products (RAGE-1); renal ubiquitous 1 (RUl); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte- associated immunoglobulin-like receptor 1 (LAIRl); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLLl), MPL, Biotin, c-MYC epitope Tag, CD34, LAMP1 TROP2, GFRalpha4, CDH17, CDH6, NYBR1, CDH19, CD200R, Slea (CA19.9; Sialyl Lewis Antigen) Fucosyl-GM1, PTK7, gpNMB, CDH1-CD324, DLL3, CD276/B7H3, IL11Ra, IL13Ra2, CD179b-IGL11, ALK TCRgamma-delta, NKG2D, CD32 (FCGR2A), CSPG4-HMW-MAA, Tim1-/HVCR1, CSF2RA (GM-CSFR-alpha), TGFbetaR2, VEGFR2/KDR, Lews Ag, TCR-beta1 chain, TCR-beta2 chain, TCR-gamma chain, TCR-delta chain, Leutenizing hormone receptor (LHR), Follicle stimulating hormone receptor (FSHR), Chorionic Gonadotropin Hormone receptor (CGHR), CCR4, SLAMF6, SLAMF4, HIV1 envelope glycoprotein, HTLV1-Tax, CMV pp65, EBV-EBNA3c, influenza A hemagglutinin (HA), GAD, PDL1, Guanylyl cyclase C (GCC), KSHV-K8.1 protein, KSHV-gH protein, auto-antibody to desmoglein 3 (Dsg3), autoantibody to desmoglein 1 (Dsg1), HLA, HLA-A, HLA-A2, HLA-B, HLA-C, HLA-DP, HLA-DM, HLA-DOA, HLA-DOB, HLA-DQ, HLA-DR, HLA-G, IGE, CD99, RAS G12V, Tissue Factor 1 (TF1), AFP, GPRC5D, claudin18.2 (CLD18A2 OR CLDN18A.2)), P- glycoprotein, STEAP1, LIV1, NECTIN-4, CRIPTO, GPA33, BST1/CD157, and low conductance chloride channel and Integrin B7.

In some embodiments, the target antigen is CD33, CD123, MPL, CD19, CD22, CD20, BCMA, CS1, FLT3, CSF2RA, IL6R, LAMP1, TSLRP, CD4, CXCR4, GPC3, CD45, CD44v, CD43, CD32, CD38, CD79b, CD138, CD179b, CD70, Folate Receptor beta, WT1, NY-ESO1, CLL1, IL1Ra, CLEC5A, PR1, TGFbeta, ROR1, TnAg, CD200R, Kappa Light Chain, TCRb1 constant chain, TCRb2 constant chain, TCRa constant chain, TCRg, TCRd, CD5, CD52, CD7, CD3e, IL1RAP, Lym1, Lym2 and/or BST1/CD157

Non-limiting examples of target antigens may be found in Jurgens et al. (2019) or international patent application publication numbers WO2012/079000, WO2015/157386, WO2015/075468, WO2013/176916, WO2011/119979, WO2012/079000, WO2014/031687, WO2011/059836, WO2014/153270, WO2015142675, WO2016049459, US20160355590, which are incorporated herein by reference.

In one embodiment, an antigen binding agent against LYM1 is an antigen binding portion, e.g., CDRs, of VL and VH fragments targeting this antigen or of an antibody, e.g., an antibody described in US20160355590A1. In one embodiment, an antigen binding agent against LYM1 is an antibody, an antibody fragment or an antibody-like moiety described in, e.g., US20160355590A1.

In some embodiments, antigens targeted by the receptors include antigens associated B cell malignancies, neuroblastoma, and/or ovarian carcinoma.

In some embodiments, the target antigen is expressed on a cell surface. In some embodiments, the target antigen is a cell surface ligand, a cell surface receptor, or a cell surface marker. In some embodiments, the target antigen is a polypeptide or a fragment thereof. In some embodiments, the target antigen is a processed peptide antigen presented, for example, by an MHC complex. In some embodiments, the target antigen is a carbohydrate. In some embodiments, the target antigen is any molecule expressed by a cell that can be targeted by the extracellular antigen binding domain contained in the CAR.

In some embodiments, the target antigen is expressed selectively, exclusively, or is overexpressed on a cell. In some embodiments, for example, the target antigen is expressed on cells associated with a disease or condition. Non-limiting examples of cells associated with a disease or condition include tumor cells, cancer cells, virus infected cells, or cells otherwise differentiable from health cells or tissues. In some embodiments, the target antigen is expressed on tumor cells. In some embodiments, the target antigen is expressed on cancer cells.

In some embodiments, the extracellular antigen binding domain contained in the CAR includes an antigen binding portion or portions of an antibody molecule, such as a single-chain antibody fragment (scFv) derived from the variable heavy (VH) and variable light (VL) chains of a monoclonal antibody (mAb), or a single domain antibody (sdAb), such as sdFv, nanobody, VHH and VNAR.

In some embodiments, the extracellular antigen binding domain is an scFv. In some embodiments, the scFv includes one or more flexible linkers joining the heavy chain variable (VH) region and the light chain variable (VL) region. The linker typically is a peptide linker, e.g., a flexible and/or soluble peptide linker. Among the linkers are those rich in glycine and serine and/or in some cases threonine. In some embodiments, the linkers further include charged residues such as lysine and/or glutamate, which can improve solubility. In some embodiments, the linkers further include one or more proline. Non-limiting examples of linkers include linkers having various numbers of repeats of the sequence GGGGS (4GS; SEQ ID NO: 48), (GGGGS)n where n is an integer between 1 and 4 (SEQ ID NO: 47), sequence GGGGSGGGGSGGGGS (SEQ ID NO: 49), GSTSGSGKPGSGEGSTKG (SEQ ID NO: 115) SRGGGGSGGGGSGGGGSLEMA (SEQ ID NO: 116).

In some embodiments, the CAR contains a transmembrane domain. In some embodiments, the transmembrane domain is connected to the extracellular antigen binding domain via a spacer sequence. Space sequence may include, but are not limited to, a full-length or portion of an immunoglobulin constant region, such as a hinge region. In some embodiments, the spacer is an IgG4 hinge region, a CH1/CL (e.g., IgG1, IgG4 constant region), Fc region. Non-limiting examples of spacers may be found in Hudecek et al. (2013) Clin. Cancer Res., 19:3153 or international patent application publication number WO2014/031687, which are incorporated herein by reference. Alternatively, in some embodiments, the transmembrane domain is linked directly to extracellular antigen binding domain.

The transmembrane domain may be a naturally occurring membrane domain, or a selected or modified transmembrane domain. In some embodiments, a selected or modified transmembrane domain is useful for preventing or minimizing interactions with native or endogenous cellular proteins.

In some embodiments, the transmembrane domain may be derived from any naturally occurring membrane-bound or transmembrane protein. Examples of naturally occurring transmembrane domains may be derived from the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137 or CD154.

In some embodiments, the transmembrane domain is a synthetic transmembrane domain. In some embodiments, the synthetic transmembrane domain includes hydrophobic residues such as leucine and valine. In some aspects, a triplet of phenylalanine, tryptophan and valine will be found at each end of a synthetic transmembrane domain.

In some embodiments, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

In some embodiments, the CAR contains one or more intracellular signaling domains or components. In some embodiments, the intracellular signaling domain or component is an intracellular component of a TCR complex. In some embodiments, the intracellular component of the TCR complex is a CD3 chain, such as a CD3-zeta chain. In some embodiments, the CAR further includes a CD3 transmembrane domain, CD3 intracellular signaling domains, and/or other CD transmembrane domains.

The CARs provided herein may further contain portions of one or more additional molecules such as an Fc receptor y, CD8, CD4, CD25, or CD16. In some embodiments, the CAR may include a molecule comprised of portions of a CD3-zeta (CD3-ζ) chain or Fc receptor γ and CD8, CD4, CD25 or CD16.

In some embodiments, binding of the extracellular antigen binding domain of the CAR to the target antigen results in the intracellular signaling domain of the receptor to activate a normal effector function or response of the immune cell, e.g., engineered T cell. In some embodiments, binding of the extracellular antigen binding domain to a target antigen induces cytolytic activity or T-helper activity. In some embodiments, the intracellular signaling domain or domains include the cytoplasmic sequences of the T cell receptor (TCR), and in some aspects also those of co-receptors that in the natural context act in concert with such receptors to initiate signal transduction following target antigen binding, and/or any derivative or variant of such molecules, and/or any synthetic sequence that has the same functional capability.

In some embodiments, the CAR further includes domains capable of secondary or co-stimulatory signaling.

In some embodiments, the CAR includes a primary cytoplasmic signaling sequence that regulates primary activation of the TCR complex. Thus, in some embodiments, the CAR contains immunoreceptor tyrosine-based activation motifs (ITAMs), for example as contained in primary cytoplasmic signaling sequences including those derived from TCR zeta, FcR gamma, FcR beta, CD3 gamma, CD3 delta, CD3 epsilon, CD8, CD22, CD79a, CD79b, and CD66d. In some embodiments, the CAR contains a cytoplasmic signaling molecule derived from CD3 zeta or a portion thereof.

In some embodiments, the CAR includes a signaling domain and/or transmembrane portion of a costimulatory receptor, such as CD28, 4-1BB, OX40, CD27, DAP10, and ICOS. In some aspects, the same CAR includes both the activating and costimulatory components.

In some embodiments, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some embodiments, the CAR is a first, second or third generation CAR.

In some embodiments, the CAR includes: an extracellular antigen binding domain, such as an sdAbs or scFvs; a spacer such as any of the Ig-hinge containing spacers; a transmembrane domain that is a portion of CD28 or a variant thereof; an intracellular signaling domain containing a signaling portion of 4-1BB or functional variant thereof; and a signaling portion of CD3 zeta signaling domain or functional variant thereof. In some embodiments, the CAR includes: an extracellular antigen binding domain, such as an sdAbs or scFvs; a spacer such as any of the Ig-hinge containing spacers; a transmembrane domain that is a portion of CD8a or a variant thereof; an intracellular signaling domain containing a signaling portion of 4-1BB or functional variant thereof; and a signaling portion of CD3 zeta signaling domain or functional variant thereof. In some embodiments, the CAR includes: an extracellular antigen binding domain, such as an sdAbs or scFvs; a transmembrane domain that is a portion of CD8a or a variant thereof; an intracellular signaling domain containing a signaling portion of 4-1BB or functional variant thereof; and a signaling portion of CD3 zeta signaling domain or functional variant thereof.

In some embodiments, the CAR contains an extracellular antigen binding domain that specifically binds the target antigen, a transmembrane domain, and an intracellular signaling domain comprising an intracellular domain of a CD3-zeta (CD3ζ) chain. In some embodiments, the CAR contains an extracellular antigen binding domain that specifically binds the target antigen, a transmembrane domain, and an intracellular signaling domain comprising an intracellular domain of a CD3-zeta (CD3ζ) chain and an intracellular signaling domain of a T cell costimulatory molecule. In some embodiments, the T cell costimulatory molecule is 41BB. In any of such embodiments, the antigen binding domain is a single chain variable fragment (scFv). The antigen binding domain may be an antigen binding domain that binds any target antigen described herein. In some embodiments, the antigen binding domain binds a CD19, such as human CD19. In some embodiments, the antigen binding domain binds a B7H3, such as human B7H3. In any of such embodiments, the transmembrane domain comprises a transmembrane portion of CD8.

In some embodiments, the CAR includes an extracellular antigen binding domain capable of binding to a target antigen present on the surface of a B cell present in a B cell malignancy. In some embodiments, the B cell malignancy is B cell lymphoma. In some embodiments, the target antigen is present on the surface of cells of Non-Hodgkin's lymphoma (NHL) and Hodgkin's lymphoma. In some embodiments, the target antigen is present on cells of diffuse large B-cell lymphoma, follicular lymphoma, marginal zone B-cell lymphoma, mantle cell lymphoma, Hodgkin's lymphoma, acute lymphoblastic leukemia (ALL), or chronic lymphocytic leukemia (CLL), or Burkitt's lymphoma. In some embodiments, the target antigen is CD19. In some embodiments, the CAR includes: an extracellular antigen binding domain having the sequence set forth by SEQ ID NO: 83; a transmembrane domain having the sequence set forth by SEQ ID NO: 52; an intracellular signaling domain containing a signaling portion of 4-1BB set forth by SEQ ID NO: 56; and a signaling portion of CD3 zeta signaling domain set forth by SEQ ID NO: 55. In some embodiments, the CAR is or comprises the sequence of amino acids set forth by SEQ ID NO: 117. In some embodiments, the chimeric signaling receptor is or comprises a sequence of amino acids that exhibits at least or about 85%, 90%, 92%, 95%, or 97% sequence identity to sequence of SEQ ID NO: 117.

In some embodiments, the CAR includes an extracellular antigen binding domain capable of binding a target antigen present on the surface of a tumor or cancer cell, e.g., a tumor antigen or solid tumor antigen. In some embodiments, the target antigen is present on the surface of cells of neuroblastoma, ovarian carcinoma, prostate cancer, non-small-cell lung cancer, pancreatic cancer, breast cancer, and/or colorectal cancer. In some embodiments, the target antigen is present on the surface of neuroblastoma cancer cells. In some embodiments, the target antigen is present on the surface of ovarian tumor cells. In some embodiments, the target antigen is B7H3. In some embodiments, the CAR includes: an extracellular antigen binding domain having the sequence set forth by SEQ ID NO: 84; a transmembrane domain having the sequence set forth by SEQ ID NO: 52; an intracellular signaling domain containing a signaling portion of 4-1BB set forth by SEQ ID NO: 56; and a signaling portion of CD3 zeta signaling domain set forth by SEQ ID NO: 55. In some embodiments, the CAR is or comprises the sequence of amino acids set forth by SEQ ID NO: 118. In some embodiments, the chimeric signaling receptor is or comprises a sequence of amino acids that exhibits at least or about 85%, 90%, 92%, 95%, or 97% sequence identity to sequence of SEQ ID NO: 118.

In some embodiments, the CARs provided herein comprise a signal peptide to facilitate localization to the cell membrane for expression on the cell surface. In some embodiments, the signal peptide is present in a precursor CAR protein and is cleaved to form a the mature CAR. In some embodiments, the signal peptide is position at the N-terminus of a heavy chain of an scFv that serves as the extracellular antigen binding domain of the CAR. In some embodiments, the signal peptide is a CD8α signal peptide. In some embodiments, the signal peptide is or comprises the amino acid sequence set forth by SEQ ID NO: 102.

In some embodiments, the CAR is or comprises the sequence of amino acids set forth by SEQ ID NO: 38. In some embodiments, the chimeric signaling receptor is or comprises a sequence of amino acids that exhibits at least or about 85%, 90%, 92%, 95%, or 97% sequence identity to sequence of SEQ ID NO: 38.

In some embodiments, the CAR is or comprises the sequence of amino acids set forth by SEQ ID NO: 45. In some embodiments, the chimeric signaling receptor is or comprises a sequence of amino acids that exhibits at least or about 85%, 90%, 92%, 95%, or 97% sequence identity to sequence of SEQ ID NO: 45.

The CARs provided herein may further include a marker or tag, e.g., myc-tag, for purposes of detection, e.g., expression, successful transduction, and/or quantification.

### B. Recombinant T Cell Receptors

As described herein, in some cases, a chimeric signaling receptor, such as described in Section I, is co-expressed with a recombinant antigen receptor, such as a recombinant T cell receptor (TCR). In some embodiments, the chimeric signaling receptor and the recombinant TCR are expressed in a cell engineered, e.g., with a polynucleotide or vector described herein, to express the chimeric signaling receptor and the TCR on the cell surface. In some embodiments, the chimeric signaling receptor and the recombinant TCR are contained in two separate polynucleotides. In some embodiments, the chimeric signaling receptor and the recombinant TCR are contained in a single polynucleotide, for example as described in Section II-A..

In some embodiments, the recombinant TCR is specific for an antigen, also referred to as a target antigen. In some embodiments, the target antigen is expressed on a cell surface. In some embodiments, the target antigen is a cell surface ligand, a cell surface receptor, or a cell surface marker. In some embodiments, the target antigen is a polypeptide or a fragment thereof. In some embodiments, the target antigen is a processed peptide antigen presented, for example, by an MHC complex. In some embodiments, the target antigen is any molecule expressed by a cell that can be targeted by the recombinant T cell receptor binding domain.

In some embodiments, the target antigen is expressed selectively, exclusively, or is overexpressed on a cell. In some embodiments, for example, the target antigen is expressed on cells associated with a disease or condition. Non-limiting examples of cells associated with a disease or condition include tumor cells, cancer cells, virus infected cells, or cells otherwise differentiable from health cells or tissues. In some embodiments, the target antigen is expressed on tumor cells. In some embodiments, the target antigen is expressed on cancer cells.

In some embodiments, the TCR is cloned from naturally occurring T cells. In some embodiments, a T cell clone is identified and isolated from a patient. In some embodiments, the TCR generated and isolated from transgenic mice engineered with human immune system genes (e.g., the human leukocyte antigen system, or HLA). See, e.g., tumor antigens (see, e.g., Parkhurst et al. (2009) Clin Cancer Res. 15:169-180 and Cohen et al. (2005) J Immunol. 175:5799-5808. In some embodiments, phage display is used to isolate TCRs against a target antigen (see, e.g., Varela-Rohena et al. (2008) Nat Med. 14:1390-1395 and Li (2005) Nat Biotechnol. 23:349-354.

In some embodiments, after the T-cell clone is obtained, the TCR alpha and beta chains are isolated and cloned into a gene expression vector. In some embodiments, the the alpha and beta chains may be isolated from the cloned TCRs and cloned into a gene expression vector. In some embodiments, the alpha and beta genes are linked via a picornavirus 2A ribosomal skip peptide, such as described in Section II-A, thus allowing both chains to be co-expressed. The recombinant TCRs provided herein may further include a marker or tag for purposes of detection, e.g., expression, successful transduction, and/or quantification.

### IV. Recombinant Secretable Molecule

In some embodiments, a chimeric signaling receptor provided herein is co-expressed in a cell, e.g. T cell, with a secretable molecule. In some embodiments, the secretable molecule is a bispecific antibody, such as a bispecific T cell engager (BiTE). In some embodiments, the secretable molecule is a cytokine.

In some embodiments, a chimeric signaling receptor provided herein is co-expressed in a cell, e.g. T cell, with a bispecific targeting molecule, such as a bispecific T cell engager (BiTE). In some embodiments, the cell is also is engineered to express a recombinant antigen receptor, such as any described in Section III above. In some embodiments, the recombinant antigen receptor is a chimeric antigen receptor (CAR). In some embodiments, the recombinant antigen receptor is a T cell receptor.

In some embodiments, the bispecific targeting molecule is a bispecific antibody. In some embodiments, a bispecific antibody comprises two different binding specificities and thus binds to two different antigens. In one embodiment, the bispecific antibody comprises a first antigen binding domain that binds to a first antigen and a second antigen binding domain that binds to a second antigen. In another embodiment, the bispecific antibody comprises an antigen binding domain comprising a first and a second single chain variable fragment (scFv) molecules. In one embodiment, the first and a second antigen binding domains bind an antigen on a target cell and an antigen on an activating T cell.

In one embodiment, the bispecific antibody comprises specificity to at least one antigen on an activating T cell. The activating T cell antigen includes antigens found on the surface of a T cell that can activate another cell. The activating T cell antigen may bind a co-stimulatory molecule. A costimulatory molecule is a cell surface molecule, other than an antigen receptor or their ligands, that is required for an efficient response of lymphocytes to an antigen. Examples of the activating T cell antigen can include but are not limited to CD3, CD4, CD8, T cell receptor (TCR), CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, a ligand that specifically binds with CD83, or any fragment thereof. In some embodiments, the T cell antigen is CD3. In these examples, the bispecific antibody recognizes a T cell antigen and is referred to as a Bispecific T Cell Engager (BiTE).

The bispecific antibody or BiTE molecule may also be expressed as a soluble protein with specificity for at least one target cell associated antigen. In provided embodiments, the target cell antigen may be the same antigen that a T cell receptor binds to or may be a different antigen. The target cell antigen includes any tumor associated antigen (TAA) or viral, bacterial and parasitic antigen, or any fragment thereof. The target cell antigen may include any type of ligand that defines the target cell. For example, the target cell antigen may be chosen to recognize a ligand that acts as a cell marker on target cells associated with a particular disease state. Thus, cell markers may act as ligands for the antigen binding domain in the bispecific antibody, including those associated with viral, bacterial and parasitic infections, autoimmune disease and cancer cells.

In some embodiments, the tumor antigen is selected from the group consisting of: CD2, CD19, CD20, CD22, CD27, CD33, CD37, CD38, CD40, CD44, CD47, CD52, CD56, CD70, CD79, and CD137. In some embodiments, the tumor antigen is selected from the group consisting of: 4-1BB, 5T4, AGS-5, AGS-16, Angiopoietin 2, B7.1, B7.2, B7DC, B7H1, B7H2, B7H3, BT-062, BTLA, CAIX, Carcinoembryonic antigen, CTLA4, Cripto, ED-B, ErbB1, ErbB2, ErbB3, ErbB4, EGFL7, EpCAM, EphA2, EphA3, EphB2, FAP, Fibronectin, Folate Receptor, Ganglioside GM3, GD2, glucocorticoid-induced tumor necrosis factor receptor (GITR), gp100, gpA33, GPNMB, ICOS, IGF1R, Integrin αν, Integrin ανβ, KIR, LAG-3, Lewis Y, Mesothelin, c-MET, MN Carbonic anhydrase IX, MUC1, MUC16, Nectin-4, NKGD2, NOTCH, OX40, OX40L, PD-1, PDL1, PSCA, PSMA, RANKL, ROR1, ROR2, SLC44A4, Syndecan-1, TACI, TAG-72, Tenascin, TIM3, TRAILR1, TRAILR2, VEGFR-1, VEGFR-2, VEGFR-3, and variants thereof.

In some embodiments, the bispecific T cell engager (BiTE) is a bispecific antibody targeted against a CD19/CD3, a CD20/CD3, a CD22/CD3, a CD38/CD3, a BCMA/CD3, a FAP/CD3, a PD-L1/CD3, PD-L2/CD3 or GD2/CD3. For instance, the bispecific antibody may be an anti-CD3 x anti-GD2 bispecific antibody. In some embodiments, each antibody of the bispecific antibody is an scFv. In some embodiments, the BiTE comprises scFvs that bind CD19 and CD3, CD20 and CD3, CD22 and cD3, CD38 and CD3, BCMA and CD3, FAP and CD3, PD-L1 and CD3, PD- L2 and CD3 or GD2 and CD3.

In one embodiment, the bispecific antibody comprises more than one antigen binding domains. In this embodiment, at least one antigen binding domain includes a synthetic antibody, human antibody, a humanized antibody, single chain variable fragment, single domain antibody, an antigen binding fragment thereof, and any combination thereof. Techniques for making human and humanized antibodies are described elsewhere herein.

In some embodiments, provided cells are engineered with a nucleic acid encoding a bispecific antibody comprising bispecificity for an antigen on a target cell and an antigen on an activating T cell, wherein the T cell transiently secretes the bispecific antibody. Techniques for engineering and expressing bispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and "knob-in-hole" engineering (see, e.g., U.S. Pat. No. 5,731,168). Multi-specific antibodies may also be made by engineering electrostatic steering effects for making antibody Fc-heterodimeric molecules (WO 2009/089004A1); cross-linking two or more antibodies or fragments (see, e.g., U.S. Pat. No. 4,676,980, and Brennan et al., Science 229:81 (1985)); using leucine zippers to produce bispecific antibodies (see, e.g., Kostelny et al., J. Immunol. 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991). Engineered antibodies with three or more functional antigen binding sites, including "Octopus antibodies," are also included herein (see, e.g. US 2006/0025576A1). Bispecific antibodies can be constructed by linking two different antibodies, or portions thereof. For example, a bispecific antibody can comprise Fab, F(ab')₂, Fab', scFv, and sdAb from two different antibodies.

In some embodiments, the recombinant molecule is a cytokine, such as an interleukin. Interleukins are a group of cytokines that are generally secreted proteins and signal molecules that mediate a broad range of immune responses. In some embodiments, an interleukin or a functional portion thereof, or a polynucleotide encoding the same, is introduced into the cell also engineered with the chimeric signaling receptor. In some embodiments, the interleukin or functional portion thereof is a partial or full peptide of one or more of IL-2, IL-4, IL-6, IL-7, IL-9, IL-10, IL-11, IL-12, IL-15, IL-18, or IL-21. In some embodiments, the cytokine is IL-2, IL-7, IL-12, IL-15, IL-18, IL-21, Flt3-L, SCF, or IL-7. In some embodiments, the cytokine is IL-2 or a functional portion thereof. In some embodiments, the cytokine is IL-12 or a functional portion thereof. In some embodiments the cytokine is IL-15 or a functional portion thereof. In some embodiments, the cytokine is IL-21 or a functional portion thereof. In some embodiments, the cytokine may be introduced with the respective receptor for the cytokine. The cytokine (e.g., IL-2, IL-12, IL- 15, or IL-21) amino acid sequences may comprise any functional portion of the mature cytokine, e.g. any functional portion of a mature,IL-2, mature IL-12, mature IL-15 or mature IL-21. The functional portion can be any portion comprising contiguous amino acids of the interleukin of which it is a part, provided that the functional portion specifically binds to the respective interleukin receptor. The term "functional portion" when used in reference to an interleukin refers to any part or fragment of the interleukin, which part or fragment retains the biological activity of the interleukin of which it is a part (the parent interleukin). Functional portions encompass, for example, those parts of an interleukin that retain the ability to specifically bind to the respective interleukin receptor, activate the downstream targets of the interleukin, and/or induce one or more of the differentiation, proliferation (or death) and activity of immune cells, e.g., T cells, to a similar extent, the same extent, or to a higher extent, as the parent interleukin. The biological activity of the functional portion of the interleukin may be measured using assays known in the art. In reference to the parent interleukin, the functional portion can comprise, for instance, about 60%, about 70%, about 80%, about 90%, about 95%, or more, of the amino acid sequence of the parent mature interleukin.

### V. CELL THERAPY

In some aspects, the chimeric signaling receptors provided herein are used in a cell therapy to treat a disease or condition, such as cancer. In some embodiments, the chimeric signaling receptors as described in Section I-A are co-expressed with a recombinant antigen receptor, e.g., CAR or recombinant TCR as described in Section III, in an engineered cell for cell therapy. In some embodiments, the presence of the chimeric signaling receptor prevents immune cell suppression in a tumor microenvironment (TME). In some embodiments, the chimeric signaling receptor activates the immune cell in the TME. For example, in cases where the TME contains TGFβ, the chimeric signaling receptors expressed on the surface of the engineered cell can bind to the TGFβ resulting in induction of a MyD88-dependent signaling pathway that can activate the immune cell. In some embodiments, the chimeric signaling receptor also decreases or minimizes TGFβ signaling through its native, immune cell inhibitory pathway. Thus, in some embodiments, the chimeric signaling receptor can increase the activity, efficacy, and/or persistence of a cell therapy, even in the presence of an immunosuppressive TME. By reversing and/or preventing the suppression of immune cells in the TME, immune cells can mount an effective response against the tumor cells.

Also provided are populations of cell engineered to express a chimeric signaling receptor and a recombinant antigen receptor, compositions containing such cells and/or enriched for such cells. Among the compositions are pharmaceutical compositions and formulations for administration, such as for adoptive cell therapy. Also provided are therapeutic methods for administering the cells and compositions to subjects, e.g., patients.

In some embodiments, the cells of the cell therapy are engineered by introducing via genetic engineering one or more nucleic acids encoding the chimeric signaling receptor and the recombinant antigen receptor to the cell, for example by transduction or electroporation, thereby causing the cell to co-express both receptors. In some embodiments, gene transfer is accomplished by first stimulating the cells, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

### A. Cells for genetic engineering

Suitable cells for genetic engineering according the methods described herein (see, e.g., Section II and below) include immune cells. In some embodiments, the immune cell is obtained from a healthy subject. In some embodiments, the immune cell is obtained from a subject that has a disease or condition. In some embodiments, the immune cells are primary cells. In some embodiments, the immune cells are autologous. In some embodiments, the immune cells are allogeneic.

In some embodiments, the cell for genetic engineering is an immune cell capable of targeting and/or infiltrating cancer cells or tumors. For example, in some cases, the immune cell is a cell that targets and/or infiltrates cancers cells and/or tumors to affect cell killing. In some embodiments, the immune cell for engineering is a lymphocyte, such as T cell, Natural Killer (NK) cell, or tumor-infiltrating lymphocyte (TIL).

In some aspects, the immune cell for genetic engineering is a T cell. In some embodiments, the engineered cell for a cell therapy is a T cell. T cells can be classified based on cell surface marker expression, such as CD3, CD4, and/or CD8 expression. Thus, in some embodiments, the T cell is a CD3+ T cell. In some embodiments, the T cell is a CD4+ T cell. In some embodiments, the T cell is a CD8+ T cell. In some embodiments, the T cell is cytotoxic T cell. For example, a cytotoxic T cell capable of killing cells such as cancer cells, cells that are damaged, and/or infected cells, e.g., virally infected cells. In some embodiments, the cytotoxic T cell is a CD8+ cytotoxic T cell.

In some cases, the T cell for engineering is a subtype of CD4+ or CD8+ T cells. For example, in some embodiments, the CD4+ or CD8+ T cell for engineering is a CD4+ or CD8+ naive T cell, a CD4+ or CD8+ central memory T cell, a CD4+ or CD8+ effector T cell, or a CD4+ or CD8+ effector memory T cell. In some embodiments, the T cell subtype is a naive T cell, an effector T cell (TEFF), a memory T cell and sub-types thereof, such as stem cell memory T (TSCM), central memory T (TCM), effector memory T (TEM), TEMRA cells or terminally differentiated effector memory T cells. In some embodiments, any, all, or a subset of T cell subtypes, for example as described herein, may be used for genetic engineering.

In some aspects, the immune cell for genetic engineering is a Natural Killer (NK) cell. In some embodiments, the engineered cell for a cell therapy is an NK cell. NK cells are cytotoxic lymphocytes of the innate immune system. In some embodiments, the NK cell for engineering is CD3-, CD56+, CD94+, CD122/IL-2 R beta+, CD127/IL-7 alpha-, Fc gamma RIII/CD16+, Fc gamma RIII/CD16-, KIR family receptor+, NKG2A+, NKG2D+, NKp30+, NKp44+, NKp46+, and/or NKp80+. In some embodiments, the NK cell is CD56+/CD3-. In some embodiments, the NK cell is CD56+. In some embodiments, the CD56+/CD3- NK cell is CD7+/CD127-/NKp46+/T-bet+/Eomes+. In some embodiments, the NK cell is CD16+/CD56+. In some embodiments, the NK cell is CD16-/CD56+.In some embodiments, the NK cell is CD3-/CD16+/CD56+. In some embodiments, the NK cell is CD3-/CD16-/CD56+. In some embodiments, any, all, or a subset of NK cell subtypes, for example as described herein, may be used for genetic engineering.

In some aspects, the immune cell for genetic engineering is a tumor-infiltrating lymphocyte (TIL). In some embodiments, the engineered cell for a cell therapy is a TIL. TILs include white blood cells such as T cells and B cells that migrate from the blood stream to tumor locations. In some embodiments, TILs are found in the tumor stroma. In some embodiments, TILs are found in the tumor proper. In some embodiments, TILs affect tumor cell killing. In some embodiments, TILs are CD3+. In some embodiments, any, all, or a subset of TIL subtypes, for example as described herein, may be used for genetic engineering.

### B. Methods for genetic engineering of cells

In some embodiments, preparation of the engineered cells includes one or more culture and/or preparation steps. The cells for introduction of the chimeric signaling receptor may be isolated from a sample, such as a biological sample, e.g., one obtained from or derived from a subject. In some embodiments, the cells are isolated from a subject having a disease or condition or who is in need of a cell therapy or to which cell therapy will be administered. In some embodiments, the subject is a human in need of a particular therapeutic intervention, such as the adoptive cell therapy for which cells are being isolated, processed, and/or engineered. Thus, in some embodiments the cells are primary cells, e.g., primary human cells. In some embodiments, the cells are further engineered to express a recombinant antigen receptor, e.g., CAR or TCR.

Cells for engineering may be derived, isolated, or in any way obtained from samples such as, but not limited to, tissue, fluid, and other samples taken directly from the subject, as well as samples resulting from one or more processing steps, such as separation, centrifugation, genetic engineering (e.g. transduction with viral vector), washing, and/or incubation. In some embodiments, the biological sample is a sample obtained directly from a biological source. In some embodiments, the biological sample is a sample that is processed. Non-limiting examples of biological samples include body fluids, such as blood, plasma, serum, cerebrospinal fluid, synovial fluid, urine and sweat, tissue and organ samples, including processed samples derived therefrom.

In some embodiments, the cells are derived or isolated from blood or a blood-derived samples, such as an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. In some embodiments, samples are obtained from autologous sources. In some embodiments, samples are obtained from allogeneic sources. In some embodiments, the cells for engineering are obtained or derived from cell lines.

In some embodiments, the cells obtained from the sample are washed, centrifuged, and/or incubated in the presence of one or more reagents, for example, to remove unwanted components, enrich for desired components, lyse or remove cells sensitive to particular reagents. In some embodiments, the cells are separated based on one or more properties, such as density, adherent properties, size, sensitivity and/or resistance to particular components.

In some embodiments, the methods include density-based cell separation methods, such as the preparation of white blood cells from peripheral blood by lysing the red blood cells and centrifugation through a Percoll or Ficoll gradient.

In some embodiments, cell types may be isolated from one another based on the expression or presence in the cell of one or more specific molecules, such as surface markers, e.g., surface proteins, intracellular markers, or nucleic acid. In some embodiments, any known method for separation based on such markers may be used. In some embodiments, the separation is affinity- or immunoaffinity-based separation.

The separation techniques contemplated herein can be based on positive selection, in which the cells having bound the reagents are retained for further use, negative selection, in which the cells having not bound to the antibody or binding partner are retained, or both positive and negative selection. In some cases, negative selection is useful where no antibody is available for separation of particular marker, e.g., surface molecule.

In some embodiments, the separation of cells leads to an enrichment of the cells of a particular type. In some embodiments, the enrichment is 65%, 70%, 75%, 80%, 85%, 90%, 92%, 95%, 97%, or more, or any percentage in between, of the particular cell type.

In some embodiments, specific subpopulations of cells are enriched. In some embodiments, the enriched cells are those described in Section III-A. In some embodiments T cells, and subtypes thereof, such as described in Section III-A are enriched. In some embodiments, NK cells are enriched. In some embodiment, TILs are enriched.

The separation and/or other steps may be carried out according to an suitable method for isolation of the cell to be engineered. In some embodiments, separation is carried out using CliniMACS system (Miltenyi Biotic), for example, for automated separation of cells on a clinical-scale level in a closed and sterile system. Components can include an integrated microcomputer, magnetic separation unit, peristaltic pump, and various pinch valves. The integrated computer in some aspects controls all components of the instrument and directs the system to perform repeated procedures in a standardized sequence. The magnetic separation unit in some aspects includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some aspects uses antibody-coupled magnetizable particles that are supplied in a sterile, non-pyrogenic solution. In some embodiments, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some embodiments, the cell populations for use with the methods described herein are unlabeled and are not retained in the column. In some embodiments, the cell populations for use with the methods described herein are labeled and are retained in the column. In some embodiments, the cell populations for use with the methods described herein are eluted from the column after removal of the magnetic field, and are collected within the cell collection bag.

In certain embodiments, separation is carried out using the CliniMACS Prodigy system (Miltenyi Biotec). The CliniMACS Prodigy system in some aspects is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood may be automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakuraet al. (2012) Blood.1:72-82, and Wang et al. (2012) J Immunother. 35(9):689-701.

In some embodiments, a cell population described herein is collected and enriched (or depleted) via flow cytometry, in which cells stained for multiple cell surface markers are carried in a fluidic stream. In some embodiments, a cell population described herein is collected and enriched (or depleted) via preparative scale (FACS)-sorting. In certain embodiments, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both cases, cells can be labeled with multiple markers, allowing for the isolation of well-defined T cell subsets at high purity.

In some embodiments, the antibodies or binding partners are labeled with one or more detectable marker, to facilitate separation for positive and/or negative selection. For example, separation may be based on binding to fluorescently labeled antibodies. In some examples, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some embodiments, the preparation methods include steps for freezing, e.g., cryopreserving, the cells, either before or after isolation, incubation, and/or engineering. In some embodiments, the freeze and subsequent thaw step removes granulocytes and, to some extent, monocytes in the cell population. In some embodiments, the cells are suspended in a freezing solution, e.g., following a washing step to remove plasma and platelets. Any of a variety of known freezing solutions and parameters in some aspects may be used.

In some embodiments, the provided methods include cultivation, incubation, culture, and/or genetic engineering steps. For example, in some embodiments, provided are methods for incubating and/or engineering the depleted cell populations and culture-initiating compositions.

Thus, in some embodiments, the cell populations are incubated in a culture-initiating composition. The incubation and/or engineering may be carried out in a culture vessel, such as a unit, chamber, well, column, tube, tubing set, valve, vial, culture dish, bag, or other container for culture or cultivating cells.

In some embodiments, the cells are incubated and/or cultured prior to or in connection with genetic engineering. The incubation steps can include culture, cultivation, stimulation, activation, and/or propagation. In some embodiments, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a chimeric signaling receptor and/or a recombinant antigen receptor, such as described herein.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some aspects, incubation is carried out in accordance with techniques such as those described in US Patent No. 6,040,1 77 to Riddell et al., Klebanoff et al.(2012) J Immunother. 35(9): 651-660, Terakuraet al. (2012) Blood.1:72-82, and/or Wang et al. (2012) J Immunother. 35(9):689-701.

Various methods for the introduction of genetically engineered components, e.g., chimeric signaling receptors and recombinant antigen receptors, e.g., CARs or TCRs, are well known and may be used with the provided methods and compositions. Exemplary methods include those for transfer of nucleic acids encoding the chimeric signaling receptors, including via viral vectors, e.g., retroviral or lentiviral, non-viral vectors or transposons, e.g. Sleeping Beauty transposon system. Methods of gene transfer can include transduction, electroporation or other method that results into gene transfer into the cell. The methods described herein are also applicable to the introduction of nucleic acids encoding recombinant antigen receptors, e.g., CAR, TCR, into the immune cell, including when such nucleic acids are part of a bi-, tri-, or multicistronic polynucleotide comprising the chimeric signaling receptor.

In some embodiments, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications. In some embodiments, the cell to be engineered is activated, for example using CD28/CD3 e.g., Dynabeads^{®} or MACS^{®} prior to transduction, transfection, or other means described herein or available in the art.

In some embodiments, nucleic acids or polynucleotides are transferred into cells using recombinant infectious virus particles, such as, e.g., vectors derived from simian virus 40 (SV40), adenoviruses, adeno-associated virus (AAV). In some embodiments, recombinant nucleic acids are transferred into immune cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some embodiments, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV), spleen focus forming virus (SFFV), or adeno-associated virus (AAV). Most retroviral vectors are derived from murine retroviruses. In some embodiments, the retroviruses include those derived from any avian or mammalian cell source. The retroviruses typically are amphotropic, meaning that they are capable of infecting host cells of several species, including humans. In one embodiment, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some embodiments, recombinant nucleic acids are transferred into immune cells via electroporation (see, e.g., Chicaybam et al, (2013) PLoS ONE 8(3): e60298 and Van Tedeloo et al. (2000) Gene Therapy 7(16): 1431-1437). In some embodiments, recombinant nucleic acids are transferred into immune cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA coprecipitation (Brash et al., Mol. Cell Biol., 7: 2031-2034 (1987)).

Other approaches and vectors for transfer of the nucleic acids encoding the chimeric signaling receptors are those described, e.g., in international patent application, Publication No.: WO2014055668, and U.S. Patent No. 7,446,190.

In some embodiments, the immune cells, e.g., T cells, NK cells, TILs, may be transfected or transduced either during or after expansion, e.g. with a chimeric signaling receptor. In some embodiments, the immune cells, e.g., T cells, are transfected or transduced either during or after expansion, e.g., with a chimeric signaling receptor. This transfection or transduction can be carried out with any suitable retroviral vector, for example. The genetically modified cell population can then be liberated from the initial stimulus (CD3/CD28 stimulus, for example) and subsequently be stimulated with a second type of stimulus e.g. via a de novo introduced receptor). This second type of stimulus may include an antigenic stimulus in form of a peptide/MHC molecule. In cases where a recombinant antigen receptor is also expressed in the cell, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some embodiments, the transfected or transduced cells are expanded. In some embodiments, the engineered cells are expanded until a desired threshold is reach.

### C. Exemplary properties of engineered cells

In some aspects, expression of a chimeric signaling receptor as described herein in an engineered cell increases the persistence, exposure. proliferation, and/or cytotoxic activity of engineered cell. For example, expression of the chimeric antigen receptor may prevent suppression of the engineered cell, e.g., immune cell, thus allowing said engineered cell to persist, proliferate, and/or kill target cells.

In some embodiments, the cells engineered to express the chimeric signaling receptors described herein have increased persistence, exposure, proliferation, and/or cytotoxic activity in the presence of an immunosuppressive environment, e.g., a TME, for example in comparison to cells not engineered to express the chimeric signaling receptor. In some embodiments, the cells engineered to express the chimeric signaling receptors described herein have increased persistence, exposure, proliferation, and/or cytotoxic activity in the presence of a TME, for example in comparison to cells not engineered to express the chimeric signaling receptor. In some embodiments, the cells engineered to express the chimeric signaling receptors described herein have increased persistence, exposure, proliferation, and/or cytotoxic activity in the presence of molecules, such as cytokines, capable of suppressing immune activity, compared to cells not engineered to express the chimeric signaling receptor. In some embodiments, the cells engineered to express the chimeric signaling receptors described herein have increased persistence, exposure, proliferation, and/or cytotoxic activity in the presence of TGFβ, compare to cells not engineered to express the chimeric signaling receptor. Thus, in some embodiments, cells engineered to express the chimeric signaling receptor described herein either by itself or in combination with a recombinant antigen receptor, e.g., CAR, TCR as described herein, have increased persistence, exposure, proliferation, and/or cytotoxic activity compared to cells not engineered to express the chimeric signaling receptor or engineered to express only a recombinant antigen receptor.

### 1. Engineered Cell Persistence, Exposure, and Proliferation

In some embodiments, the exposure, persistence, and proliferation of cells engineered to express a chimeric signaling receptor can be measured by assessing the characteristics of the engineered cell in vitro, ex vivo, or in vivo. In some embodiments, the cell engineered to expression the chimeric signaling receptor is further engineered to express a recombinant antigen receptor e.g., CAR, TCR. Persistence and proliferation of cells engineered to co-express chimeric signaling receptors as described herein and recombinant antigen receptors, e.g., CAR, TCR, can also be assessed in vitro, ex vivo, or in vivo assays as described herein.

In some embodiments, the presence and/or amount of cells expressing the chimeric signaling receptor (e.g., chimeric signaling receptor-expressing cells administered for cell therapy, optionally further containing a recombinant antigen receptor) in a subject following the administration of the engineered cells is assessed. In some aspects, quantitative PCR (qPCR) is used to assess the quantity of cells expressing the chimeric signaling receptor in the blood or serum or organ or tissue sample (e.g., disease site, e.g., tumor sample) of the subject. In some aspects, persistence is quantified as copies of DNA or plasmid encoding the chimeric signaling receptor, per microgram of DNA, or as the number of chimeric signaling receptor-expressing cells per microliter of the sample, e.g., of blood or serum, or per total number of peripheral blood mononuclear cells (PBMCs) or white blood cells or T cells per microliter of the sample. In cases where the cell has been engineered to co-express a chimeric signaling receptor and a recombinant antigen receptor (e.g., CAR, TCR), the methods described herein may also be used to assess or quantify the recombinant antigen receptor.

The exposure, e.g., number of chimeric signaling receptor-expressing cells, e.g., administered for cell therapy, indicative of expansion and/or persistence, may be stated in terms of maximum numbers of the chimeric signaling receptor-expressing cells to which the subject is exposed, duration of detectable chimeric signaling receptor-expressing cells or chimeric signaling receptor-expressing cells above a certain number or percentage, area under the curve for number of chimeric signaling receptor-expressing cells over time, and/or combinations thereof and indicators thereof. Exposure may be assessed using known methods, such as qPCR to detect copy number of nucleic acid encoding the chimeric signaling receptor compared to total amount of nucleic acid or DNA in the particular sample, e.g., blood, serum, plasma or tissue, such as a tumor sample, and/or flow cytometric assays detecting cells expressing the chimeric signaling receptor generally using antibodies specific for the chimeric signaling receptor. In cases where the cell has been engineered to co-express a chimeric signaling receptor and a recombinant antigen receptor (e.g., CAR, TCR) the methods described herein may also be used to assess or quantify the recombinant antigen receptor.

Cell-based assays may also be used to detect the number or percentage of functional cells, such as chimeric signaling receptor-expressing cells capable of binding to and/or neutralizing and/or inducing responses, e.g., cytotoxic responses, against cells of the disease or condition, and/or expressing the antigen recognized by a recombinant antigen receptor if the cell is engineered to co-express a chimeric signaling receptor and a recombinant antigen receptor.

In some aspects, the chimeric signaling receptor-expressing cells demonstrate high in vivo proliferation, for example, as measured by flow cytometry. In some aspects, high peak proportions of the cells are detected. For example, in some embodiments, at a peak or maximum level following administration of chimeric signaling receptor-expressing cells, in the blood or disease-site of the subject or white blood cell fraction thereof, e.g., PBMC fraction, at least about 10%, at least about 20%, at least about 30%, at least about 40%, at least about 50%, at least about 60%, at least about 70%, at least about 80%, or at least about 90% of the cells express the chimeric signaling receptor.

In some embodiments, cells expressing the chimeric signaling receptor are detectable in the serum, plasma, blood or tissue, e.g., tumor sample, of the subject, e.g., by a specified method, such as qPCR or flow cytometry-based detection methods, at least 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 or more days following administration of engineered cells expressing the chimeric signaling receptor. Cell numbers may be as detected by flow cytometry-based or quantitative PCR-based methods and extrapolation to total cell numbers using known methods. See, e.g., Brentjens et al., Sci Transl Med. 2013 5(177), Park et al, Molecular Therapy 15(4):825-833 (2007), Savoldo et al., JCI 121(5):1822-1826 (2011), Davila et al., (2013) PLoS ONE 8(4):e61338, Davila et al., Oncoimmunology 1(9):1577-1583 (2012), Lamers, Blood 2011 117:72-82, Jensen et al., Biol Blood Marrow Transplant 2010 September; 16(9): 1245-1256, Brentjens et al., Blood 2011 118(18):4817-4828.

In some embodiments, the area under the curve (AUC) for concentration of chimeric signaling receptor-expressing cells in a fluid, plasma, serum, blood, tissue, organ and/or disease site, e.g. tumor site, of the subject over time following the administration of the engineered cells, is greater as compared to that achieved compared to that achieved by administering cells lacking the chimeric signaling receptor.

In some aspects, the increased or prolonged expansion and/or persistence of the engineered cells in the subject is associated with a benefit in tumor related outcomes in the subject. In some embodiments, the tumor related outcome includes a decrease in tumor burden in the subject. In some embodiments, the tumor burden is decreased by or by at least at or about 10, 20, 30, 40, 50, 60, 70, 80, 90, or 100 percent after administration of the method. In some embodiments, disease burden, tumor size, tumor volume, tumor mass, and/or tumor load or bulk is reduced following dosing of engineered cells containing the chimeric signaling receptor by at least at or about 50%, 60%, 70%, 80%, 90% or more compared a subject that has been administered cells lacking the chimeric signaling receptor.

### 2. Engineered cell functional activity

In some embodiments, the functional activity of cells engineered to express a chimeric signaling receptor can be measured by assessing the characteristics of the engineered cell in vitro, ex vivo, or in vivo. In some embodiments, the cell engineered to expression the chimeric signaling receptor is further engineered to express a recombinant antigen receptor e.g., CAR, TCR. The functional activity of cells engineered to co-express chimeric signaling receptors as described herein and recombinant antigen receptors, e.g., CAR, TCR, can also be assessed in vitro, ex vivo, or in vivo.

In some embodiments, any of the known assays in the art for assessing the activity, e.g., cytotoxic or cytolytic activity, of cells containing the chimeric signaling receptor can be used. In some embodiments, the specific binding of a chimeric signaling receptor-expressing cells to target antigen can be assess in vivo, e.g., by imaging, or ex vivo, e.g., by ELISA or flow cytometry. In certain embodiments, the ability of the chimeric signaling receptor-expressing cells to destroy target cells can be measured using any suitable method known in the art, such as cytotoxicity assays described in, for example, Kochenderfer et al., J. Immunotherapy, 32(7): 689-702 (2009), and Herman et al., J. Immunological Methods, 285(1): 25-40 (2004). In certain embodiments, the biological activity of the chimeric signaling receptor-expressing cells is measured by assaying expression and/or secretion of one or more cytokines, such as CD107a, IFNγ, IL-2, GM-CSF and TNFα, and/or by assessing cytolytic or cytotoxic activity.

In some embodiments, assays for the activity, proliferation, and/or function of the engineered chimeric signaling receptor-expressing cells include, but are not limited to, ELISPOT, ELISA, cellular proliferation, cytotoxic lymphocyte (CTL) assay, binding to antigen, or intracellular cytokine staining, proliferation assays, lymphokine secretion assays, direct cytotoxicity assays, and limiting dilution assays. In some embodiments, proliferative responses of the engineered cells can be measured, e.g. by incorporation of 3H-thymidine, BrdU (5-Bromo-2'-Deoxyuridine) or 2'-deoxy-5-ethynyluridine (EdU) into their DNA or dye dilution assays, using dyes such as carboxyfluorescein diacetate succingamma secretase inhibitoryl ester (CFSE), CellTrace Violet, or membrane dye PKH26.

In some embodiments, assessing the activity, proliferation, and/or function of the engineered chimeric signaling receptor-expressing cells, includes measuring cytokine production from the engineered cells, and/or measuring cytokine production in a biological sample from the subject, e.g., plasma, serum, blood, and/or tissue samples, e.g., tumor samples. In some cases, such measured cytokines can include, without limitation, interleukin-2 (IL-2), interferon-gamma (IFNγ), interleukin-4 (IL-4), TNF-alpha (TNFα), interleukin-6 (IL-6), interleukin-10 (IL-10), interleukin-12 (IL-12), granulocyte-macrophage colony-stimulating factor (GM-CSF), CD107a, and/or TGF-beta (TGFβ). Assays to measure cytokines are well known in the art, and include but are not limited to, ELISA, intracellular cytokine staining, cytometric bead array, RT-PCR, ELISPOT, flow cytometry and bio-assays in which cells responsive to the relevant cytokine are tested for responsiveness (e.g. proliferation) in the presence of a test sample.

In some embodiments, assessing the activity, proliferation, and/or function of the engineered chimeric signaling receptor-expressing cells includes assessing cell surface marker expression e.g., phenotype. In some embodiments, the engineered cells are assessed for expression of cell activation markers, cell exhaustion markers, and/or markers of differentiation. In some embodiments, the cell phenotype is assessed before administration. In some embodiments, the cell phenotype is assessed after administration to a subject. Exemplary immune cell, e.g., T cell, NK cell, TIL, cell activation markers, exhaustion markers, and/or differentiation markers for assessment include any markers known in the art for particular subsets of immune cells, e.g., CD25, CD38, human leukocyte antigen-DR (HLA-DR), CD69, CD44, CD137, KLRG1, CD62Llow, CCR7low, CD71, CD2, CD54, CD58, CD244, CD160, programmed cell death protein 1 (PD-1), lymphocyte activation gene 3 protein (LAG-3), T-cell immunoglobulin domain and mucin domain protein 3 (TIM-3), cytotoxic T lymphocyte antigen-4 (CTLA-4), band T lymphocyte attenuator (BTLA) and/or T-cell immunoglobulin and immunoreceptor tyrosine-based inhibitory motif domain (TIGIT) (see, e.g., Liu et al., Cell Death and Disease (2015) 6, e1792).

In some aspects, detecting the expression levels includes performing an in vitro assay. In some embodiments, the in vitro assay is an immunoassay, an aptamer-based assay, a histological or cytological assay, or an mRNA expression level assay. In some embodiments, the parameter or parameters for one or more of each of the one or more factors, effectors, enzymes and/or surface markers are detected by an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay or avidity assay. In some embodiments, detection of cytokines and/or surface markers is determined using a binding reagent that specifically binds to at least one marker. In some cases, the binding reagent is an antibody or antigen-binding fragment thereof, an aptamer or a nucleic acid probe.

### VI. COMPOSITIONS, FORMULATIONS AND METHODS OF ADMINISTRATION

Also provided are compositions containing the chimeric signaling receptor, such as described herein. In some embodiments, the compositions provided herein contain cells engineered to express the chimeric signaling receptor, and optionally a recombinant antigen receptor, e.g., CAR, TCR. In some embodiments, the compositions provided herein contain cells engineered to express the chimeric signaling receptor and a recombinant antigen receptor, e.g., CAR, TCR. Further provided are composition containing engineered cells including pharmaceutical compositions and formulations. Also provided are methods of using and uses of the compositions, such as in the treatment of diseases, conditions, and disorders, such as cancer.

In some embodiments, the composition comprises a plurality of cells engineered with the chimeric signaling receptor. In some embodiments, at least at or about 50% cells (e.g. T cells) in the composition comprise the chimeric signaling receptor. In some embodiments, at least at or about 50% cells (e.g. T cells) in the composition are engineered with or express the chimeric signaling receptor. In some embodiments, at least at or about 60% cells (e.g. T cells) in the composition are engineered with or express the chimeric signaling receptor. In some embodiments, at least at or about 70% cells (e.g. T cells) in the composition are engineered with or express the chimeric signaling receptor. In some embodiments, at least at or about 80% cells (e.g. T cells) in the composition are engineered with or express the chimeric signaling receptor. In some embodiments, at least at or about 90% cells (e.g. T cells) in the composition are engineered with or express the chimeric signaling receptor. In some embodiments, the cells are T cells.

In some embodiments, the composition comprises a plurality of cells engineered with the chimeric signaling receptor and a recombinant antigen receptor (e.g. CAR or TCR). In some embodiments, at least at or about 50% cells (e.g. T cells) in the composition comprise the chimeric signaling receptor and a recombinant antigen receptor (e.g. CAR or TCR). In some embodiments, at least at or about 50% cells (e.g. T cells) in the composition are engineered with or express the chimeric signaling receptor and a recombinant antigen receptor (e.g. CAR or TCR). In some embodiments, at least at or about 60% cells (e.g. T cells) in the composition are engineered with or express the chimeric signaling receptor and a recombinant antigen receptor (e.g. CAR or TCR). In some embodiments, at least at or about 70% cells (e.g. T cells) in the composition are engineered with or express the chimeric signaling receptor and a recombinant antigen receptor (e.g. CAR or TCR). In some embodiments, at least at or about 80% cells (e.g. T cells) in the composition are engineered with or express the chimeric signaling receptor and a recombinant antigen receptor (e.g. CAR or TCR). In some embodiments, at least at or about 90% cells (e.g. T cells) in the composition are engineered with or express the chimeric signaling receptor and a recombinant antigen receptor (e.g. CAR or TCR). In some embodiments, the cells are T cells.

In some embodiments, the composition comprises a plurality of cells engineered with the chimeric signaling receptor and a polynucleotide encoding a recombinant secretable molecule (e.g. bispecific antibody, such as a BiTE, or a cytokine). In some embodiments, at least at or about 50% cells (e.g. T cells) in the composition comprise the chimeric signaling receptor and a polynucleotide encoding a recombinant secretable molecule (e.g. bispecific antibody, such as a BiTE, or a cytokine). In some embodiments, at least at or about 50% cells (e.g. T cells) in the composition are engineered with or express the chimeric signaling receptor and a polynucleotide encoding a recombinant secretable molecule (e.g. bispecific antibody, such as a BiTE, or a cytokine). In some embodiments, at least at or about 60% cells (e.g. T cells) in the composition are engineered with or express the chimeric signaling receptor and a polynucleotide encoding a recombinant secretable molecule (e.g. bispecific antibody, such as a BiTE, or a cytokine). In some embodiments, at least at or about 70% cells (e.g. T cells) in the composition are engineered with or express the chimeric signaling receptor and a polynucleotide encoding a recombinant secretable molecule (e.g. bispecific antibody, such as a BiTE, or a cytokine). In some embodiments, at least at or about 80% cells (e.g. T cells) in the composition are engineered with or express the chimeric signaling receptor and a polynucleotide encoding a recombinant secretable molecule (e.g. bispecific antibody, such as a BiTE, or a cytokine). In some embodiments, at least at or about 90% cells (e.g. T cells) in the composition are engineered with or express the chimeric signaling receptor and a polynucleotide encoding a recombinant secretable molecule (e.g. bispecific antibody, such as a BiTE, or a cytokine). In some embodiments, the cells are T cells.

### A. Compositions/Formulations

Provided herein are compositions containing any of the engineered cells expressing a chimeric signaling receptor described herein. The pharmaceutical composition can further comprise a pharmaceutically acceptable excipient. For example, the pharmaceutical composition can contain one or more excipients for modifying, maintaining or preserving, for example, the pH, osmolarity, viscosity, clarity, color, isotonicity, odor, sterility, stability, rate of dissolution or release, adsorption, or penetration of the composition. In some aspects, a skilled artisan understands that a pharmaceutical composition containing cells may differ from a pharmaceutical composition containing a protein.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some aspects, the choice of carrier is determined in part by the particular cell and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some aspects, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some aspects are included in the compositions. Suitable buffering agents include, for example, citric acid, sodium citrate, phosphoric acid, potassium phosphate, and various other acids and salts. In some aspects, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being treated with the chimeric signaling receptor-expressing cells, preferably those with activities complementary to the cell, where the respective activities do not adversely affect one another. Such active ingredients are suitably present in combination in amounts that are effective for the purpose intended. Thus, in some embodiments, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some embodiments, the chimeric signaling receptor-expressing cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and aryl sulfonic acids, for example, p-toluenesulfonic acid.

The pharmaceutical composition in some embodiments contains engineered cells expressing a chimeric signaling receptor as described herein in amounts effective to treat or prevent the disease or condition, such as a therapeutically effective or prophylactically effective amount. In some embodiments, the pharmaceutical composition contains engineered cells expressing a chimeric signaling receptor as described herein in and a recombinant antigen receptor, e.g., CAR, TCR, amounts effective to treat or prevent the disease or condition, such as a therapeutically effective or prophylactically effective amount. Therapeutic or prophylactic efficacy in some embodiments is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

In some embodiments, engineered cells expressing a chimeric signaling receptor as described herein are administered using standard administration techniques, formulations, and/or devices. In some embodiments, engineered cells expressing a chimeric signaling receptor as described herein and a recombinant antigen receptor, e.g., CAR, TCR, are administered using standard administration techniques, formulations, and/or devices. Provided are formulations and devices, such as syringes and vials, for storage and administration of the compositions. Administration of the engineered cells can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell), it will generally be formulated in a unit dosage injectable form (solution, suspension, emulsion).

Formulations include those for intravenous, intraperitoneal, or subcutaneous, administration. In some embodiments, the cell populations are administered parenterally. The term "parenteral," as used herein, includes intravenous, intramuscular, subcutaneous, rectal, vaginal, and intraperitoneal administration. In some embodiments, the cell populations are administered to a subject using peripheral systemic delivery by intravenous, intraperitoneal, or subcutaneous injection.

Compositions in some embodiments are provided as sterile liquid preparations, e.g., isotonic aqueous solutions, suspensions, emulsions, or dispersions, which may in some aspects be buffered to a selected pH. Liquid compositions are somewhat more convenient to administer, especially by injection. Liquid compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the cells in a solvent, such as in admixture with a suitable carrier, diluent, or excipient such as sterile water, physiological saline, glucose, dextrose, or the like. The compositions can also be lyophilized. The compositions can contain auxiliary substances such as wetting, dispersing, or emulsifying agents (e.g., methylcellulose), pH buffering agents, gelling or viscosity enhancing additives, preservatives, flavoring agents, colors, and the like, depending upon the route of administration and the preparation desired. Standard texts may in some aspects be consulted to prepare suitable preparations.

Various additives which enhance the stability and sterility of the compositions, including antimicrobial preservatives, antioxidants, chelating agents, and buffers, can be added. Prevention of the action of microorganisms can be ensured by various antibacterial and antifungal agents, for example, parabens, chlorobutanol, phenol, sorbic acid, and the like. Prolonged absorption of the injectable pharmaceutical form can be brought about by the use of agents delaying absorption, for example, aluminum monostearate and gelatin.

The formulations to be used for in vivo administration are generally sterile. Sterility may be readily accomplished, e.g., by filtration through sterile filtration membranes.

### B. Methods of Administration

Provided are methods and uses of the provided engineered cells expressing chimeric signaling receptors, and compositions of such engineered cells, in a variety of therapeutic applications, such as to treat or prevent diseases, conditions, and disorders, including cancers. In particular embodiments, the disease, condition or disorder is a cancer and the target cell is a tumor cell. In some embodiments, the engineered cells exhibit therapeutic activity against target cells of the disease, condition or disorder, thereby treating the disease, condition or disorder. In some embodiments, the engineered cells express the provided chimeric signaling receptors and also express a recombinant antigen receptors, such as CARs or TCRs, in which the recombinant antigen receptor is directed against a target antigen associated with or expressed on a target cell of the disease, condition or disorder. In some embodiments, the engineered cells exhibit cytotoxicity against target cells of the disease, disorder, or conditions, such as exhibit cytotoxicity against tumor cells.. In some embodiments, the disease, condition, or disorder is a solid tumor.

Such methods and uses include therapeutic methods and uses, for example, involving administration of the engineered cells, or compositions containing the same, to a subject having a disease, condition, or disorder. In some cases, such as described, the disease or disorder is a tumor or cancer. In some embodiments, the engineered cell is administered in an effective amount to effect treatment of the disease or disorder. Uses include uses of the engineered cells in such methods and treatments, and in the preparation of a medicament in order to carry out such therapeutic methods. In some embodiments, the methods are carried out by administering the engineered cells, or compositions comprising the same, to the subject having or suspected of having the disease or condition. In some embodiments, the methods thereby treat the disease or condition or disorder in the subject.

In some embodiments, the engineered cells, e.g., expressing chimeric signaling receptors or chimeric signaling receptors and recombinant antigen receptors, and compositions thereof are administered to a subject or patient having the particular disease or condition to be treated, e.g., via adoptive cell therapy. In some embodiments, the adoptive cell therapy is T cell therapy. In some embodiments, the adoptive cell therapy is NK cell therapy. In some embodiments, the adoptive cell therapy is TIL cell therapy. In some embodiments, provided engineered cells, e.g., expressing chimeric signaling receptors or chimeric signaling receptors and recombinant antigen receptors, and compositions thereof are administered to a subject, such as a subject having or at risk for the disease or condition. In some aspects, the methods thereby treat, e.g., ameliorate one or more symptom of, the disease or condition.

Methods for administration of engineered cells for adoptive cell therapy are known and may be used in connection with the provided methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

As used herein, a "subject" is a mammal, such as a human or other animal, and typically is human. In some embodiments, the subject, e.g., patient, to whom the cells, cell populations, or compositions are administered is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The disease or condition that is treated can be any in which expression of antigens is associated with and/or involved in the etiology of a disease condition or disorder, e.g. causes, exacerbates or otherwise is involved in such disease, condition, or disorder. In some embodiments, an antigen associated with and/or involved in the etiology of a disease condition or disorder, e.g. causes, exacerbates or otherwise is involved in such disease, condition, or disorder, is a target antigen. An antigen associated with and/or involved in the etiology of a disease condition or disorder, e.g. causes, exacerbates or otherwise is involved in such disease, condition, or disorder, may be referred to herein alternatively as a target antigen. In some embodiments, a target antigen is a tumor antigen. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, e.g., target antigen, which include antigens associated with various diseases and conditions that can be treated, are described above, for example in Section II-B-1. In some embodiments, the chimeric signaling receptor-expressing cells are TILs, which naturally bind a target antigen. In some embodiments, the chimeric signaling receptor-expressing cells are NK cells, which naturally have cytotoxic activity. In some embodiments, the cells engineered to express the chimeric signaling receptor provided herein, are also engineered to express a recombinant antigen receptor, e.g., CAR or TCR, as described herein. In some embodiments, engineered cells expressing the chimeric signaling receptor and a recombinant antigen receptor are T cells or NK cells. In some embodiments, the CAR or recombinant TCR specifically binds to an antigen associated with the disease or condition and the chimeric signaling receptor binds to an immunosuppressive or antiinflammatory cytokine, e.g., TGFβ, IL10, IL4, IL1Ra, present, for example, in a tumor microenvironment.

In some embodiments, the disease or condition to be treated includes cells expressing, e.g., on the cell surface, B7H3. In some embodiments, the target antigen to which the CAR, recombinant TCR, or TIL binds is B7H3. In some embodiments, the disease or condition containing B7H3-expressing cells to be treated is neuroblastoma, ovarian carcinoma, prostate cancer, non-small-cell lung cancer, pancreatic cancer, breast cancer, and/or colorectal cancer. In some embodiments, the disease or condition to be treated is neuroblastoma. In some embodiments, the disease or condition to be treated is ovarian carcinoma.

In some embodiments, the disease or condition to be treated includes cells expressing, e.g., on the cell surface, CD19. In some embodiments, the target antigen to which the CAR, recombinant TCR, or TIL binds is CD19. In some embodiments, the disease or condition containing CD19-expressing cells to be treated is a B cell malignancy. In some embodiments, the B cell malignancy is B cell lymphoma. In some embodiments, the disease or condition to be treated is Non-Hodgkin's lymphoma (NHL) and Hodgkin's lymphoma. In some embodiments, the disease or condition to be treated is diffuse large B-cell lymphoma, follicular lymphoma, marginal zone B-cell lymphoma, mantle cell lymphoma, Hodgkin's lymphoma, acute lymphoblastic leukemia (ALL), or chronic lymphocytic leukemia (CLL), or Burkitt's lymphoma.

Thus, the provided methods and uses include methods and uses for adoptive cell therapy. In some embodiments, the methods include administration of the engineered cells or a composition containing the engineered cells to a subject, tissue, or cell, such as one having, at risk for, or suspected of having the disease, condition or disorder. In some embodiments, the cells, populations, and compositions are administered to a subject having the particular disease or condition to be treated, e.g., via adoptive cell therapy, such as adoptive T cell therapy. In some embodiments, the cells or compositions are administered to the subject, such as a subject having or at risk for the disease or condition, ameliorate one or more symptom of the disease or condition.

In some embodiments, the cell therapy, e.g., adoptive cell therapy, is carried out by autologous transfer, in which the cells are isolated and/or otherwise prepared from the subject who is to receive the cell therapy, or from a sample derived from such a subject. Thus, in some aspects, the cells are derived from a subject, e.g., patient, in need of a treatment and the cells, following isolation and processing are administered to the same subject.

In some embodiments, the cell therapy, e.g., adoptive cell therapy, is carried out by allogeneic transfer, in which the cells are isolated and/or otherwise prepared from a subject other than a subject who is to receive or who ultimately receives the cell therapy, e.g., a first subject. In such embodiments, the cells then are administered to a different subject, e.g., a second subject, of the same species. In some embodiments, the first and second subjects are genetically identical. In some embodiments, the first and second subjects are genetically similar. In some embodiments, the second subject expresses the same HLA class or supertype as the first subject. The cells can be administered by any suitable means. Dosing and administration may depend in part on whether the administration is brief or chronic. Various dosing schedules include but are not limited to single or multiple administrations over various time-points, bolus administration, and pulse infusion.

Dosages may vary depending on attributes particular to the disease or disorder and/or patient and/or other treatments. In some embodiments, the cells are administered as part of a combination treatment, such as simultaneously with or sequentially with, in any order, another therapeutic intervention, such as an antibody or engineered cell or receptor or agent, such as a cytotoxic or therapeutic agent. The cells in some embodiments are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some embodiments, the cells are administered prior to the one or more additional therapeutic agents. In some embodiments, the cells are administered after the one or more additional therapeutic agents. In some embodiments, the methods comprise administration of a chemotherapeutic agent.

Following administration of the engineered cells, the biological activity of the engineered cell populations in some embodiments is measured, e.g., by any of a number of known methods, for example as described in Section III-C. Parameters to assess include specific binding of an engineered or natural cells to antigen, in vivo, e.g., by imaging, or ex vivo, e.g., by ELISA or flow cytometry. In certain embodiments, the ability of the engineered cells to destroy target cells can be measured using any suitable method known in the art, such as cytotoxicity assays described in, for example, Kochenderfer et al., J. Immunotherapy, 32(7): 689-702 (2009), and Herman et al. J. Immunological Methods, 285(1): 25-40 (2004). In certain embodiments, the biological activity of the engineered cells is measured by assaying expression and/or secretion of one or more cytokines, such as CD 107a, IFNγ, IL-2, and TNF. In some aspects the biological activity is measured by assessing clinical outcome, such as reduction in tumor burden or load.

### VII. KITS AND ARTICLES OF MANUFACTURE

Also provided are articles of manufacture or kits containing the provided chimeric signaling receptors, recombinant antigen receptors (e.g., CARs, TCRs), genetically engineered cells, including cells engineered to express the chimeric signaling receptors described herein or cells engineered to co-express the chimeric signaling receptors described herein and a recombinant antigen receptor, for example as described herein, and/or compositions comprising said engineered cells. The articles of manufacture may include a container and a label or package insert on or associated with the container. Suitable containers include, for example, bottles, vials, syringes, test tubes, IV solution bags, etc. The containers may be formed from a variety of materials such as glass or plastic. In some embodiments, the container has a sterile access port. Exemplary containers include an intravenous solution bags, vials, including those with stoppers pierceable by a needle for injection. The article of manufacture or kit may further include a package insert indicating that the compositions can be used to treat a particular condition such as a condition described herein. Alternatively, or additionally, the article of manufacture or kit may further include another or the same container comprising a pharmaceutically-acceptable buffer. It may further include other materials such as other buffers, diluents, filters, needles, and/or syringes.

The label or package insert may indicate that the composition is used for treating a particular disease, disorder or condition in an individual, for example a disease as described herein. The label or a package insert, which is on or associated with the container, may indicate directions for reconstitution and/or use of the formulation. The label or package insert may further indicate that the formulation is useful or intended for subcutaneous, intravenous, or other modes of administration for treating or preventing a disease, disorder or condition in an individual. In some aspects, the label or package insert can include instructions for use, for example instructions for administering the engineered cells, engineering cells to express the chimeric signaling receptor described herein, engineering cells to co-express the chimeric signaling receptor described herein and a recombinant antigen receptor, in some aspects in accordance with any of the methods or uses described herein.

The container in some embodiments holds a composition which is effective for treating and/or preventing a disease or condition, e.g., cancer. The article of manufacture or kit may include a container with a composition contained therein, wherein the composition includes engineered cells expressing the chimeric signaling receptors provided herein, and which article or kit further comprises instructions on the label or package insert for treating the subjects with a therapeutically effective amount of engineered cells. The article of manufacture or kit may include a container with a composition contained therein, wherein the composition includes engineered cells expressing the chimeric signaling receptors provided herein and a recombinant antigen receptor, e.g., CAR or TCR, and which article or kit further comprises instructions on the label or package insert for treating the subjects with a therapeutically effective amount of engineered cells.

### VIII. Definitions

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used herein, the singular forms "a," "an," and "the" include plural referents unless the context clearly dictates otherwise. For example, "a" or "an" means "at least one" or "one or more." It is understood that aspects and variations described herein include "consisting" and/or "consisting essentially of" aspects and variations.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

The term "about" as used herein refers to the usual error range for the respective value readily known to the skilled person in this technical field. Reference to "about" a value or parameter herein includes (and describes) embodiments that are directed to that value or parameter per se. For example, description referring to "about X" includes description of "X".

As used herein, domain (typically a sequence of three or more, generally 5 or 7 or more amino acids, such as 10 to 200 amino acid residues) refers to a portion of a molecule, such as a protein or encoding nucleic acid, that is structurally and/or functionally distinct from other portions of the molecule and is identifiable. For example, domains include those portions of a polypeptide chain that can form an independently folded structure within a protein made up of one or more structural motifs and/or that is recognized by virtue of a functional activity, such as binding activity. A protein can have one, or more than one, distinct domains. For example, a domain can be identified, defined or distinguished by homology of the primary sequence or structure to related family members, such as homology to motifs. In another example, a domain can be distinguished by its function, such as an ability to interact with a biomolecule, such as interactions between domains of a MyD88 adaptor and an IRAK4 signaling kinase. A domain independently can exhibit a biological function or activity such that the domain independently or fused to another molecule can perform an activity, such as, for example binding. A domain can be a linear sequence of amino acids or a non-linear sequence of amino acids. Many polypeptides contain a plurality of domains. Such domains are known, and can be identified by those of skill in the art. For exemplification herein, definitions are provided, but it is understood that it is well within the skill in the art to recognize particular domains by name. If needed appropriate software can be employed to identify domains.

The term "extracellular domain" or "ectodomain," which can be used interchangeably, as used herein refers to the region of a membrane protein, such as a transmembrane protein, that lies outside the vesicular membrane. Ectodomains often comprise binding domains that specifically bind to ligands or cell surface receptors, such as via a binding domain that specifically binds to the ligand or cell surface receptor.

The term "endodomain" or "intracellular domain," or "cytoplasmic domain" which can be used interchangeably, as used herein refers to the region found in some membrane proteins, such as transmembrane proteins, that extends into the interior space defined by the cell surface membrane. In mammalian cells, the endodomain is the cytoplasmic region of the membrane protein. In cells, the endodomain interacts with intracellular constituents and can be play a role in signal transduction and thus, in some cases, can be an intracellular signaling domain. The endodomain of a cellular transmembrane protein is alternately referred to as a cytoplasmic domain, which, in some cases, can be a cytoplasmic signaling domain.

The term "transmembrane domain" as used herein means a domain found in a membrane protein that substantially or completely spans a lipid bilayer such as those lipid bilayers found in a biological membrane such as a mammalian cell, or in an artificial construct such as a liposome. Transmembrane domains are generally predictable from their amino acid sequence via any number of commercially available bioinformatics software applications on the basis of their elevated hydrophobicity relative to regions of the protein that interact with aqueous environments (e.g., cytosol, extracellular fluid). A transmembrane domain is often a hydrophobic alpha helix that spans the membrane. A transmembrane protein can pass through the both layers of the lipid bilayer once or multiple times.

The term "specifically binds" as used herein means the ability of a protein, under specific binding conditions, to bind to a target protein such that its affinity or avidity is at least 5 times as great, but optionally at least 10, 20, 30, 40, 50, 100, 250 or 500 times as great, or even at least 1000 times as great as the average affinity or avidity of the same protein to a collection of random peptides or polypeptides of sufficient statistical size. A specifically binding protein need not bind exclusively to a single target molecule but may specifically bind to a non-target molecule due to similarity in structural conformation between the target and non-target (e.g., paralogs or orthologs). Those of skill will recognize that specific binding to a molecule having the same function in a different species of animal (i.e., ortholog) or to a non-target molecule having a substantially similar epitope as the target molecule (e.g., paralog) is possible and does not detract from the specificity of binding which is determined relative to a statistically valid collection of unique non-targets (e.g., random polypeptides). Thus, a polypeptide may specifically bind to more than one distinct species of target molecule due to cross-reactivity. Solid-phase ELISA immunoassays or Biacore measurements can be used to determine specific binding between two proteins. Generally, interactions between two binding proteins have dissociation constants (Kd) less than 1x10-5 M, and often as low as 1 x 10-12 M. In certain embodiments of the present disclosure, interactions between two binding proteins have dissociation constants of 1x10-6 M, 1x10-7 M, 1x10-8 M, 1x10-9 M, 1x10-10 M or 1x10-11 M.

As used herein, a composition refers to any mixture of two or more products, substances, or compounds, including cells. It may be a solution, a suspension, liquid, powder, a paste, aqueous, non-aqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100 % in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control under otherwise identical conditions, and/or at a level substantially lower than that for cell known to be positive for the marker, and/or at a level substantially similar as compared to that for a cell known to be negative for the marker.

The term "expression", as used herein, refers to the process by which a polypeptide is produced based on the encoding sequence of a nucleic acid molecule, such as a gene. The process may include transcription, post-transcriptional control, post-transcriptional modification, translation, post-translational control, post-translational modification, or any combination thereof.

As used herein, a subject includes any living organism, such as humans and other mammals. Mammals include, but are not limited to, humans, and non-human animals, including farm animals, sport animals, rodents and pets.

As used herein, a control refers to a sample that is substantially identical to the test sample, except that it is not treated with a test parameter, or, if it is a plasma sample, it can be from a normal volunteer not affected with the condition of interest. A control also can be an internal control.

As used herein, "operably linked" or "operatively linked" refers to the association of components, such as a DNA sequence, e.g. a heterologous nucleic acid) and a regulatory sequence(s), in such a way as to permit gene expression when the appropriate molecules (e.g. transcriptional activator proteins) are bound to the regulatory sequence. Hence, it means that the components described are in a relationship permitting them to function in their intended manner.

As used herein, "percent (%) sequence identity" and "percent identity" when used with respect to a nucleotide sequence (reference nucleotide sequence) or amino acid sequence (reference amino acid sequence) is defined as the percentage of nucleotide residues or amino acid residues, respectively, in a candidate sequence that are identical with the residues in the reference sequence, after aligning the sequences and introducing gaps, if necessary, to achieve the maximum percent sequence identity. Alignment for purposes of determining percent sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Among the vectors are viral vectors, such as lentiviral vectors.

### IX. EXEMPLARY EMBODIMENTS

Among the provided embodiments are:
1. A chimeric signaling receptor comprising:
   (a) an extracellular domain of a TGFβ receptor (TGFβR) or a portion thereof that binds TGFβ;
   (b) a transmembrane domain;
   (c) a first truncated MyD88 polypeptide that lacks the full-length TIR domain of full-length MyD88; and
   (d) a second truncated MyD88 domain that lacks the full-length TIR domain of full-length MyD88.
2. The chimeric signaling receptor of embodiment 1, wherein the TGFβR is a TGFβR2.
3. The chimeric signaling receptor of embodiment 1 or embodiment 2, wherein the extracellular domain or the portion thereof comprises:
   (i) the sequence of amino acids set forth in SEQ ID NO: 20;
   (ii) a sequence of amino acids that exhibits at least or about 85%, at least or about 90%, at least or about 92%, at least or about 95%, at least or about 97% sequence identity to the sequence set forth in SEQ ID NO: 20; or
   (iii) a portion of (i) or (ii) that binds TGFβ.
4. The chimeric signaling receptor of any of embodiments 1-3, wherein the extracellular domain or the portion thereof is set forth in SEQ ID NO:20.
5. The chimeric signaling receptor of any of embodiments 1-4, wherein the transmembrane domain comprises the native transmembrane domain of the TGFβR.
6. The chimeric signaling receptor of any of embodiments 1-5, wherein the transmembrane domain comprises the sequence of amino acids set forth in SEQ ID NO: 22 or a sequence of amino acids that exhibits at least or about 85%, at least or about 90%, at least or about 92%, at least or about 95%, at least or about 97% sequence identity to the sequence set forth in SEQ ID NO: 22.
7. The chimeric signaling receptor of any of embodiments 1-6, wherein the transmembrane domain is set forth in SEQ ID NO:22.
8. The chimeric signaling receptor of any of embodiments 1-4, wherein the transmembrane domain is a heterologous transmembrane domain from a transmembrane protein other than the TGFβR.
9. The chimeric signaling receptor of any of embodiments 1-8, wherein the transmembrane domain and the first truncated MyD88 polypeptide are directly linked.
10. The chimeric signaling receptor of any of embodiments 1-9, wherein the transmembrane domain and the first truncated MyD88 polypeptide are indirectly linked by a linker.
11. The chimeric signaling receptor of embodiment 10, wherein the linker is or comprises a peptide linker.
12. The chimeric signaling receptor of embodiment 10, wherein the linker is or comprises a partial sequence of N-terminal contiguous amino acids of the cytoplasmic domain of the TGFβR that is a non-functional portion, wherein the non-functional portion is not a functional inhibitory signaling domain capable of mediating inhibitory signaling.
13. The chimeric signaling receptor of any of embodiments 1-12, wherein the intracellular domain is not capable of recruiting an inhibitory adaptor molecule, optionally a SMAD.
14. The chimeric signaling receptor of embodiment 10, 12 or 13, wherein the linker is amino acids 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, or 1-25 of the TGFβR2 cytoplasmic domain set forth in SEQ ID NO:129, optionally wherein the linker is set forth in SEQ ID NO:24.
15. A chimeric signaling receptor comprising (i) a portion of a TGFβ receptor (TGFβR) comprising the extracellular domain and the transmembrane domain, wherein the portion is less than the full-length TGFβR and lacks a functional inhibitory signaling domain of the full-length TGFβR; and (ii) an intracellular domain comprising a first truncated MyD88 polypeptide that lacks the full-length TIR domain of full-length MyD88, and a second truncated MyD88 domain that lacks the full-length TIR domain of full-length MyD88.
16. The chimeric signaling receptor of embodiment 15, wherein the TGFβR is a TGFβR2.
17. The chimeric signaling receptor of embodiment 15 or embodiment 16, wherein the portion of the TGFβR comprises the sequence of amino acids set forth in SEQ ID NO: 3 or a sequence of amino acids that exhibits at least or about 85%, at least or about 90%, at least or about 92%, at least or about 95%, at least or about 97% sequence identity to the sequence set forth in SEQ ID NO: 3.
18. The chimeric signaling receptor of any of embodiments 15-17, wherein the portion of the TGFβR is set forth in SEQ ID NO:3.
19. The chimeric signaling receptor of any of embodiments 15-18, wherein the portion of the TGFβR and the intracellular domain are directly linked.
20. The chimeric signaling receptor of any of embodiments 15-18, wherein the portion of the TGFβR and the intracellular domain are indirectly linked by a peptide linker.
21. The chimeric signaling receptor of any of embodiments 1-20, wherein the first truncated MyD88 polypeptide and the second MyD88 polypeptide each comprise the death domain (DD), the intermediate domain (ID) and a portion of the full-length TIR domain of MyD88.
22. The chimeric signaling receptor of any of embodiments 1-21, wherein the first truncated MyD88 polypeptide and the second MyD88 polypeptide each independently is a sequence of amino acids selected from the group consisting of amino acids 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179 or 2-180 of the full-length MyD88, optionally of SEQ ID NO:128.
23. The chimeric signaling receptor of any of embodiments 1-21, wherein the first truncated MyD88 polypeptide and the second truncated polypeptide each independently is a sequence of amino acids 2-171 or 2-172 of the full-length MyD88, optionally of SEQ ID NO: 128.
24. The chimeric signaling receptor of any of embodiments 1-23, wherein the first truncated MyD88 polypeptide is set forth in SEQ ID NO: 2.
25. The chimeric signaling receptor of any of embodiments 1-24, wherein the second truncated MyD88 polypeptide is set forth in SEQ ID NO: 2.
26. The chimeric signaling receptor of any of embodiments 1-25, wherein the first truncated MyD88 polypeptide and the second truncated MyD88 polypeptide are the same.
27. The chimeric signaling receptor of any of embodiments 1-26, wherein the first MyD88 polypeptide and the second MyD88 polypeptide are connected by a peptide linker.
28. The chimeric signaling receptor of embodiment 11, 20 and 27, wherein the peptide linker is (G₄S)ₙ (SEQ ID NO: 47), wherein n is an integer between 1 to 4, inclusive.
29. The chimeric signaling receptor of embodiment 11, 20, 27 and 28, wherein the peptide linker is set forth in SEQ ID NO: 47 ((GGGGS)ₙ), where n is an integer between 1 and 4, inclusive, optionally wherein the peptide linker is selected from the group consisting of SEQ ID NO:48 (GGGGS), SEQ ID NO: 85 (GGGGSGGGGS), SEQ ID NO: 49 (GGGGSGGGGSGGGGS), and SEQ ID NO: 50 (GGGGSGGGGSGGGGSGGGGS).
30. The chimeric signaling receptor of any of embodiments 1-29, wherein the chimeric signaling receptor is set forth in SEQ ID NO:93 or a sequence of amino acids that exhibits at least at or about 85%, at least at or about 90%, at least at or about 92%, at least at or about 95%, or at least at or about 97% sequence identity to the sequence set forth in SEQ ID NO:93.
31. The chimeric signaling receptor of any of embodiments 1-30, wherein the chimeric signaling receptor is set forth in SEQ ID NO:93.
32. The chimeric signaling receptor of any of embodiments 1-29, wherein the chimeric signaling receptor is set forth in SEQ ID NO:94 or a sequence of amino acids that exhibits at least at or about 85%, at least at or about 90%, at least at or about 92%, at least at or about 95%, or at least at or about97% sequence identity to the sequence set forth in SEQ ID NO:94.
33. The chimeric signaling receptor of any of embodiments 1-32, wherein the chimeric signaling receptor is set forth in SEQ ID NO:94.
34. The chimeric signaling receptor of any of embodiments 1-33, wherein, when the chimeric signaling receptor is expressed from a cell, binding of TGFβ to the extracellular domain induces dimerization of the first and second MyD88 polypeptide.
35. The chimeric signaling receptor of any of embodiments 1-34, wherein, when the chimeric signaling receptor is expressed from a cell, binding of TGFβ to the extracellular domain recruits an IRAK4 to the first MyD88 polypeptide and the second MyD88 polypeptide.
36. The chimeric signaling receptor of any of embodiments 1-35, wherein, when the chimeric signaling receptor is expressed from a cell, binding of TGFβ to the extracellular domain results in phosphorylation of IRAK4 .
37. A polynucleotide comprising a sequence of nucleotides encoding the chimeric signaling receptor of any of embodiments 1-36.
38. The polynucleotide of embodiment 37, wherein the sequence of nucleotides is a first sequence of nucleotides and the polynucleotide further comprises a second sequence of nucleotides encoding a recombinant antigen receptor.
39. The polynucleotide of embodiment 38, wherein the recombinant antigen receptor is a recombinant T cell receptor (TCR) or is a chimeric antigen receptor (CAR).
40. The polynucleotide of embodiment 38 or 39, wherein the recombinant antigen receptor is a CAR.
41. The polynucleotide of any of embodiments 38-40, wherein the recombinant antigen receptor binds a tumor antigen.
42. The polynucleotide of embodiment 41, wherein the tumor antigen is a CD19.
43. The polynucleotide of embodiment 41, wherein the tumor antigen is a B7H3.
44. The polynucleotide of any of embodiments 38-43, wherein the first sequence of nucleotides and the second sequence of nucleotides are separated by a bicistronic element.
45. The polynucleotide of embodiment 44, wherein the bicistronic element is an IRES or is a cleavable peptide.
46. The polynucleotide of embodiment 45, wherein the cleavable peptide is selected from the group consisting of a P2A, a T2A and an F2A.
47. The polynucleotide of embodiment 45 or embodiment 46, wherein the cleavable peptide is a T2A.
48. The polynucleotide of any of embodiments 37-47, wherein the sequence of nucleotides is operably linked to a promoter.
49. The polynucleotide of any of embodiments 38-47, wherein the first sequence of nucleotides and the second sequence of nucleotides are each operably linked to a promoter.
50. The polynucleotide of embodiment 49, wherein the promoter is the same promoter.
51. The polynucleotide of any of embodiments 48-50, wherein the promoter is an EF1 promoter.
52. A vector comprising the polynucleotide of embodiment 37, 48 or 51.
53. A vector comprising the polynucleotide of any of embodiments 37-51.
54. The vector of embodiment 52 or embodiment 53, wherein the vector is a viral vector.
55. The vector of embodiment 54, wherein the viral vector is a retroviral vector.
56. The vector of embodiment 54 or embodiment 55, wherein the viral vector is a lentiviral vector.
57. A method of producing an engineered cell, the method comprising introducing the polynucleotide of any of embodiments 38-51 or the vector of any of embodiments 53-56 into a cell under conditions for expression of the chimeric signaling receptor and the recombinant antigen receptor on the surface of the cell.
58. A method of producing an engineered cell, the method comprising introducing the polynucleotide of embodiment 37-51 or the vector of any of embodiments 52-56 into a cell under conditions for expression of the chimeric signaling receptor on the surface of the cell.
59. The method of embodiment 58, further comprising introducing a polynucleotide encoding a recombinant antigen receptor into the cell under conditions for expression of the recombinant antigen receptor on the surface of the cell.
60. The method of embodiment 59, wherein the recombinant antigen receptor is a recombinant T cell receptor (TCR) or is a chimeric antigen receptor (CAR).
61. The method of embodiment 59 or embodiment 60, wherein the recombinant antigen receptor is a CAR.
62. The method of embodiment 60 or embodiment 61, wherein the recombinant antigen receptor binds a tumor antigen.
63. The method of embodiment 62, wherein the tumor antigen is a CD19.
64. The method of embodiment 62, wherein the tumor antigen is a B7H3.
65. The method of any of embodiments 57-64, wherein the cell is a primary cell from a subject.
66. The method of any of embodiments 57-65, wherein the cell is a T cell, a tumor-infiltrating cell (TIL), a B cell, a natural killer cell, or a macrophage.
67. The method of any of embodiments 57-66, wherein the cell is a T cell, optionally a CD3+, CD4+, or CD8+ T cells.
68. The method any of embodiments 57-67, wherein the cell is a CD8+ T cell.
69. The method any of embodiments 57-67, wherein the cell is a CD4+ T cell.
70. An engineered cell produced by the method of any of embodiments 57-69.
71. An engineered cell comprising a chimeric signaling receptor of any of embodiments 1-36.
72. The engineered cell of embodiment 71, wherein the engineered cell further comprises a recombinant antigen receptor.
73. The engineered cell of embodiment 72, wherein the recombinant antigen receptor is a recombinant T cell receptor (TCR) or is a chimeric antigen receptor (CAR).
74. An engineered cell comprising a polynucleotide of embodiment 37.
75. The engineered cell of embodiment 74, further comprising a polynucleotide encoding a recombinant antigen receptor.
76. The engineered cell of embodiment 75, wherein the recombinant antigen receptor is a recombinant T cell receptor (TCR) or is a chimeric antigen receptor (CAR).
77. The engineered cell of any of embodiments 72-73 and 75-76, wherein the recombinant antigen receptor is a CAR.
78. The engineered cell of any of embodiments 72-72 and 75-77, wherein the recombinant antigen receptor binds a tumor antigen.
79. The engineered cell of embodiment 78, wherein the tumor antigen is a CD19.
80. The engineered cell of embodiment 78, wherein the tumor antigen is a B7H3.
81. An engineered cell comprising a polynucleotide of any of embodiments 38-51.
82. The engineered cell of any of embodiments 70-81, wherein the cell is a primary cell from a subject.
83. The engineered cell of any of embodiments 70-82, wherein the engineered cell is a T cell, a tumor infiltrating lymphocyte (TIL), a B cell, a natural killer (NK) cell, or a macrophage.
84. The engineered cell of any of embodiments 70-83, wherein the engineered cell is a T cell, optionally a CD3+, CD4+, and/or CD8+ T cells.
85. The engineered cell of any of embodiments 70-84, wherein the engineered cell is a CD8+ T cell.
86. The engineered cell of any of embodiments 70-85, wherein the engineered cell is a CD4+ T cell.
87. A pharmaceutical composition comprising the engineered cell of any of embodiments 70-86.
88. The pharmaceutical composition of embodiment 87 further comprising a pharmaceutically acceptable excipient.
89. The pharmaceutical composition of embodiment 87 or embodiment 88 wherein the pharmaceutical composition is sterile.
90. A method of treatment comprising administering the engineered cell of any of embodiments 70-86 or the pharmaceutical composition of any of embodiments 87-89 to a subject that has a cancer.
91. The method of embodiment 90, wherein the engineered cells comprise an antigen receptor targeted against an antigen of the cancer.
92. The method of embodiment 90, wherein the engineered cells express a recombinant antigen receptor comprising an antigen-binding domain that binds to a tumor antigen associated with the cancer, optionally where cells of the cancer express the tumor antigen.
93. The method of embodiment 92, wherein the recombinant antigen receptor is a chimeric antigen receptor (CAR) or a T cell receptor (TCR).
94. The method of embodiment 92 or embodiment 93, wherein the recombinant antigen receptor is a CAR.
95. The method of any of embodiments 90-94, wherein the cancer is a hematologic cancer or is a solid tumor.
96. The method of any of embodiments 92-95, wherein the tumor antigen is a B7H3.
97. The method of any of embodiments 92-96, wherein the cancer is a prostate cancer, melanoma, Head and neck squamous cell carcinoma (HNSCC), non-small cell lung cancer (NSCLC), urothelial cancer, ovarian cancer, neuroblastoma, rhabdomyosarcoma, osteosarcoma, Ewing sarcoma, Wilms' tumor, optionally wherein the cancer is an ovarian carcinoma or a neuroblastoma.
98. The method of any of embodiments 92-95, wherein the tumor antigen is a CD19.
99. The method of any of embodiments 90-95 and 98, wherein the cancer is a B cell cancer.
100. The method of any of embodiments 90-95, 98 and 99, wherein the cancer is a leukemia or a lymphoma, optionally metastatic lymphoma.

### X. EXAMPLES

The following examples are included for illustrative purposes only and are not intended to limit the scope of the invention.

### Example 1 Generation of Chimeric TGFβ Signaling Receptors

Polynucleotides encoding chimeric TGFβ signaling receptors including human TGFβR extracellular domains connected to myeloid differentiation primary response 88 (MyD88) polypeptides were generated. MyD88 is an adaptor signaling molecule that provides positive cell signaling to promote T cell activation, and is normally part of Toll-like receptor (TLR) (see, **FIG. 1**). The constructs were generated to contain the exemplary MyD88 sequence set forth in SEQ ID NO: 2, which contains three domains: (1) a death domain (DD; SEQ ID NO: 10); (2) an intermediate domain (ID; SEQ ID NO: 12); and (3) a termination immediate domain (TIR; SEQ ID NO: 14). The constructs were generated to assess the ability of MyD88 to reverse TGFβ inhibitory signaling through activation of MyD88 independent of TLR.

### A. Polynucleotide Constructs

Polynucleotide constructs encoding exemplary chimeric TGFβ signaling receptors were designed to encode from N- to C-terminus: a human TGFβR extracellular domain from either TGFβR2 (e.g., a SEQ ID NO: 20) or TGFβR1 (SEQ ID NO: 4), a TGFβR transmembrane domain from either TGFβR2 (e.g., SEQ ID NO: 22) or TGFβR1 (SEQ ID NO: 6), and an intracellular domain that included at least one MyD88 polypeptide (SEQ ID NO:2). In some cases, the intracellular domain included two MyD88 polypeptides in tandem, a first MyD88 polypeptide and a second MyD88 polypeptide, each set forth in SEQ ID NO:2. In the constructs, a linker sequence (L1), containing a contiguous non-signaling portion of the intracellular domain of the respective TGFβR or a heterologous flexible peptide linker sequence was positioned between the transmembrane domain and the MyD88 polypeptide. In addition, in some cases, a flexible peptide linker sequence (L2) separated the first MyD88 polypeptide and the second MyD88 polypeptide in constructs containing MyD88 in tandem. Exemplary flexible peptide linker sequences include single or repeating GGGGS motifs, such as shown in SEQ ID NOs: 48, 50, or 85. Control constructs also were generated that did not include an intracellular domain with a MyD88 adaptor polypeptide sequence or did not include a TGFβR extracellular domain. All constructs also included a polyhistidine tag (HHHHHH, SEQ ID NO: 103) or myc-tag (EQKLISEEDL, SEQ ID NO: 101) to aid in purification and detection. The encoding polynucleotides also included a signal sequence encoding a CD8α signal peptide (MALPVTALLLPLALLLHAARP, SEQ ID NO: 102).

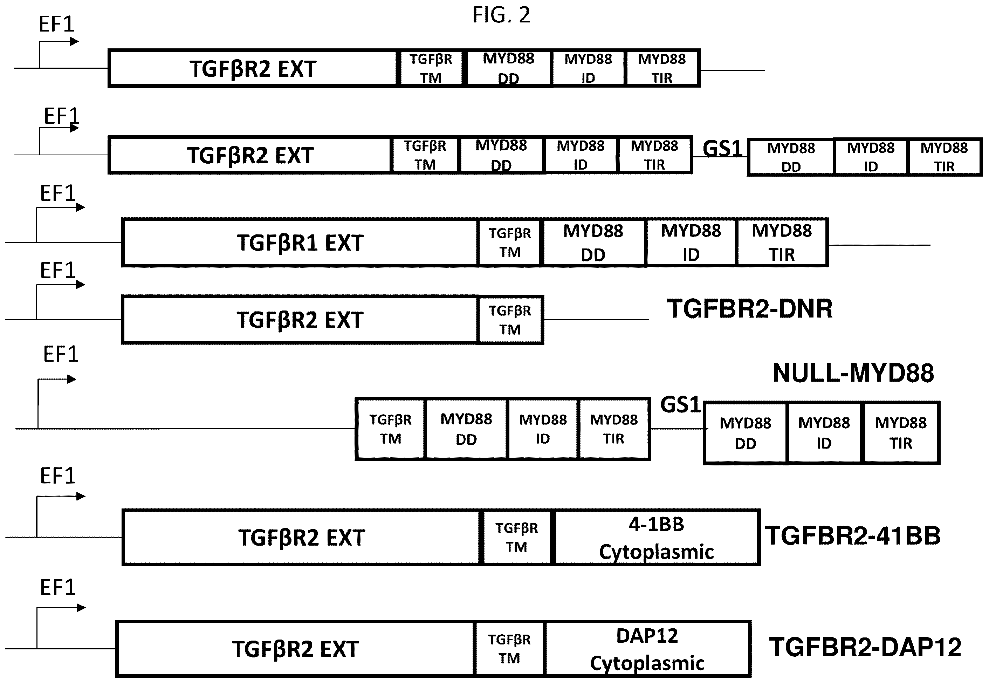

Exemplary chimeric TGFβ signaling receptor polypeptides encoded by the polynucleotide constructs are shown in Table E1. Exemplary control signaling receptor polypeptides encoded by the polynucleotide constructs are shown in Table E2. **FIG. 2** also depicts generated constructs.

| **Table E1: Exemplary chimeric TGFβ signaling receptor encoded by polynucleotide constructs** | | | | | | | | | | |
|---|---|---|---|---|---|---|---|---|---|---|
| | SEQ ID NO | Tag | Precursor Constructs (SEQ ID NOs) | | TGFβ ECD (SEQ ID NO) | TM (SEQ ID NO) | L1 (SEQ ID NO) | MyD88 (SEQ ID NO) | L2 (SEQ ID NO) | MyD88 (SEQ ID NO) |
| | | | Amino Acid | Nucleic Acid | | | | | | |
| **CTSR-1A** | 90 | 103 | 16 | 17 | 4 | 6 | 8 | 2 | - | - |
| **CTSR-1B** | 91 | - | 18 | 19 | 4 | 6 | 85 | 2 | - | - |
| **CTSR-2A** | 92 | 101 | 26 | 27 | 20 | 22 | 24 | 2 | - | - |
| **CTSR-2B** | 93 | 101 | 28 | 29 | 20 | 22 | 24 | 2 | - | 2 |
| **CTSR-2C** | 94 | 101 | 30 | 31 | 20 | 22 | 24 | 2 | 48 | 2 |

| | | | | | | | | | | |
|---|---|---|---|---|---|---|---|---|---|---|
| ECD: Extracellular domain; TM: Transmembrane domain | | | | | | | | | | |

| **Table E2: Exemplary control chimeric signaling receptors encoded by polynucleotide constructs** | | | | | | | | | | |
|---|---|---|---|---|---|---|---|---|---|---|
| Name | SEQ ID NO | Tag | Precursor Constructs (SEQ ID NOs) | | TGFβ ECD (SEQ ID NO) | TM (SEQ ID NO) | L1 (SEQ ID NO) | MyD88 (SEQ ID NO) | L2 (SEQ ID NO) | MyD88 (SEQ ID NO) |
| | | | Amino Acid | Nucleic Acid | | | | | | |
| **CTSR-2IN** | 95 | 101 | 32 | 33 | 20 | 22 | 86 | - | - | - |
| **CTSR-2EN** | 97 | 101 | 36 | 37 | - | 22 | 24 | 2 | 48 | 2 |

| | | | | | | | | | | |
|---|---|---|---|---|---|---|---|---|---|---|
| ECD: Extracellular domain; TM: Transmembrane domain | | | | | | | | | | |

### B. Cell Expression Assays

To assess expression of chimeric TGFβ signaling receptors, polynucleotide constructs encoding exemplary chimeric TGFβ signaling receptors and controls were cloned into third generation lentiviral vectors. The sequences encoding the chimeric TGFβ signaling receptors were placed under the operable control of the human elongation factor 1 alpha (EF1α) promoter (sequence set forth in SEQ ID NO: 112).

Jurkat cells or primary human T cells were transduced with vectors encoding the exemplary TGFβ signaling receptors CTSR-1A, CTSR-2A, or CTSR-2C, or vectors encoding the control TGFβ signaling receptor CTSR-2IN. In some cases, primary human T cells were co-transduced with a polynucleotide encoding a chimeric antigen receptor (CAR) including an extracellular antigen-binding domain containing a single chain variable fragment (scFv) (in this case, targeting B7H3 or CD19).

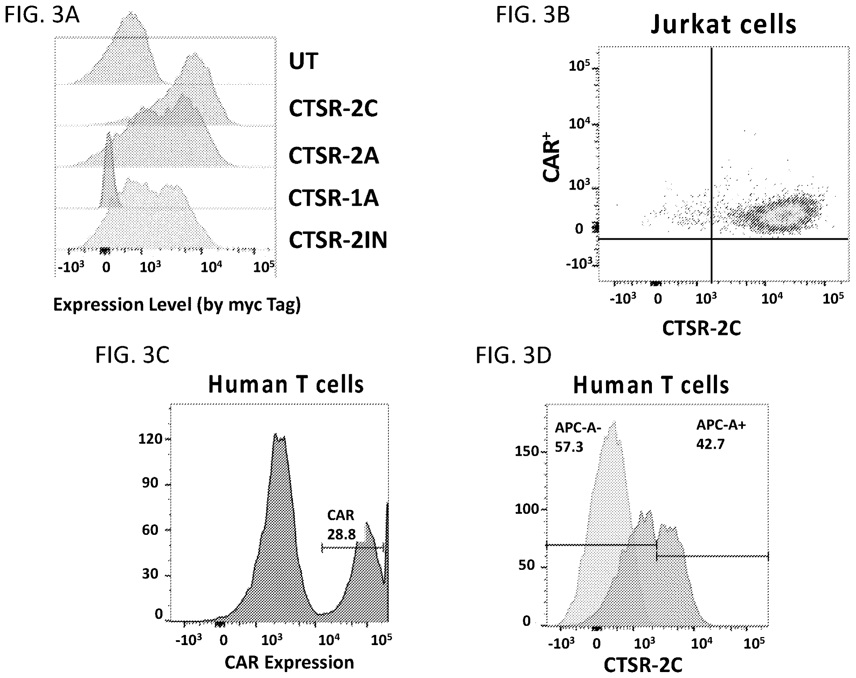

**FIG. 3A** shows expression levels of myc-tag in untransduced (UT) and transduced Jurkat cells determined by flow cytometry. These data show that the exemplary chimeric TGFβ signaling receptors and controls including a myc-tag were expressed in Jurkat cells.

As shown in **FIG. 3B****,** flow cytometric analysis revealed high efficiency in expression of both the CAR and the exemplary chimeric TGFβ signaling receptor CTSR-2C in Jurkat cells.

**FIG. 3C** and **FIG. 3D** show expression efficiency of the CAR and the exemplary chimeric TGFβ signaling receptor CTSR-2C in primary human T cells, respectively.

These results demonstrate the ability of the exemplary chimeric TGFβ signaling receptors to be expressed in T cells, including when co-transduced with a CAR.

### Example 2 Assessment of Chimeric TGFβ Signaling Receptors on the TGFβ Signaling Pathway

The ability of the exemplary chimeric TGFβ signaling receptors to block TGFβR signaling via its native inhibitory pathway and to engage the MyD88 pathway for T cell activation was assessed.

Jurkat cells were transduced with vectors encoding the exemplary chimeric TGFβ signaling receptors CTSR-2A or CTSR-2C. Transduced cells were incubated with 0, 1, 5, or 10 ng/mL of TGFβ for 6 hours prior to protein extraction. Western blot analysis was used to determine the amount of phosphorylated SMAD 2 (pSMAD2), phosphorylated IRAK4 (pIRAK4), and SMAD 2 protein levels.

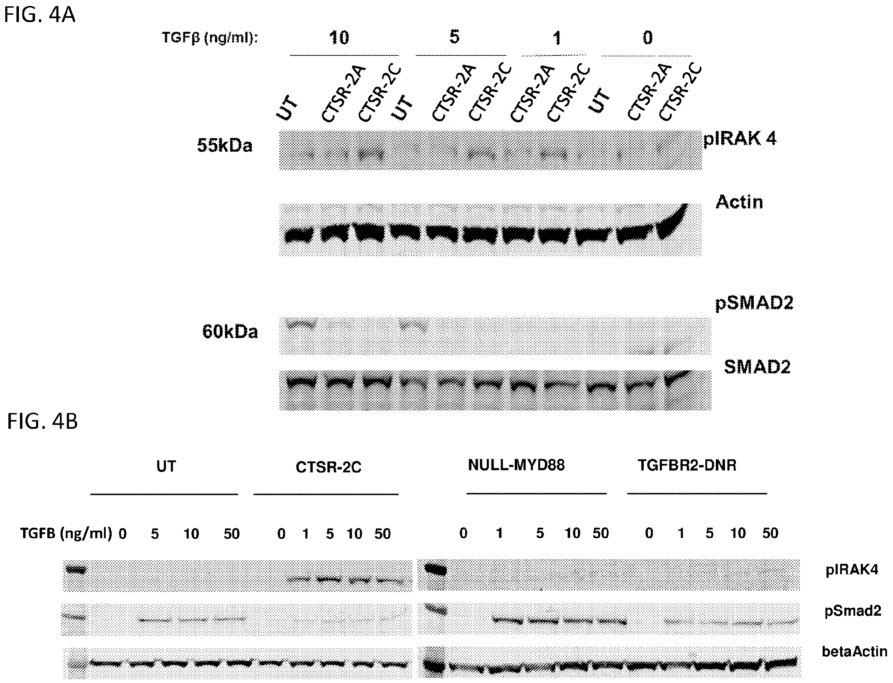

As shown in **FIG. 4****,** cells transduced with exemplary chimeric TGFβ signaling receptors showed decreased levels of pSMAD2 compared to those transduced with TGFβR2-DNR, NULL-MYD88, or untransduced (UT) Jurkat cells. These results indicate that the exemplary chimeric TGFβ signaling receptors reduce signaling through the native TGFβR signaling pathway. **FIG. 4** also shows an increase in the level of pIRAK4 in cells transduced with the exemplary chimeric TGFβ signaling receptors, particularly in the chimeric construct CTSR-2C containing two MyD88 domains in tandem, compared to untransduced control cells. These results indicate that the exemplary chimeric TGFβ signaling receptors engage the activating MyD88 pathway.

### Example 3 Assessment of Chimeric TGFβ Signaling Receptor Signaling on T Cell Proliferation

To assess the ability of the exemplary chimeric TGFβ signaling receptors to induce T cell proliferation, a CFDA-SE (5[6]-carboxyfluorescein diacetate succinimidyl ester) proliferation assay was performed.

Human T cells were labeled with carboxyfluorescein diacetate succinimidyl ester (CFDA-SE) and transduced with a vector encoding the exemplary chimeric TGFβ signaling receptor CTSR-2C, a vector encoding an exemplary CAR (anti-B7H3 CAR; SEQ ID NO: 45), or two vectors, one encoding the exemplary TGFβ signaling receptor and the other encoding the exemplary CAR.

Transduced cells were incubated with TGFβ at 1, 5, 10, or 20 ng/mL and human neuroblastoma cell line CHLA255 target cells at an effector to target ratio of 1:1. Target cells were replenished every 48 hours. After 6 days of exposure, CFDA intensity was measured.

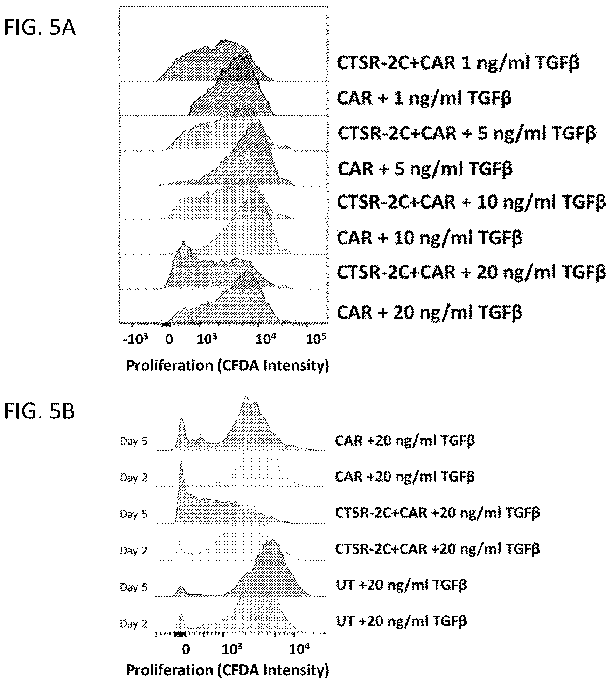

**FIG. 5A** shows CFDA intensity after 6 days of exposure to target cells in the presence of TGFβ. These data indicate that the presence of the exemplary chimeric TGFβ signaling receptor induces T cell proliferation.

CFDA intensity was also assessed on days 2 and 5 in the presence of 20 ng/mL TGFβ and target cells (E:T of 1:1, replenished every 48 hours). As shown in **FIG. 5B****,** cells expressing both the exemplary chimeric TGFβ signaling receptor and the CAR showed increased proliferation compared to the CAR alone or untransduced (UT) control cells.

T cell proliferation was also assessed directly by determining changes in T cell count during incubation with TGFβ. Human T cells were transduced with a vector encoding the exemplary chimeric TGFβ signaling receptors CTSR-2A or CTSR-2C and a vector encoding an exemplary CAR (anti-B7H3 CAR; SEQ ID NO: 45), or the exemplary CAR alone. Transduced cells were cultured with 0, 10, or 50 ng/mL of TGFβ and human neuroblastoma cell line CHLA255 target cancer cells (E:T of 1:1, replenished every 48 hours) for 3 weeks. T cell counts were determined over the duration of culturing.

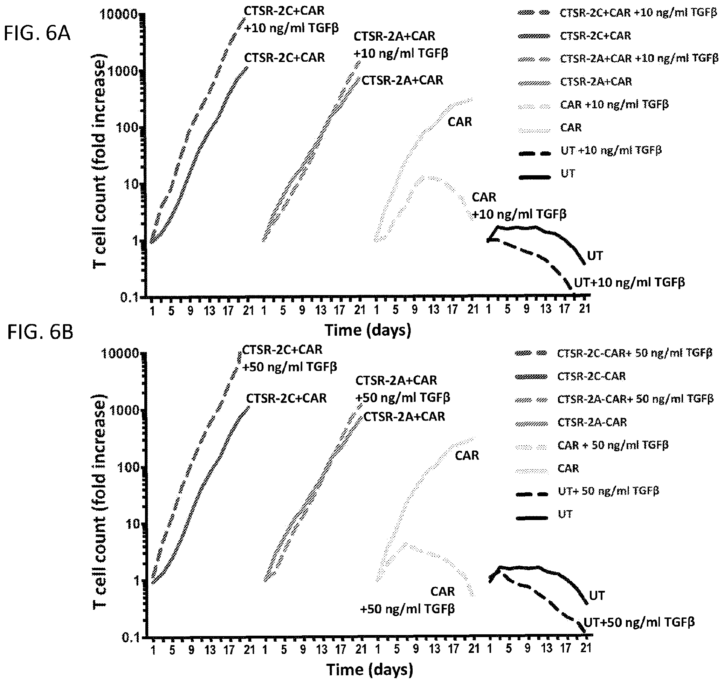

**FIG. 6A** and **FIG. 6B** show T cell count fold changes over time for transduced cells and untransduced controls in the presence of 10 and 50 ng/mL TGFβ, respectively. These data indicate that the presence of the exemplary chimeric TGFβ signaling receptors CTSR-2C containing two MyD88 domains in tandem improves T cell proliferation of CAR-expressing T cells in the presence of TGFβ. In contrast, in CAR-expressing T cells that do not co-express the chimeric TGFβ signaling receptor, T cell proliferation was substantially reduced in the presence of TGFβ.

### Example 4 Assessment of the Cytotoxic Effects of Chimeric TGFβ Signaling Receptors on Neuroblastoma In Vitro

The cytotoxic effects of human T cells expressing exemplary chimeric TGFβ signaling receptors and CARs on neuroblastoma tumor cells were assessed in vitro.

Human T cells were transduced with a vector encoding the exemplary chimeric TGFβ signaling receptors CTSR-2A or CTSR-2C and a vector encoding an exemplary CAR (SEQ ID NO: 45), or the exemplary CAR alone.

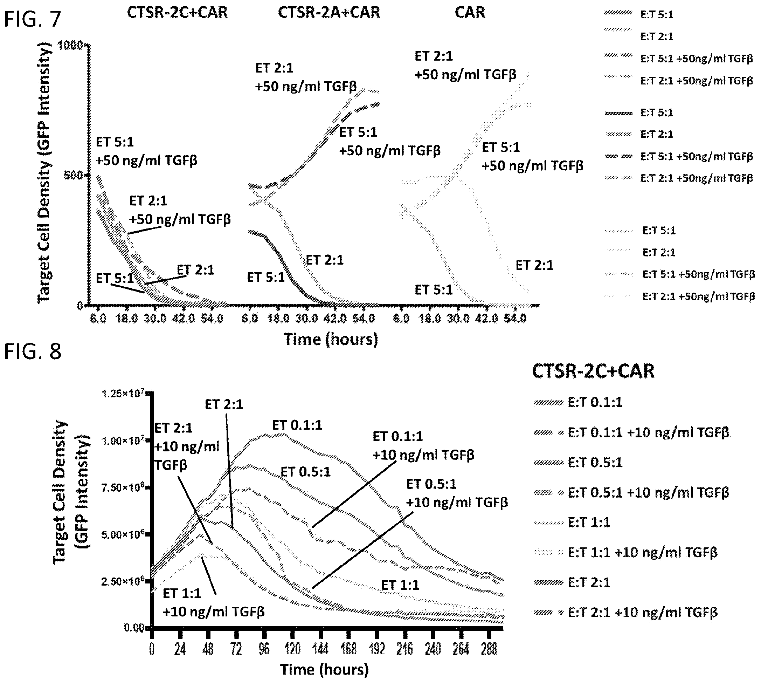

Transduced cells were cultured with neuroblastoma cancer cells expressing green fluorescent protein (GFP) at different effector to target ratios (E:T) in the presence or absence of TGFβ (50 ng/mL). Live cell imaging was used to assess GFP intensity over 54 hours in culturing conditions to monitor cell death. **FIG. 7** shows the kinetics of cell killing under the different culturing conditions. As shown, cytotoxic killing was robust in CAR-expressing T cells in the absence of TGFβ (solid lines), with greater cytotoxic activity generally observed at the higher E:T ratio of 5:1. In cells only expressing the CAR, cytotoxic killing by the T cells was substantially reduced in the presence of TGFβ (CAR condition, dashed lines). In contrast, in T cells co-expressing the CAR and the exemplary chimeric TGFβ signaling receptor CTSR-2C containing two MyD88 domains in tandem, cytotoxic activity in the presence of TGFβ (CTSR-2C+CAR, dashed lines) was substantially similar to that observed in the absence of TGFβ (CTSR-2C+CAR, solid lines). The improved activity of the chimeric TGFβ signaling receptor was not observed in CAR-T cells co-expressing the CTSR-2A chimeric TGFβ signaling receptor containing only a single MyD88 domain.

The cytotoxic effects of cells transduced with the exemplary chimeric TGFβ signaling receptor CTSR-2C and the CAR were also assessed at lower E:T ratios and lower concentrations of TGFβ (10 ng/mL) over 288 hours of culturing. As shown in **FIG. 8****,** improved killing was observed in the presence of TGFβ compared to the absence of TGFβ, with greatest improvements observed at the lower E:T ratios. These results are consistent with the finding that TGFβ induces a positive stimulating signal in the engineered T cells via the chimeric TGFβ signaling receptor containing two MyD88 domains in tandem.

### Example 5 Assessment of the Cytotoxic Effects of Chimeric TGFβ Signaling Receptors on Ovarian Carcinoma In Vitro

The cytotoxic effects of human T cells expressing exemplary chimeric TGFβ signaling receptors and CARs on ovarian tumor cells were assessed in vitro.

Human T cells were transduced with a vector encoding the exemplary chimeric TGFβ signaling receptor CTSR-2C and a vector encoding an exemplary B7H3-targeting CAR (SEQ ID NO: 45) or the exemplary CAR alone. Transduced cells were cultured with the ovarian carcinoma 622 cell line at different effector to target ratios (E:T) from 20:1 to 1: 10 in the presence or absence of TGFβ (10 ng/mL) for 8 days. Tumor cell count was determined and normalized to maximum cell count.

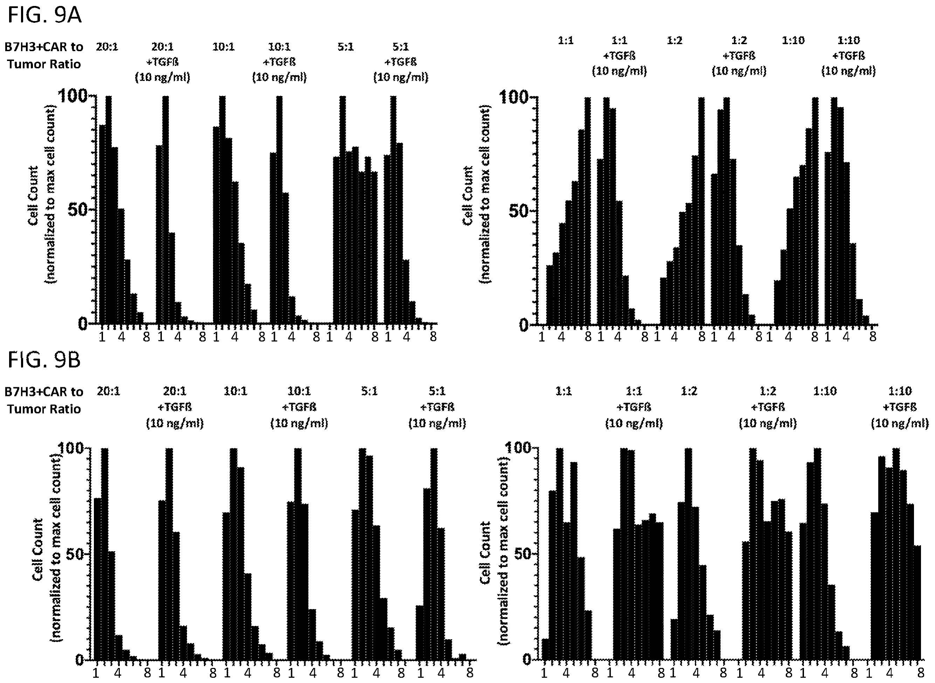

**FIG. 9A** (CTSR-2C + CAR) and **FIG.9B** (CAR only) shows the normalized ovarian tumor (SW 626) cell count at the different E:T ratios and TGFβ concentrations. In T cells co-transduced with the B7H3 CAR and the chimeric TGFβ signaling receptor containing two MyD88 domains in tandem, increased ovarian tumor cell killing was observed over time under all conditions, with a greater reduction in cell count being observed in cells stimulated with TGFβ supporting the positive stimulating effect of the chimeric TGFβ signaling receptor in modulating T cell activity (**FIG. 9A**). In contrast, in T cells only expressing the B7H3 CAR, greater tumor cell counts were observed in the presence of TGFβ, particularly at lower E:T ratios, consistent with the observation that TGFβ inhibits CAR-mediated cytotoxic killing (**FIG. 9B**). These data indicate that inclusion of the exemplary chimeric TGFβ signaling receptor containing two MyD88 domains in improves CAR-mediated cell killing activity, particularly in the presence of TGFβ.

### Example 6 Assessment of the Cytotoxic Effects of Chimeric TGFβ Signaling Receptors in an In Vivo Mouse Model of Neuroblastoma

To assess the effects of chimeric TGFβ signaling receptors in vivo, an orthotopic neuroblastoma mouse tumor model was used.

Immunodeficient NSG mice received an intrarenal injection of 1x10⁶ human neuroblastoma cells (cell line CHLA255). On day 14 post tumor inoculation, animals received an intravenous (i.v.) injection of 1 x 10⁷ untransduced T cells (UT), B7H3-targeted CAR-T cells (SEQ ID NO: 45), T cells transduced with a vector encoding the exemplary chimeric TGFβ signaling receptor CTSR-2C, or T cells transduced with separate vectors encoding CTSR-2C and the anti-B7H3 CAR (SEQ ID NO: 45). Bioluminescence imaging was used to monitor tumor growth in each treatment group.

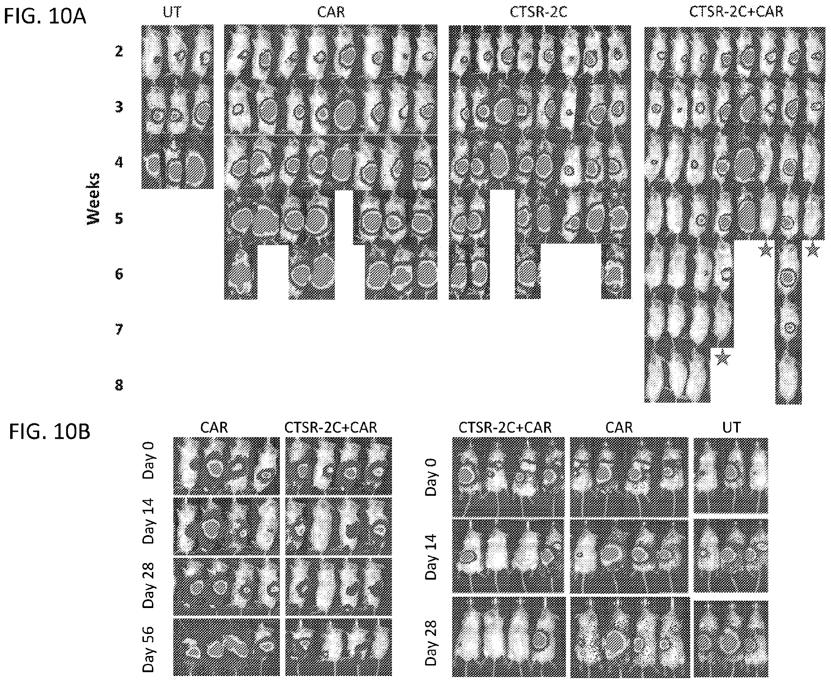

**FIG. 10A** shows bioluminescence images of neuroblastoma tumor growth over 8 weeks for each treatment group. These results demonstrated substantially reduced tumor growth and improved survival in animals treated with T cells expressing both the CAR and the chimeric TGFβ signaling receptor. Three animals in the co-transduced T cell group were euthanized due to presumed graft-vs-host disease (GVHD), which is a known phenomenon in NSG mice engrafted with xenogeneic human T cells capable of recognizing mouse HLA. Improved activity also was observed in mice administered engineered cells expressing only the chimeric TGFβ signaling receptor (without an engineered CAR).

**FIG. 10B** shows bioluminescence images of two pancreatic tumor models (using CFPAC and CF10.05 cell lines) growth over 8 weeks for each treatment group. These results demonstrated substantially reduced tumor growth and improved survival in animals treated with T cells expressing the CAR and the chimeric TGFβ signaling receptor.

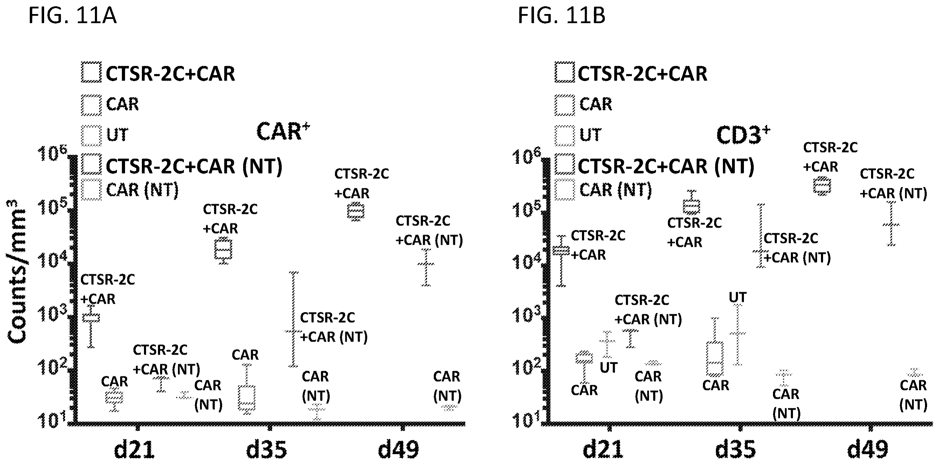

Median and boxplot summary data showing the presence of CAR+ and CD3 T cells in peripheral blood counts (counts/mm³ of blood) for tumor bearing mice and non-tumor (NT) bearing mice (saline injected) that received treatment as described above are shown in **FIG. 11A** and FIG. 11B. As can be seen in **FIG. 11A** and FIG. 11B. animals treated with T cells expressing both the CAR and the chimeric TGFβ signaling receptor had increased numbers of CAR+ and CD3+ T cells compared to mice administered T cells only (untransduced (UT)), T cells expressing the CAR alone, and treated non-tumor control (NT) groups at each of days 21 (d21), d35 or d49 post tumor inoculation.

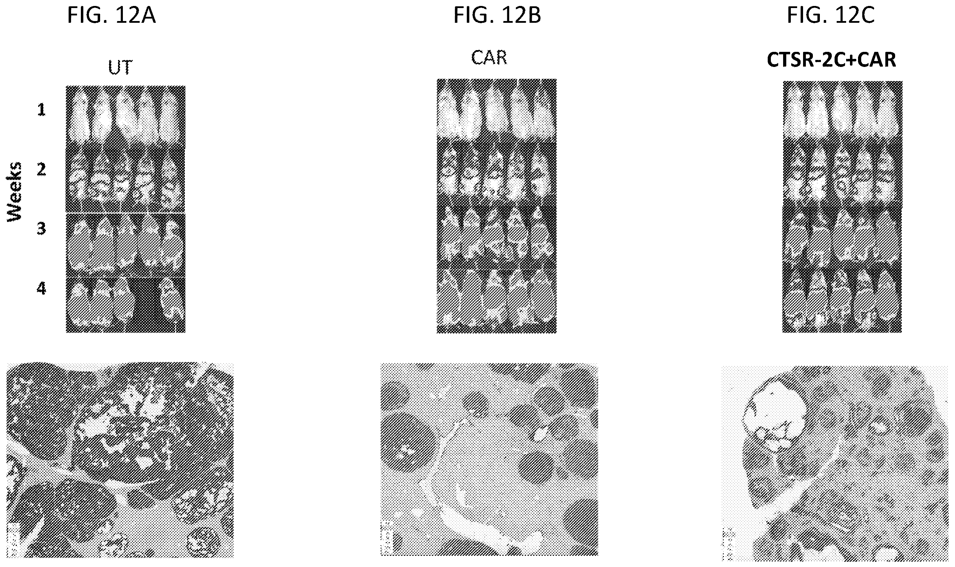

To assess cytotoxic activity under high tumor burden, the same neuroblastoma tumor model was used and mice were treated as above on day 28 post-inoculation. The top panels of **FIG. 12A-12C** show bioluminescence images of metastatic neuroblastoma tumor growth at different times post tumor inoculation, while the bottom panels of the figures provide representative images from immunohistochemical analysis of liver tissue obtained from mice 7 days post treatment injection after staining for neuroblastoma specific marker PHOX2b (brown stain) and human CD3 (red stain). Some reduction in the high tumor burden was observed in mice treated with T cells expressing both the CAR and the chimeric TGFβ signaling receptor (**FIG. 12C**) compared to mice treated with T cells only expressing the CAR (FIG. 12B). Further, histological analysis revealed a greater infiltration of T cells into liver tissue with metastatic neuroblastoma tumor cells in mice treated with T cells expressing both the CAR and the chimeric TGFβ signaling receptor compared to mice treated with T cells only expressing the CAR (compare **FIG. 12B** and **FIG. 12C**).

### Example 7 Generation of Bicistronic Polynucleotide Constructs Encoding Chimeric TGFβ Signaling Receptors and Chimeric Antigen Receptor

Polynucleotide constructs were generated to encode for both an exemplary chimeric TGFβ signaling receptor as described in Example 1 and an exemplary chimeric antigen receptor (CAR). A polynucleotide sequence encoding a T2A (SEQ ID NO: 61) cleavable peptide separated the nucleic acids encoding the receptors.

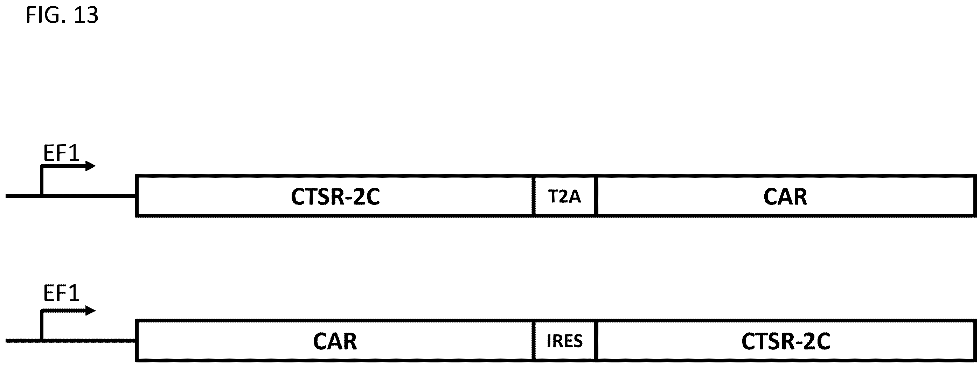

Exemplary constructs are shown in Table E3 and **FIG. 13****.** Exemplary control constructs are shown in Table E4.

| **Table E3:** Exemplary chimeric TGFβ signaling receptors and CARs encoded by single polynucleotide constructs | | | | |
|---|---|---|---|---|
| | **SEQ ID NO** | **First Receptor** | **Cleavable Peptide** | **Second Receptor** |
| **CTSR-2C-T2A-CAR-A** | 104 | TGFβ signaling receptor (CTSR-2C; SEQ ID NO: 30) | T2A (SEQ ID NO: 61) | **CAR** (SEQ ID NO: 45) |
| **CTSR-2C-T2A-CAR-B** | 105 | TGFβ signaling receptor (CTSR-2C; SEQ ID NO: 30) | T2A (SEQ ID NO: 61) | **CAR** (SEQ ID NO: 38) |
| **CTSR-2A-T2A-CAR-A** | 106 | TGFβ signaling receptor (CTSR-2A; SEQ ID NO: 41) | T2A (SEQ ID NO: 61) | **CAR** (SEQ ID NO: 45) |
| **CTSR-2A-T2A-CAR-B** | 107 | TGFβ signaling receptor (CTSR-2A; SEQ ID NO: 41) | T2A (SEQ ID NO: 61) | **CAR** (SEQ ID NO: 38) |

| **Table E4:** Exemplary Control TGFβ signaling receptors and CARs encoded by single polynucleotide constructs | | | | |
|---|---|---|---|---|
| | **SEQ ID NO** | **First Receptor** | **Cleavable Peptide** | **Second Receptor** |
| **CTSR-2IN-T2A-CAR-A** | 108 | Control TGFβ signaling receptor (CTSR-IN; SEQ ID NO: 32) | T2A (SEQ ID NO: 61) | CAR (SEQ ID NO: 45) |
| **CTSR-2IN-T2A-CAR-B** | 109 | Control TGFβ signaling receptor (CTSR-2IN; SEQ ID NO: 32) | T2A (SEQ ID NO: 61) | CAR (SEQ ID NO: 38) |
| **CTSR-2EN-T2A-CAR-A** | 110 | Control TGFβ signaling receptor (CTSR-2EN: SEQ ID NO: 36) | T2A (SEQ ID NO: 61) | CAR (SEQ ID NO: 45) |
| **CTSR-2EN-T2A-CAR-B** | 111 | Control TGFβ signaling receptor (CTSR-2EN; SEQ ID NO: 36) | T2A (SEQ ID NO: 61) | CAR (SEQ ID NO: 38) |

To assess cellular expression of the bicistronic polynucleotide constructs encoding both a TGFβ signaling receptor and a CAR, polynucleotides encoding exemplary TGFβ signaling receptors and exemplary CARs were cloned into third generation lentiviral vectors. The sequences encoding the bicistronic polypeptide chimeric TGFβ signaling receptor/CAR constructs were placed under the operable control of the human elongation factor 1 alpha (EF1α) promoter (sequence set forth in SEQ ID NO: 112).

Human T cells were transduced with vectors encoding the bicistronic construct CTSR-2A-T2A-CAR-B or CTSR-2C-T2A-CAR-B, or for comparison only the chimeric TGFβ signaling receptor (CTSR-2A or CTSR-2C), or only the CAR-B (anti-CD19 CAR; SEQ ID NO: 38). Cell expression was determined by flow cytometry. Protein L and myc-tag were used as surrogate markers of CAR and TGFβ signaling receptor expression, respectively.

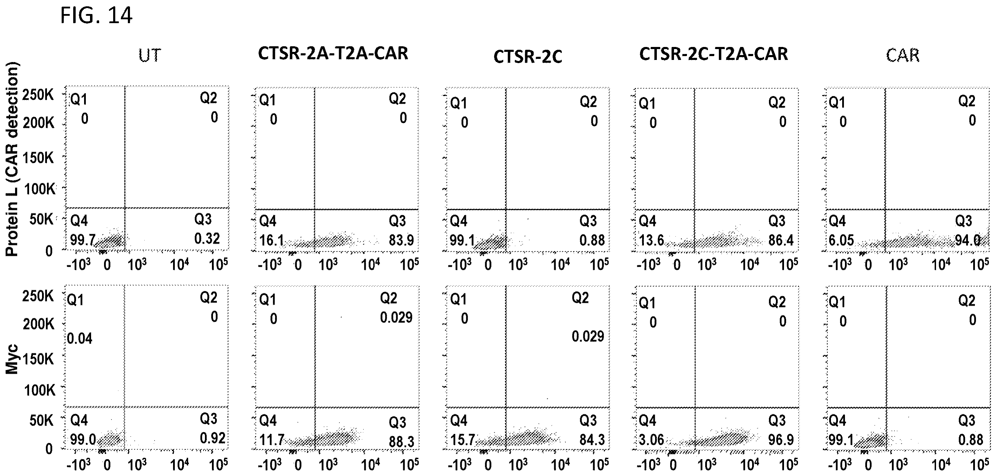

**FIG. 14** shows representative dot plots from flow cytometric analysis. These data demonstrate that the bicistronic constructs are able to be expressed in human T cells.

### Example 8 Assessment of Co-Expressing of Chimeric TGFβ Signaling Receptors and CARs on the TGFβ Signaling Pathway

Jurkat cells were transduced with bicistronic constructs described in Example 7, or control constructs encoding only the CAR (Ani-CD19 CAR; SEQ ID NO: 38) or only the chimeric TGFβ signaling receptor. Transduced cells were incubated with 0, 1, 5, or 10 ng/mL of TGFβ for 2 hours prior to protein extraction. Western blot analysis was used to determine the amount of phosphorylated SMAD 2 (pSMAD2) and phorphylated IRAK4 (pIRAK4).

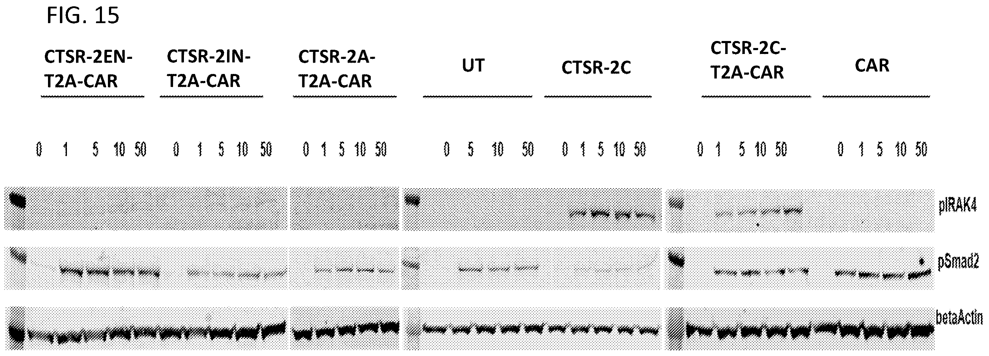

As shown in **FIG. 15****,** cells transduced with exemplary TGFβR signaling receptor containing MyD88 in tandem, either as a single encoding construct (CTSR-2C) or as a bicistronic construct also encoding a CAR (CTSR-2C-T2A-CAR-B) showed decreased levels of pSMAD2 and increased levels of pIRAK4 compared to cells transduced with CARs alone or untransduced cells (UT). These results indicate that the exemplary chimeric TGFβ signaling receptor containing two MyD88 domains in tandem is able to reduce signaling through the native TGFβR signaling pathway, even when co-expressed with CARs from a single bicistronic construct.

### Example 9 Assessment of the Cytotoxic Effects of Chimeric TGFβ Signaling Receptors Co-Expressed with CARs on CD19+ Tumor Cells In Vitro

The bicistronic constructs described in Example 7 were transduced into human T cells, and the cytotoxic effects on CD19+ tumor cells were assessed.

Human T cells were transduced with vectors encoding the bicistronic construct CTSR-2A-T2A-CAR-B or CTSR-2C-T2A-CAR-B, vectors encoding the exemplary chimeric TGFβ signaling receptor CTSR-2C, or vectors encoding only the CAR. Transduced cells were cultured with CD19+ tumor cells expressing GFP at different effector to target cell ratios (E:T) over a period of 21 hours. Tumor cell death was monitored by GFP intensity over time.

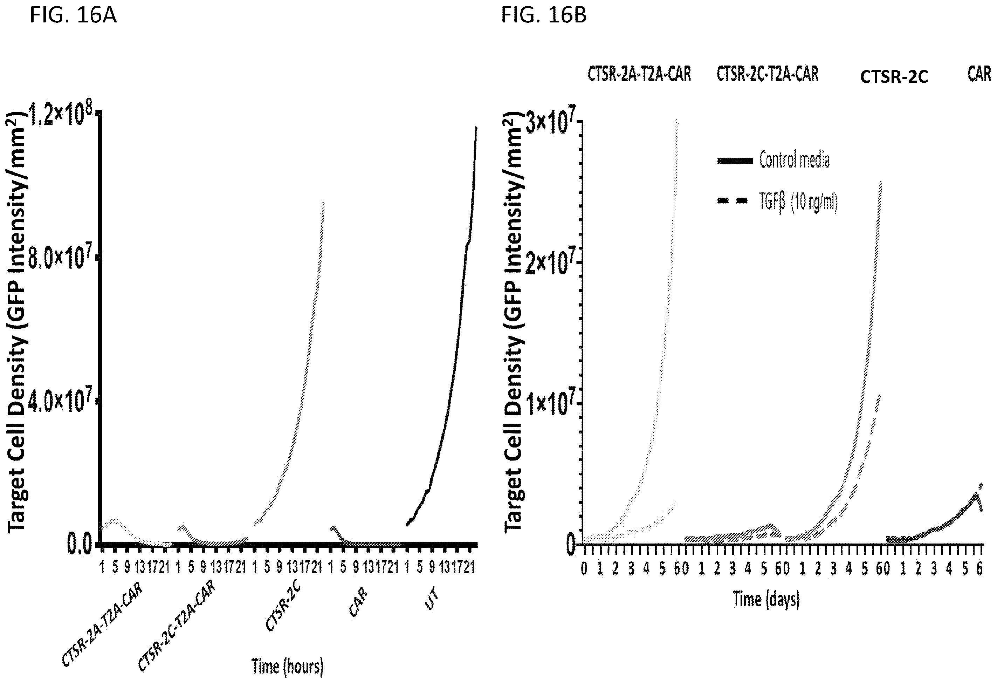

**FIG. 16A** shows the kinetics of cell killing under the different culturing conditions. These results indicate that cells expressing both the exemplary chimeric TGFβ signaling receptor and the CAR have similar killing activity to cells expressing the CAR alone.

The cytotoxic effects of the transduced cells were also assessed in the presence of TGFβ at 10 ng/mL. As shown in **FIG. 16B****,** reduced cytotoxic activity was observed in the presence of TGFβ in cells not expressing the CAR (CTSR-2C) or in cells expressing a chimeric TGFβ signaling receptor containing only a single MyD88 domain, consistent with results above (compare solid lines and dashed lines). T cells in which the chimeric TGFβ signaling receptor containing two MyD88 domains in tandem was expressed as a bicistronic construct with the anti-CD19 CAR showed robust killing even in the presence of TGFβ, which was greater than that observed in T cells only expressing the CAR and may be improved compared to killing in the absence of TGFβ.

### Example 10 Assessment of the Cytotoxic Effects of Chimeric TGFβ Signaling Receptors Co-Expressed with CARs on Metastatic Lymphoma In Vivo

To assess the anti-tumor activity of T cells expressing the chimeric TGFβ signaling receptor as a bicistronic construct with an anti-CD19 CAR in vivo, a mouse model of metastatic lymphoma was used.

Immunodeficient NSG mice received a tail vein injection of 1x10⁶ Raji cells. On day 3 post tumor inoculation, animals received an intravenous (i.v.) injection of 1 x 10⁷ untransduced T cells (UT), anti-CD19 CAR-T cells, T cells transduced vectors encoding CTSR-2A-T2A-CAR-B or CTSR-2C-T2A-CAR-B, or T cells transduced with a vector encoding the exemplary TGFβ signaling receptor CTSR-2C. Bioluminescence imaging was used to monitor tumor growth in each treatment group.

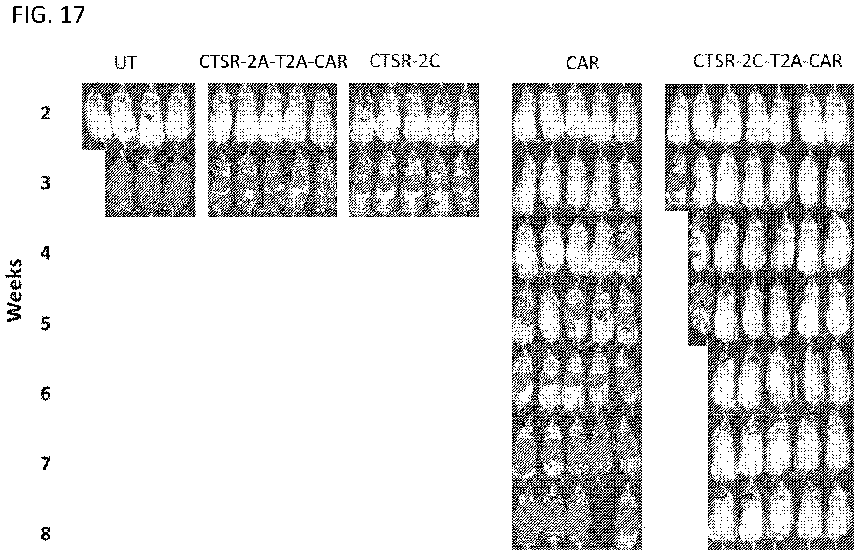

**FIG. 17** shows bioluminescence images of tumor growth over 8 weeks for each treatment group. These results demonstrate reduced tumor growth and improved survival in animals treated with T cells transduced with the exemplary chimeric TGFβ signaling receptor containing two MyD88 domains expressed from a bicistronic construct with the exemplary anti-CD19 CAR.

### Example 11 Assessment of the Exhaustion Markers, Expansion, and Cytotoxic Effects of Exemplary Chimeric TGFβ Signaling Receptors Co-Expressed with CARs Compared with other Chimeric TGFβ Signaling Constructs

The exhaustion profile, expansion ability, and cytotoxic effects of human T cells expressing exemplary chimeric TGFβ signaling receptors and CARs along with T cells transduced with CAR and other chimeric TGFβ signaling constructs (TGFβR2-DNR, NULL-MYD88, TGFβR2-41bb, TGFβR2-DAP12) was assessed in vitro.

Human T cells were transduced with a vector encoding the exemplary chimeric TGFβ signaling receptors CTSR-2A, CTSR-2C, TGFβR2-DNR, NULL-MYD88, TGFβR2-41bb, or TGFβR2-DAP12, and a vector encoding an exemplary CAR (anti-B7H3 CAR; SEQ ID NO: 45), or a vector encoding the exemplary CAR alone.

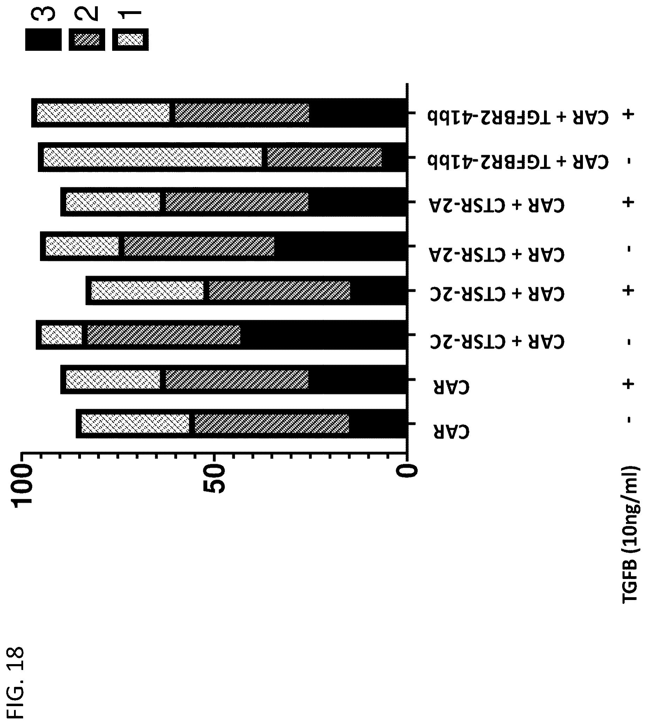

Transduced cells were cultured with neuroblastoma cancer cells expressing green fluorescent protein (GFP) at 1:1 target ratios (E:T) in the presence or absence of TGFβ (10 ng/mL). Flow cytometry was used to assess the frequency of cells with expression of PD1, Tim3, and LAG3 exhaustion markers after 72 hours of culture with tumor cells. **FIG. 18** shows the percentage of cells expressing 1, 2, or all 3 of the exhaustion markers in the presence or absences of TGFβ. As shown, the percentage of cells with 1, 2, or 3 exhaustion markers decreased only in the T cells co-expressing the CAR and the exemplary chimeric TGFβ signaling receptor CTSR-2C containing two MyD88 domains in tandem. The decrease in number of cells with 1, 2, or 3 exhaustion markers was also observed in CAR-T cells co-expressing the CTSR-2A chimeric TGFβ signaling receptor containing only a single MyD88 domain but to a lower extent. In contrast, cells expressing CAR alone or CAR with TGFβR2-41bb vector had a higher percentage of T cells with 3 exhaustion markers in the presence of TGFβ.

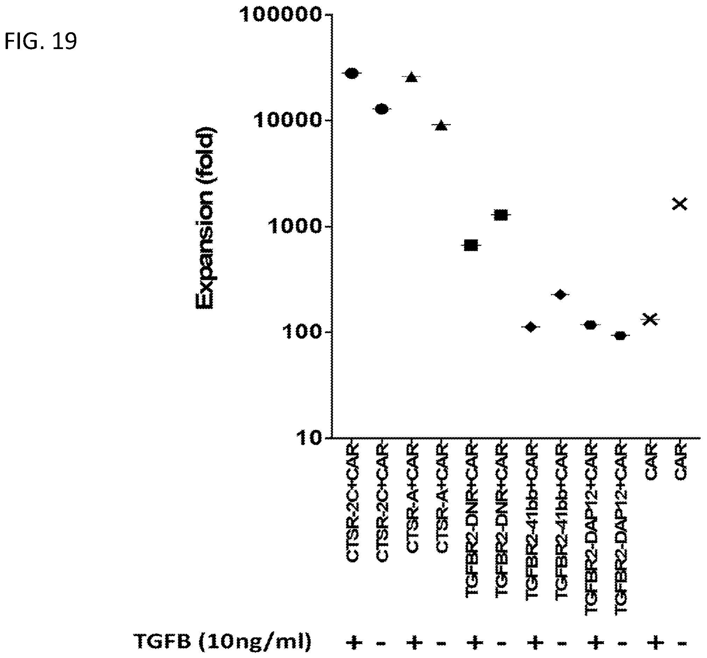

The proliferation ability of the exemplary chimeric TGFβ signaling receptors CTSR-2C and CTSR-2A compared to other chimeric TGFβR signaling constructs was evaluated over a 4-week period in presence of tumor cells for the first week followed by daily IL2 treatment and with or without TGFβ (10 ng/mL). **FIG. 19** shows T cell count fold changes over time was significantly higher for T cells co-expressing the CAR and the exemplary chimeric TGFβ signaling receptors CTSR-2C and CTSR-2A compared to T cells co-expressing the CAR and the chimeric TGFβ signaling receptors TGFβR2-DNR, TGFβR2-41bb, TGFβR2-DAP12, or cells expressing CAR alone.

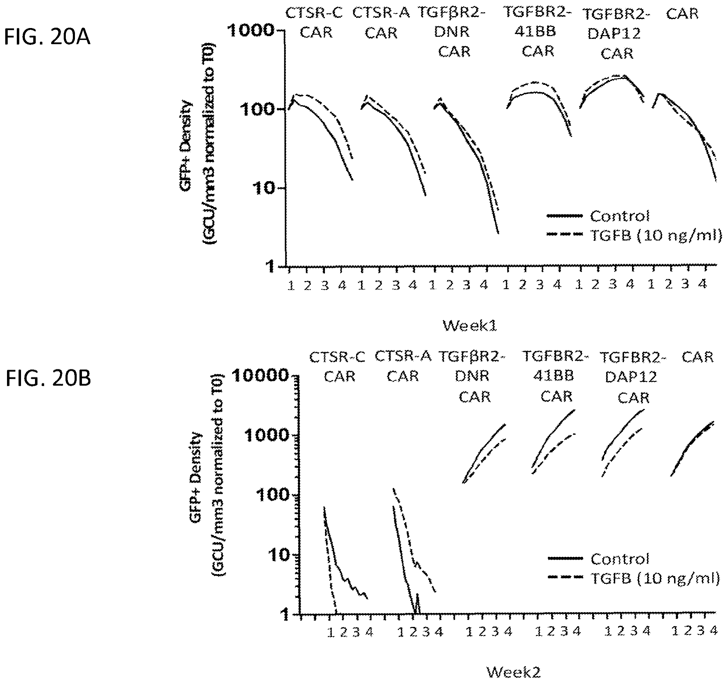

The cytotoxic effects of cells transduced with the CAR and the chimeric TGFβ signaling receptors were compared by first co-culturing the T cells with tumor cells at 1:1 target ratio (E:T) for one week in the presence or absence of TGFβ (10 ng/mL) , then retrieving the T cells from the culture and re-culturing them for additional week with fresh tumor cells at 1:1 target ratio (E:T) in the presence or absence of TGFβ (10 ng/mL). As shown in **FIG. 20A****,** all the CAR T cells with indicated chimeric TGFβ signaling demonstrated cytotoxicity to some level against the tumor cells. However, as shown in **FIG. 20B** only T cells transduced with CAR and the and the exemplary chimeric TGFβ signaling receptors CTSR-2C and CTSR-2A retained their cytotoxic effect with the T cells transduced with CTSR-2C showing improved killing in presence of TGFβ.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

### SEQUENCES

| # | SEQUENCE | ANNOTATION |
|---|---|---|
| 1 | LQCFCHLCTKDNFTCVTDGLCFVSVTETTDKVIHNSMCIAEIDLIPRDRP FVCAPSSKTGSVTTTYCCNQDHCNKIELPTTVKSSPGLGPVELAAVIAG PVCFVCISLMLMVYICHNRTVIHHRVPNEEDPSLDRPFISEGTTLKDLIY D | TGFBR1, amino acids 34-183 of UniProt P36897 (ectodomain, amino acids 34-126; transmembrane domain, amino acids 127-147); portion of cytoplasmic domain, amino acids 148-183) |
| 2 | AAGGPGAGSAAPVSSTSSLPLAALNMRVRRRLSLFLNVRTQVAADWT ALAEEMDFEYLEIRQLETQADPTGRLLDAWQGRPGASVGRLLELLTKL GRDDVLLELGPSIEEDCQKYILKQQQEEAEKPLQVAAVDSSVPRTAELA GITTLDDPLGHMPERFDAFICYCPSD | MYD88, amino acids 2-171 of UniProt Q99836 |
| 3 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQ VTGISLLPPLGVAISVIIIFY CYRVNRQQKLS | TGFBR2 |
| 4 | LQCFCHLCTKDNFTCVTDGLCFVSVTETTDKVIHNSMCIAEIDLIPRDRP FVCAPSSKTGSVTTTYCCNQDHCNKIELPTTVKSSPGLGPVEL | TGFBR1 Extracellular AA |
| 5 | CTGCAATGTTTTGTCACCTCTGTACGAAAGATAATTTTACGTGTGT CACTGATGGCCTTTGTTTTGTCTCAGTCACTGAAACTACGGATAAAG TAATACATAATAGCATGTGTATTGCAGAGATTGATCTCATCCCGAG AGACCGGCCTTTCGTCTGTGCGCCTAGCTCCAAAACAGGATCAGTT ACCACCACGTATTGCTGTAACCAAGACCACTGCAACAAAATCGAGC TTCCAACAACTGTAAAGTCTTCCCCTGGCCTTGGCCCTGTAGAACTG | TGFBR1 Extracellular NA |
| 6 | AAVIAGPVCFVCISLMLMVYI | TGFBR1 |
| | | Transmembrane AA |
| 7 | GCTGCGGTAATTGCTGGGCCCGTATGCTTTGTTTGTATCTCCCTCAT GCTCATGGTCTACATC | TGFBR1 Transmembrane NA |
| 8 | CHNRTVIHHRVPNEEDPSLDRPFISEGTTLKDLIYD | TGFBR1 Intracellular AA |
| 9 | TGTCATAACCGCACGGTCATACACCATCGCGTGCCTAATGAAGAGG ATCCATCACTCGATCGGCCTTTTATTTCTGAGGGCACTACTCTTAAA GACTTGATCTATGAC | TGFBR1 Intracellular NA |
| 10 | AAGGPGAGSAAPVSSTSSLPLAALNMRVRRRLSLFLNVRTQVAADWT ALAEEMDFEYLEIRQLETQADPTGRLLDAWQGRPGASVGRLLELLTKL GRDDVLLELGPSI | MYD88 DD AA |
| 11 | GCGGCTGGCGGACCAGGGGCAGGTTCAGCTGCTCCAGTAAGTAGCA CCTCCTCATTGCCTTTGGCTGCATTGAATATGCGGGTACGGAGAAGA CTTAGTCTCTTCTTGAACGTAAGAACTCAAGTCGCCGCAGATTGGAC GGCTCTTGCCGAAGAAATGGACTTTGAATATCTCGAGATTCGACAA CTCGAAACCCAAGCGGATCCAACTGGTCGGTTGCTTGACGCTTGGC AGGGTCGCCCTGGAGCGTCTGTCGGCCGATTGCTTGAATTGTTGACA AAACTTGGCAGAGATGACGTGCTCCTGGAACTCGGCCCCTCCATC | MYD88 DD NA |
| 12 | EEDCQKYILKQQQEEAEKPLQVAAVDSSVPRTAELAGITTLDDPLGHM P | MYD88 ID AA |
| 13 | GAAGAGGACTGTCAGAAATACATTCTGAAACAGCAACAAGAGGAA GCGGAAAAGCCCCTGCAGGTCGCTGCAGTTGATTCTAGCGTTCCTC GGACCGCGGAACTTGCAGGCATAACCACTCTCGACGATCCCTTGGG GCACATGCCT | MYD88 ID NA |
| 14 | ERFDAFICYCPSD | MYD88 TIR AA |
| 15 | GAACGCTTTGACGCCTTCATTTGTTATTGCCCAAGCGAC | MYD88 TIR NA |
| 16 | MALPVTALLLPLALLLHAARPHHHHHHLQCFCHLCTKDNFTCVTDGL CFVSVTETTDKVIHNSMCIAEIDLIPRDRPFVCAPSSKTGSVTTTYCCNQ DHCNKIELPTTVKSSPGLGPVELAAVIAGPVCFVCISLMLMVYICHNRT VIHHRVPNEEDPSLDRPFISEGTTLKDLIYDAAGGPGAGSAAPVSSTSSL PLAALNMRVRRRLSLFLNVRTQVAADWTALAEEMDFEYLEIRQLETQ ADPTGRLLDAWQGRPGASVGRLLELLTKLGRDDVLLELGPSIEEDCQK | CTSR-1A |
| | YILKQQQEEAEKPLQVAAVDSSVPRTAELAGITTLDDPLGHMPERFDAF ICYCPSD* | |
| 17 | ATGGCACTTCCCGTTACAGCACTGCTCCTGCCCCTGGCGCTGCTCCT GCACGCGGCCCGCCCcatcatcaccatcaccacCTGCAATGTTTTGTCACCT CTGTACGAAAGATAATTTACGTGTGTCACTGATGGCCTTTGTTTTG TCTCAGTCACTGAAACTACGGATAAAGTAATACATAATAGCATGTG TATTGCAGAGATTGATCTCATCCCGAGAGACCGGCCTTTCGTCTGTG CGCCTAGCTCCAAAACAGGATCAGTTACCACCACGTATTGCTGTAA CCAAGACCACTGCAACAAAATCGAGCTTCCAACAACTGTAAAGTCT TCCCCTGGCCTTGGCCCTGTAGAACTGGCTGCGGTAATTGCTGGGCC CGTATGCTTTGTTTGTATCTCCCTCATGCTCATGGTCTACATCTGTCA TAACCGCACGGTCATACACCATCGCGTGCCTAATGAAGAGGATCCA TCACTCGATCGGCCTTTTATTTCTGAGGGCACTACTCTTAAAGACTT GATCTATGACGCGGCTGGCGGACCAGGGGCAGGTTCAGCTGCTCCA GTAAGTAGCACCTCCTCATTGCCTTTGGCTGCATTGAATATGCGGGT ACGGAGAAGACTTAGTCTCTTCTTGAACGTAAGAACTCAAGTCGCC GCAGATTGGACGGCTCTTGCCGAAGAAATGGACTTTGAATATCTCG AGATTCGACAACTCGAAACCCAAGCGGATCCAACTGGTCGGTTGCT TGACGCTTGGCAGGGTCGCCCTGGAGCGTCTGTCGGCCGATTGCTTG AATTGTTGACAAAACTTGGCAGAGATGACGTGCTCCTGGAACTCGG CCCCTCCATCGAAGAGGACTGTCAGAAATACATTCTGAAACAGCAA CAAGAGGAAGCGGAAAAGCCCCTGCAGGTCGCTGCAGTTGATTCTA GCGTTCCTCGGACCGCGGAACTTGCAGGCATAACCACTCTCGACGA TCCCTTGGGGCACATGCCTGAACGCTTTGACGCCTTCATTTGTTATT GCCCAAGCGACtag | CTSR-1A |
| 18 | MALPVTALLLPLALLLHAARPHHHHHHLQCFCHLCTKDNFTCVTDGL CFVSVTETTDKVIHNSMCIAEIDLIPRDRPFVCAPSSKTGSVTTTYCCNQ DHCNKIELPTTVKSSPGLGPVELAAVIAGPVCFVCISLMLMVYIGGGGS GGGGSAAGGPGAGSAAPVSSTSSLPLAALNMRVRRRLSLFLNVRTQVA ADWTALAEEMDFEYLEIRQLETQADPTGRLLDAWQGRPGASVGRLLE LLTKLGRDDVLLELGPSIEEDCQKYILKQQQEEAEKPLQVAAVDSSVPR TAELAGITTLDDPLGHMPERFDAFICYCPSD | CTSR-1B |
| 19 | ATGGCACTTCCCGTTACAGCACTGCTCCTGCCCCTGGCGCTGCTCCT GCACGCGGCCCGCCCCcatcatcaccatcaccacCTGCAATGTTTTGTCACCT | CTSR-1B |
| | CTGTACGAAAGATAATTTTACGTGTGTCACTGATGGCCTTTGTTTTG TCTCAGTCACTGAAACTACGGATAAAGTAATACATAATAGCATGTG TATTGCAGAGATTGATCTCATCCCGAGAGACCGGCCTTTCGTCTGTG CGCCTAGCTCCAAAACAGGATCAGTTACCACCACGTATTGCTGTAA CCAAGACCACTGCAACAAAATCGAGCTTCCAACAACTGTAAAGTCT TCCCCTGGCCTTGGCCCTGTAGAACTGGCTGCGGTAATTGCTGGGCC CGTATGCTTTGTTTGTATCTCCCTCATGCTCATGGTCTACATCGGCG GCGGTGGTAGCGGTGGCGGTGGCAGCGCGGCTGGCGGACCAGGGG CAGGTTCAGCTGCTCCAGTAAGTAGCACCTCCTCATTGCCTTTGGCT GCATTGAATATGCGGGTACGGAGAAGACTTAGTCTCTTCTTGAACG TAAGAACTCAAGTCGCCGCAGATTGGACGGCTCTTGCCGAAGAAAT GGACTTTGAATATCTCGAGATTCGACAACTCGAAACCCAAGCGGAT CCAACTGGTCGGTTGCTTGACGCTTGGCAGGGTCGCCCTGGAGCGT CTGTCGGCCGATTGCTTGAATTGTTGACAAAACTTGGCAGAGATGA CGTGCTCCTGGAACTCGGCCCCTCCATCGAAGAGGACTGTCAGAAA TACATTCTGAAACAGCAACAAGAGGAAGCGGAAAAGCCCCTGCAG GTCGCTGCAGTTGATTCTAGCGTTCCTCGGACCGCGGAACTTGCAGG CATAACCACTCTCGACGATCCCTTGGGGCACATGCCTGAACGCTTTG ACGCCTTCATTTGTTATTGCCCAAGCGACtag | |
| 20 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQ | TGFβR2 Extracellular domain AA |
| 21 | ACTATTCCCCCTCATGTTCAGAAATCCGTAAACAATGATATGATTGT CACGGACAACAACGGTGCGGTCAAATTCCCGCAGCTTTGCAAATTT TGTGATGTTAGATTCTCAACGTGTGATAATCAAAAGTCCTGCATGAG TAATTGTTCAATTACTTCCATCTGCGAAAAGCCTCAGGAAGTTTGCG TCGCAGTCTGGAGGAAGAACGACGAAAACATTACACTTGAGACTGT GTGTCATGATCCTAAACTCCCCTATCATGACTTCATCCTTGAGGATG CGGCGTCCCCCAAATGTATCATGAAAGAGAAAAAAAAGCCCGGTG AGACGTTCTTCATGTGCAGCTGCTCCAGCGACGAGTGCAATGATAA CATCATTTTCTCTGAAGAATACAATACCTCAAATCCCGACCTCCTCT TGGTAATATTTCAG | TGFβR2 Extracellular domain NA |
| 22 | VTGISLLPPLGVAISVIIIFY | TGFβR2 Transmembrane AA |
| 23 | GTGACGGGTATAAGTTTGCTTCCGCCTCTCGGTGTGGCGATCTCCGT TATCATTATCTTTTAT | TGFβR2 Transmembrane NA |
| 24 | CYRVNRQQKLS | TGFβR2 Cytoplasmic domain AA 1-11 |
| 25 | TGCTATCGCGTGAACAGACAACAAAAATTGTCC | TGFβR2 Cytoplasmic domain NA |
| 26 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSD | CTSR-2A |
| 27 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT | CTSR-2A |
| | GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGCGGACCAGGGGCAGGTTCAGCTGCTCCAGTAAGTA GCACCTCCTCATTGCCTTTGGCTGCATTGAATATGCGGGTACGGAGA AGACTTAGTCTCTTCTTGAACGTAAGAACTCAAGTCGCCGCAGATTG GACGGCTCTTGCCGAAGAAATGGACTTTGAATATCTCGAGATTCGA CAACTCGAAACCCAAGCGGATCCAACTGGTCGGTTGCTTGACGCTT GGCAGGGTCGCCCTGGAGCGTCTGTCGGCCGATTGCTTGAATTGTTG ACAAAACTTGGCAGAGATGACGTGCTCCTGGAACTCGGCCCCTCCA TCGAAGAGGACTGTCAGAAATACATTCTGAAACAGCAACAAGAGG AAGCGGAAAAGCCCCTGCAGGTCGCTGCAGTTGATTCTAGCGTTCC TCGGACCGCGGAACTTGCAGGCATAACCACTCTCGACGATCCCTTG GGGCACATGCCTGAACGCTTTGACGCCTTCATTTGTTATTGCCCAAG CGACtag | |
| 28 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDAAGGPGAGSAAPVSS TSSLPLAALNMRVRRRLSLFLNVRTQVAADWTALAEEMDFEYLEIRQL ETQADPTGRLLDAWQGRPGASVGRLLELLTKLGRDDVLLELGPSIEED CQKYILKQQQEEAEKPLQVAAVDSSVPRTAELAGITTLDDPLGHMPER FDAFICYCPSD | CTSR-2B |
| 29 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC | CTSR-2B |
| | CTAAACTCCCCTATCATGACTTCATCCTTGAGGATGCGGCGTCCCCC AAATGTATCATGAAAGAGAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGCGGACCAGGGGCAGGTTCAGCTGCTCCAGTAAGTA GCACCTCCTCATTGCCTTTGGCTGCATTGAATATGCGGGTACGGAGA AGACTTAGTCTCTTCTTGAACGTAAGAACTCAAGTCGCCGCAGATTG GACGGCTCTTGCCGAAGAAATGGACTTTGAATATCTCGAGATTCGA CAACTCGAAACCCAAGCGGATCCAACTGGTCGGTTGCTTGACGCTT GGCAGGGTCGCCCTGGAGCGTCTGTCGGCCGATTGCTTGAATTGTTG ACAAAACTTGGCAGAGATGACGTGCTCCTGGAACTCGGCCCCTCCA TCGAAGAGGACTGTCAGAAATACATTCTGAAACAGCAACAAGAGG AAGCGGAAAAGCCCCTGCAGGTCGCTGCAGTTGATTCTAGCGTTCC TCGGACCGCGGAACTTGCAGGCATAACCACTCTCGACGATCCCTTG GGGCACATGCCTGAACGCTTTGACGCCTTCATTTGTTATTGCCCAAG CGACGCGGCTGGCGGACCAGGGGCAGGTTCAGCTGCTCCAGTAAGT AGCACCTCCTCATTGCCTTTGGCTGCATTGAATATGCGGGTACGGAG AAGACTTAGTCTCTTCTTGAACGTAAGAACTCAAGTCGCCGCAGATT GGACGGCTCTTGCCGAAGAAATGGACTTTGAATATCTCGAGATTCG ACAACTCGAAACCCAAGCGGATCCAACTGGTCGGTTGCTTGACGCT TGGCAGGGTCGCCCTGGAGCGTCTGTCGGCCGATTGCTTGAATTGTT GACAAAACTTGGCAGAGATGACGTGCTCCTGGAACTCGGCCCCTCC ATCGAAGAGGACTGTCAGAAATACATTCTGAAACAGCAACAAGAG GAAGCGGAAAAGCCCCTGCAGGTCGCTGCAGTTGATTCTAGCGTTC CTCGGACCGCGGAACTTGCAGGCATAACCACTCTCGACGATCCCTT GGGGCACATGCCTGAACGCTTTGACGCCTTCATTTGTTATTGCCCAA GCGACtag | |
| 30 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT | CTSR-2C |
| | QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGGGGSAAGGPGAGS AAPVSSTSSLPLAALNMRVRRRLSLFLNVRTQVAADWTALAEEMDFE YLEIRQLETQADPTGRLLDAWQGRPGASVGRLLELLTKLGRDDVLLEL GPSIEEDCQKYILKQQQEEAEKPLQVAAVDSSVPRTAELAGITTLDDPL GHMPERFDAFICYCPSD | |
| 31 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGCGGACCAGGGGCAGGTTCAGCTGCTCCAGTAAGTA GCACCTCCTCATTGCCTTTGGCTGCATTGAATATGCGGGTACGGAGA AGACTTAGTCTCTTCTTGAACGTAAGAACTCAAGTCGCCGCAGATTG GACGGCTCTTGCCGAAGAAATGGACTTTGAATATCTCGAGATTCGA CAACTCGAAACCCAAGCGGATCCAACTGGTCGGTTGCTTGACGCTT GGCAGGGTCGCCCTGGAGCGTCTGTCGGCCGATTGCTTGAATTGTTG ACAAAACTTGGCAGAGATGACGTGCTCCTGGAACTCGGCCCCTCCA TCGAAGAGGACTGTCAGAAATACATTCTGAAACAGCAACAAGAGG AAGCGGAAAAGCCCCTGCAGGTCGCTGCAGTTGATTCTAGCGTTCC TCGGACCGCGGAACTTGCAGGCATAACCACTCTCGACGATCCCTTG GGGCACATGCCTGAACGCTTTGACGCCTTCATTTGTTATTGCCCAAG CGACGGTGGCGGTGGCTCGGCGGCTGGCGGACCAGGGGCAGGTTCA GCTGCTCCAGTAAGTAGCACCTCCTCATTGCCTTTGGCTGCATTGAA TATGCGGGTACGGAGAAGACTTAGTCTCTTCTTGAACGTAAGAACT | CTSR-2C |
| | CAAGTCGCCGCAGATTGGACGGCTCTTGCCGAAGAAATGGACTTTG AATATCTCGAGATTCGACAACTCGAAACCCAAGCGGATCCAACTGG TCGGTTGCTTGACGCTTGGCAGGGTCGCCCTGGAGCGTCTGTCGGCC GATTGCTTGAATTGTTGACAAAACTTGGCAGAGATGACGTGCTCCT GGAACTCGGCCCCTCCATCGAAGAGGACTGTCAGAAATACATTCTG AAACAGCAACAAGAGGAAGCGGAAAAGCCCCTGCAGGTCGCTGCA GTTGATTCTAGCGTTCCTCGGACCGCGGAACTTGCAGGCATAACCA CTCTCGACGATCCCTTGGGGCACATGCCTGAACGCTTTGACGCCTTC ATTTGTTATTGCCCAAGCGACtag | |
| 32 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR V | CTSR-2IN |
| 33 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGtag | CTSR-2IN |
| 34 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGSGEGRGSLLTCGDVEENPGP | CTSR-2IN-T2A |
| 35 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT | CTSR-2IN-T2A |
| | CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGATCTGGTGAGGGGCGGGGGTCCCTGCTGACCTGCG GCGACGTCGAAGAAAATCCCGGTCCC | |
| 36 | MALPVTALLLPLALLLHAARPEQKLISEEDLTIPPHVQKSVNNDVTGISL LPPLGVAISVIIIFYCYRVNRQQKLSAAGGPGAGSAAPVSSTSSLPLAAL NMRVRRRLSLFLNVRTQVAADWTALAEEMDFEYLEIRQLETQADPTG RLLDAWQGRPGASVGRLLELLTKLGRDDVLLELGPSIEEDCQKYILKQ QQEEAEKPLQVAAVDSSVPRTAELAGITTLDDPLGHMPERFDAFICYCP SDGGGGSAAGGPGAGSAAPVSSTSSLPLAALNMRVRRRLSLFLNVRTQ VAADWTALAEEMDFEYLEIRQLETQADPTGRLLDAWQGRPGASVGRL LELLTKLGRDDVLLELGPSIEEDCQKYILKQQQEEAEKPLQVAAVDSSV PRTAELAGITTLDDPLGHMPERFDAFICYCPSD | CTSR-2EN |
| 37 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATGTGACGGGTATAAGTTTGC TTCCGCCTCTCGGTGTGGCGATCTCCGTTATCATTATCTTTATTGCT ATCGCGTGAACAGACAACAAAAATTGTCCGCGGCTGGCGGACCAGG GGCAGGTTCAGCTGCTCCAGTAAGTAGCACCTCCTCATTGCCTTTGG CTGCATTGAATATGCGGGTACGGAGAAGACTTAGTCTCTTCTTGAAC GTAAGAACTCAAGTCGCCGCAGATTGGACGGCTCTTGCCGAAGAAA TGGACTTTGAATATCTCGAGATTCGACAACTCGAAACCCAAGCGGA TCCAACTGGTCGGTTGCTTGACGCTTGGCAGGGTCGCCCTGGAGCGT CTGTCGGCCGATTGCTTGAATTGTTGACAAAACTTGGCAGAGATGA CGTGCTCCTGGAACTCGGCCCCTCCATCGAAGAGGACTGTCAGAAA | CTSR-2EN |
| | TACATTCTGAAACAGCAACAAGAGGAAGCGGAAAAGCCCCTGCAG GTCGCTGCAGTTGATTCTAGCGTTCCTCGGACCGCGGAACTTGCAGG CATAACCACTCTCGACGATCCCTTGGGGCACATGCCTGAACGCTTTG ACGCCTTCATTTGTTATTGCCCAAGCGACGGTGGCGGTGGCTCGGCG GCTGGCGGACCAGGGGCAGGTTCAGCTGCTCCAGTAAGTAGCACCT CCTCATTGCCTTTGGCTGCATTGAATATGCGGGTACGGAGAAGACTT AGTCTCTTCTTGAACGTAAGAACTCAAGTCGCCGCAGATTGGACGG CTCTTGCCGAAGAAATGGACTTTGAATATCTCGAGATTCGACAACTC GAAACCCAAGCGGATCCAACTGGTCGGTTGCTTGACGCTTGGCAGG GTCGCCCTGGAGCGTCTGTCGGCCGATTGCTTGAATTGTTGACAAAA CTTGGCAGAGATGACGTGCTCCTGGAACTCGGCCCCTCCATCGAAG AGGACTGTCAGAAATACATTCTGAAACAGCAACAAGAGGAAGCGG AAAAGCCCCTGCAGGTCGCTGCAGTTGATTCTAGCGTTCCTCGGACC GCGGAACTTGCAGGCATAACCACTCTCGACGATCCCTTGGGGCACA TGCCTGAACGCTTTGACGCCTTCATTTGTTATTGCCCAAGCGAC | |
| 38 | MALPVTALLLPLALLLHAARPDIQMTQTTSSLSASLGDRVTISCRASQDI SKYLNWYQQKPDGTVKLLIYHTSRLHSGVPSRFSGSGSGTDYSLTISNL EQEDIATYFCQQGNTLPYTFGGGTKLEITGGGGSGGGGSGGGGSEVKL QESGPGLVAPSQSLSVTCTVSGVSLPDYGVSWIRQPPRKGLEWLGVIW GSETTYYNSALKSRLTIIKDNSKSQVFLKMNSLQTDDTAIYYCAKHYY YGGSYAMDYWGQGTSVTVSSTTTPAPRPPTPAPTIASQPLSLRPEACRP AAGGAVHTRGLDFACDIYIWAPLAGTCGVLLLSLVITLYCKRGRKKLL YIFKQPFMRPVQTTQEEDGCSCRFPEEEEGGCELRVKFSRSADAPAYKQ GQNQLYNELNLGRREEYDVLDKRRGRDPEMGGKPRRKNPQEGLYNEL QKDKMAEAYSEIGMKGERRRGKGHDGLYQGLSTATKDTYDALHMQA LPPR | CAR-B |
| 39 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGGGGSAAGGPGAGS | CTSR-2C-T2A |
| | AAPVSSTSSLPLAALNMRVRRRLSLFLNVRTQVAADWTALAEEMDFE YLEIRQLETQADPTGRLLDAWQGRPGASVGRLLELLTKLGRDDVLLEL GPSIEEDCQKYILKQQQEEAEKPLQVAAVDSSVPRTAELAGITTLDDPL GHMPERFDAFICYCPSDGSGEGRGSLLTCGDVEENPGP | |
| 40 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGCGGACCAGGGGCAGGTTCAGCTGCTCCAGTAAGTA GCACCTCCTCATTGCCTTTGGCTGCATTGAATATGCGGGTACGGAGA AGACTTAGTCTCTTCTTGAACGTAAGAACTCAAGTCGCCGCAGATTG GACGGCTCTTGCCGAAGAAATGGACTTTGAATATCTCGAGATTCGA CAACTCGAAACCCAAGCGGATCCAACTGGTCGGTTGCTTGACGCTT GGCAGGGTCGCCCTGGAGCGTCTGTCGGCCGATTGCTTGAATTGTTG ACAAAACTTGGCAGAGATGACGTGCTCCTGGAACTCGGCCCCTCCA TCGAAGAGGACTGTCAGAAATACATTCTGAAACAGCAACAAGAGG AAGCGGAAAAGCCCCTGCAGGTCGCTGCAGTTGATTCTAGCGTTCC TCGGACCGCGGAACTTGCAGGCATAACCACTCTCGACGATCCCTTG GGGCACATGCCTGAACGCTTTGACGCCTTCATTTGTTATTGCCCAAG CGACGGTGGCGGTGGCTCGGCGGCTGGCGGACCAGGGGCAGGTTCA GCTGCTCCAGTAAGTAGCACCTCCTCATTGCCTTTGGCTGCATTGAA TATGCGGGTACGGAGAAGACTTAGTCTCTTCTTGAACGTAAGAACT CAAGTCGCCGCAGATTGGACGGCTCTTGCCGAAGAAATGGACTTTG AATATCTCGAGATTCGACAACTCGAAACCCAAGCGGATCCAACTGG TCGGTTGCTTGACGCTTGGCAGGGTCGCCCTGGAGCGTCTGTCGGCC | CTSR-2C-T2A |
| | GATTGCTTGAATTGTTGACAAAACTTGGCAGAGATGACGTGCTCCT GGAACTCGGCCCCTCCATCGAAGAGGACTGTCAGAAATACATTCTG AAACAGCAACAAGAGGAAGCGGAAAAGCCCCTGCAGGTCGCTGCA GTTGATTCTAGCGTTCCTCGGACCGCGGAACTTGCAGGCATAACCA CTCTCGACGATCCCTTGGGGCACATGCCTGAACGCTTTGACGCCTTC ATTTGTTATTGCCCAAGCGACGGATCTGGTGAGGGGCGGGGGTCCC TGCTGACCTGCGGCGACGTCGAAGAAAATCCCGGTCCC | |
| 41 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGSGEGRGSLLTCGDV EENPGP | CTSR-2A-T2A |
| 42 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATATGATTGTCACGGACAACA ACGGTGCGGTCAAATTCCCGCAGCTTTGCAAATTTTGTGATGTTAGA TTCTCAACGTGTGATAATCAAAAGTCCTGCATGAGTAATTGTTCAAT TACTTCCATCTGCGAAAAGCCTCAGGAAGTTTGCGTCGCAGTCTGG AGGAAGAACGACGAAAACATTACACTTGAGACTGTGTGTCATGATC CTAAACTCCCCTATCATGACTTCATCCTTGAGGATGCGGCGTCCCCC AAATGTATCATGAAAGAGAAAAAAAAGCCCGGTGAGACGTTCTTCA TGTGCAGCTGCTCCAGCGACGAGTGCAATGATAACATCATTTTCTCT GAAGAATACAATACCTCAAATCCCGACCTCCTCTTGGTAATATTTCA GGTGACGGGTATAAGTTTGCTTCCGCCTCTCGGTGTGGCGATCTCCG TTATCATTATCTTTTATTGCTATCGCGTGAACAGACAACAAAAATTG TCCGCGGCTGGCGGACCAGGGGCAGGTTCAGCTGCTCCAGTAAGTA GCACCTCCTCATTGCCTTTGGCTGCATTGAATATGCGGGTACGGAGA AGACTTAGTCTCTTCTTGAACGTAAGAACTCAAGTCGCCGCAGATTG GACGGCTCTTGCCGAAGAAATGGACTTTGAATATCTCGAGATTCGA CAACTCGAAACCCAAGCGGATCCAACTGGTCGGTTGCTTGACGCTT | CTSR-2A-T2A |
| | GGCAGGGTCGCCCTGGAGCGTCTGTCGGCCGATTGCTTGAATTGTTG ACAAAACTTGGCAGAGATGACGTGCTCCTGGAACTCGGCCCCTCCA TCGAAGAGGACTGTCAGAAATACATTCTGAAACAGCAACAAGAGG AAGCGGAAAAGCCCCTGCAGGTCGCTGCAGTTGATTCTAGCGTTCC TCGGACCGCGGAACTTGCAGGCATAACCACTCTCGACGATCCCTTG GGGCACATGCCTGAACGCTTTGACGCCTTCATTTGTTATTGCCCAAG CGACGGATCTGGTGAGGGGCGGGGGTCCCTGCTGACCTGCGGCGAC GTCGAAGAAAATCCCGGTCCC | |
| 43 | MALPVTALLLPLALLLHAARPEQKLISEEDLTIPPHVQKSVNNDVTGISL LPPLGVAISVIIIFYCYRVNRQQKLSAAGGPGAGSAAPVSSTSSLPLAAL NMRVRRRLSLFLNVRTQVAADWTALAEEMDFEYLEIRQLETQADPTG RLLDAWQGRPGASVGRLLELLTKLGRDDVLLELGPSIEEDCQKYILKQ QQEEAEKPLQVAAVDSSVPRTAELAGITTLDDPLGHMPERFDAFICYCP SDGGGGSAAGGPGAGSAAPVSSTSSLPLAALNMRVRRRLSLFLNVRTQ VAADWTALAEEMDFEYLEIRQLETQADPTGRLLDAWQGRPGASVGRL LELLTKLGRDDVLLELGPSIEEDCQKYILKQQQEEAEKPLQVAAVDSSV PRTAELAGITTLDDPLGHMPERFDAFICYCPSDGSGEGRGSLLTCGDVE ENPGP | CTSR-2EN-T2A |
| 44 | ATGGCACTTCCCGTTACAGCACTGCTCCTGCCCCTGGCGCTGCTCCT GCACGCGGCCCGCCCCgagcaaaaacttatctctgaagaggacctcACTATTCCCCCT CATGTTCAGAAATCCGTAAACAATGATGTGACGGGTATAAGTTTGC TTCCGCCTCTCGGTGTGGCGATCTCCGTTATCATTATCTTTATTGCT ATCGCGTGAACAGACAACAAAAATTGTCCGCGGCTGGCGGACCAGG GGCAGGTTCAGCTGCTCCAGTAAGTAGCACCTCCTCATTGCCTTTGG CTGCATTGAATATGCGGGTACGGAGAAGACTTAGTCTCTTCTTGAAC GTAAGAACTCAAGTCGCCGCAGATTGGACGGCTCTTGCCGAAGAAA TGGACTTTGAATATCTCGAGATTCGACAACTCGAAACCCAAGCGGA TCCAACTGGTCGGTTGCTTGACGCTTGGCAGGGTCGCCCTGGAGCGT CTGTCGGCCGATTGCTTGAATTGTTGACAAAACTTGGCAGAGATGA CGTGCTCCTGGAACTCGGCCCCTCCATCGAAGAGGACTGTCAGAAA TACATTCTGAAACAGCAACAAGAGGAAGCGGAAAAGCCCCTGCAG GTCGCTGCAGTTGATTCTAGCGTTCCTCGGACCGCGGAACTTGCAGG CATAACCACTCTCGACGATCCCTTGGGGCACATGCCTGAACGCTTTG ACGCCTTCATTTGTTATTGCCCAAGCGACGGTGGCGGTGGCTCGGCG | CTSR-2EN-T2A |
| | GCTGGCGGACCAGGGGCAGGTTCAGCTGCTCCAGTAAGTAGCACCT CCTCATTGCCTTTGGCTGCATTGAATATGCGGGTACGGAGAAGACTT AGTCTCTTCTTGAACGTAAGAACTCAAGTCGCCGCAGATTGGACGG CTCTTGCCGAAGAAATGGACTTTGAATATCTCGAGATTCGACAACTC GAAACCCAAGCGGATCCAACTGGTCGGTTGCTTGACGCTTGGCAGG GTCGCCCTGGAGCGTCTGTCGGCCGATTGCTTGAATTGTTGACAAAA CTTGGCAGAGATGACGTGCTCCTGGAACTCGGCCCCTCCATCGAAG AGGACTGTCAGAAATACATTCTGAAACAGCAACAAGAGGAAGCGG AAAAGCCCCTGCAGGTCGCTGCAGTTGATTCTAGCGTTCCTCGGACC GCGGAACTTGCAGGCATAACCACTCTCGACGATCCCTTGGGGCACA TGCCTGAACGCTTTGACGCCTTCATTTGTTATTGCCCAAGCGACGGA TCTGGTGAGGGGCGGGGGTCCCTGCTGACCTGCGGCGACGTCGAAG AAAATCCCGGTCCC | |
| 45 | MALPVTALLLPLALLLHAARPDIAMTQSQKFMSTSVGDRVSVTCKASQ NVDTNVAWYQQKPGQSPKALIYSASYRYSGVPDRFTGSGSGTDFTLTI NNVQSEDLAEYFCQQYNNYPFTFGSGTKLEIKGGGGSGGGGSGGGGS GGGGSDVQLVESGGGLVQPGGSRKLSCAASGFTFSSFGMHWVRQAPE KGLEWVAYISSDSSAIYYADTVKGRFTISRDNPKNTLFLQMTSLRSEDT AMYYCGRGRENIYYGSRLDYWGQGTTLTVSSTTTPAPRPPTPAPTIASQ PLSLRPEACRPAAGGAVHTRGLDFACDIYIWAPLAGTCGVLLLSLVITL YCKRGRKKLLYIFKQPFMRPVQTTQEEDGCSCRFPEEEEGGCELRVKFS RSADAPAYKQGQNQLYNELNLGRREEYDVLDKRRGRDPEMGGKPRR KNPQEGLYNELQKDKMAEAYSEIGMKGERRRGKGHDGLYQGLSTAT KDTYDALHMQALPPR | CAR-A |
| 46 | atggccttaccagtgaccgccttgctcctgccgctggccttgctgctccacgccgccaggccgGACATCG CCATGACACAATCTCAAAAGTTCATGTCCACGTCAGTCGGAGACCG AGTCTCCGTCACTTGCAAGGCCAGTCAGAATGTGGATACCAATGTA GCATGGTACCAGCAAAAACCCGGACAGTCACCTAAGGCTCTTATAT ACTCCGCTTCTTATAGGTATTCTGGTGTACCTGACAGGTTTACTGGG TCCGGGTCCGGCACAGACTTCACTCTGACAATAAACAATGTGCAAT CAGAGGACCTCGCGGAGTATTTCTGTCAGCAATATAACAACTACCC ATTTACTTTCGGTTCAGGCACTAAACTGGAGATAAAGGGCGGAGGA GGGTCAGGCGGGGGCGGTTCAGGGGGAGGTGGGTCAGGCGGGGG GGTTCCGATGTTCAGTTGGTAGAGTCCGGGGGGGGTCTGGTACAGC | CAR-A |
| | CTGGTGGATCAAGAAAACTGTCTTGCGCGGCGAGTGGGTTCACCTT CTCTAGTTTTGGAATGCACTGGGTACGCCAAGCACCGGAGAAAGGT TTGGAATGGGTCGCATACATTTCTTCAGATAGTTCAGCAATTTATTA CGCGGACACAGTGAAAGGACGGTTTACGATCTCCAGAGACAACCCT AAGAATACGCTCTTTCTCCAAATGACCAGCCTCCGATCTGAAGATA CGGCAATGTATTATTGTGGTCGGGGCAGAGAGAACATTTATTATGG CTCCCGCCTCGATTACTGGGGCCAAGGTACCACTCTGACGGTCTCTT CAaccacgacgccagcgccgcgaccaccaacaccggcgcccaccatcgcgtcgcagcccctgtccctgcg cccagaggcgtgccggccagcggcgggggcgcagtgcacacgagggggctggacttcgcctgtgatatct acatctgggcgcccttggccgggacttgtggggtccttctcctgtcactggttatcaccctttactgcaaacgggg cagaaagaaactcctgtatatattcaaacaaccatttatgagaccagtacaaactactcaagaggaagatggctgt agctgccgatttccagaagaagaagaaggaggatgtgaactgagagtgaagttcagcaggagcgcagacgcc cccgcgtacaagcagggccagaaccagctctataacgagctcaatctaggacgaagagaggagtacgatgtttt ggacaagagacgtggccgggaccctgagatgggggggaaagccgagaaggaagaaccctcaggaaggcctg tacaatgaactgcagaaagataagatggcggaggcctacagtgagattgggatgaaaggcgagcgccggagg ggcaaggggcacgatggcctttaccagggtctcagtacagccaccaaggacacctacgacgcccttcacatgc aggccctgccccctcgctaa | |
| 47 | (GGGGS)ₙ | Linker; n is a number from 1-4 |
| 48 | GGGGS | linker |
| 49 | GGGGSGGGGSGGGGS | linker |
| 50 | GGGGSGGGGSGGGGSGGGGS | linker |
| 51 | IYIWAPLAGTCGVLLLSLVITLYC | CD8a TM |
| 52 | IYIWAPLAGTCGVLLLSLVIT | CD8a TM |
| 53 | RVKFSRSADAPAYQQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | CD3 zeta Homo sapiens |
| 54 | RVKFSRSAEPPAYQQGQNQLYNELNLGRREEYDVLDKRRGRDPEMGG KPRRKNPQEGLYNELQKDKMAEAYSEIGMKGERRRGKGHDGLYQGL STATKDTYDALHMQALPPR | CD3 zeta Homo sapiens |
| 55 | RVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | CD3 zeta Homo sapiens |
| 56 | KRGRKKLLYIFKQPFMRPVQTTQEEDGCSCRFPEEEEGGCEL | 4-1BB (amino acids 214-255 of Q07011.1) Homo sapiens |
| 57 | FWVLVVVGGVLACYSLLVTVAFIIFWV | CD28 (amino acids 153-179 of |
| | | Accession No. P10747) Homo sapiens |
| 58 | IEVMYPPPYLDNEKSNGTIIHVKGKHLCPSPLFPGPSKPFWVLVVVGGV LACYSLLVTVAFIIFWV | CD28 (amino acids 114-179 of Accession No. P10747) Homo sapiens |
| 59 | RSKRSRLLHSDYMNMTPRRPGPTRKHYQPYAPPRDFAAYRS | CD28 (amino acids 180-220 of P10747) Homo sapiens |
| 60 | RSKRSRGGHSDYMNMTPRRPGPTRKHYQPYAPPRDFAAYRS | CD28 (LL to GG) Homo sapiens |
| 61 | EGRGSLLTCGDVEENPGP | T2A |
| 62 | LEGGGEGRGSLLTCGDVEENPGPR | T2A |
| 63 | GSGATNFSLLKQAGDVEENPGP | P2A |
| 64 | ATNFSLLKQAGDVEENPGP | P2A |
| 65 | QCTNYALLKLAGDVESNPGP | E2A |
| 66 | VKQTLNFDLLKLAGDVESNPGP | F2A |
| 67 | MALPVTALLLPLALLLHAARPDIQMTQTTSSLSASLGDRVTISCRASQDI SKYLNWYQQKPDGTVKLLIYHTSRLHSGVPSRFSGSGSGTDYSLTISNL EQEDIATYFCQQGNTLPYTFGGGTKLEIT | CAR-B Light chain |
| 68 | EVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCAK HYYYGGSYAMDYWGQGTSVTVSSTTTPAPRPPTPAPTIASQPLSLRPEA CRPAAGGAVHTRGLDFACD | CAR-B Heavy chain |
| 69 | DYGVS | CAR-B Heavy chain CDR1 |
| 70 | VIWGSETTYYNSALKS | CAR-B Heavy chain CDR2 |
| 71 | HYYYGGSYAMDY | CAR-B Heavy chain CDR3 |
| 72 | RASQDISKYLN | CAR-B Light chain CDR1 |
| 73 | HTSRLHS | CAR-B Light chain CDR2 |
| 74 | QQGNTLPYT | CAR-B Light chain CDR3 |
| 75 | MALPVTALLLPLALLLHAARPDIAMTQSQKFMSTSVGDRVSVTCKASQ NVDTNVAWYQQKPGQSPKALIYSASYRYSGVPDRFTGSGSGTDFTLTI NNVQSEDLAEYFCQQYNNYPFTFGSGTKLEIK | CAR-A Light chain |
| 76 | DVQLVESGGGLVQPGGSRKLSCAASGFTFSSFGMHWVRQAPEKGLEW VAYISSDSSAIYYADTVKGRFTISRDNPKNTLFLQMTSLRSEDTAMYYC | CAR-A Heavy chain |
| | GRGRENIYYGSRLDYWGQGTTLTVSSTTTPAPRPPTPAPTIASQPLSLRP EACRPAAGGAVHTRGLDFACD | |
| 77 | SFGMH | CAR-A Heavy chain CDR1 |
| 78 | YISSDSSAIYYADTVKG | CAR-A Heavy chain CDR2 |
| 79 | GRENIYYGSRLDY | CAR-A Heavy chain CDR3 |
| 80 | KASQNVDTNVA | CAR-A Light chain CDR1 |
| 81 | SASYRYS | CAR-A Light chain CDR2 |
| 82 | QQYNNYPFT | CAR-A Light chain CDR3 |
| 83 | MALPVTALLLPLALLLHAARPDIQMTQTTSSLSASLGDRVTISCRASQDI SKYLNWYQQKPDGTVKLLIYHTSRLHSGVPSRFSGSGSGTDYSLTISNL EQEDIATYFCQQGNTLPYTFGGGTKLEITGGGGSGGGGSGGGGSEVKL QESGPGLVAPSQSLSVTCTVSGVSLPDYGVSWIRQPPRKGLEWLGVIW GSETTYYNSALKSRLTIIKDNSKSQVFLKMNSLQTDDTAIYYCAKHYY YGGSYAMDYWGQGTSVTVSSTTTPAPRPPTPAPTIASQPLSLRPEACRP AAGGAVHTRGLDFACD | CAR-B scFv |
| 84 | MALPVTALLLPLALLLHAARPDIAMTQSQKFMSTSVGDRVSVTCKASQ NVDTNVAWYQQKPGQSPKALIYSASYRYSGVPDRFTGSGSGTDFTLTI NNVQSEDLAEYFCQQYNNYPFTFGSGTKLEIKGGGGSGGGGSGGGGS GGGGSDVQLVESGGGLVQPGGSRKLSCAASGFTFSSFGMHWVRQAPE KGLEWVAYISSDSSAIYYADTVKGRFTISRDNPKNTLFLQMTSLRSEDT AMYYCGRGRENIYYGSRLDYWGQGTTLTVSSTTTPAPRPPTPAPTIASQ PLSLRPEACRPAAGGAVHTRGLDFACD | CAR-A scFv |
| 85 | GGGGSGGGGS | linker |
| 86 | CYRV | TGFBR2 cyto domain 4 aa |
| 87 | TIPPHVQKSVNND | Partial of TGFBR2 extracellular domain |
| 88 | AAGSG | Linker |
| 89 | GSG | Linker |
| 90 | LQCFCHLCTKDNFTCVTDGLCFVSVTETTDKVIHNSMCIAEIDLIPRDRP FVCAPSSKTGSVTTTYCCNQDHCNKIELPTTVKSSPGLGPVELAAVIAG PVCFVCISLMLMVYICHNRTVIHHRVPNEEDPSLDRPFISEGTTLKDLIY DAAGGPGAGSAAPVSSTSSLPLAALNMRVRRRLSLFLNVRTQVAADW TALAEEMDFEYLEIRQLETQADPTGRLLDAWQGRPGASVGRLLELLTK LGRDDVLLELGPSIEEDCQKYILKQQQEEAEKPLQVAAVDSSVPRTAEL AGITTLDDPLGHMPERFDAFICYCPSD* | CTSR-1A |
| 91 | LQCFCHLCTKDNFTCVTDGLCFVSVTETTDKVIHNSMCIAEIDLIPRDRP FVCAPSSKTGSVTTTYCCNQDHCNKIELPTTVKSSPGLGPVELAAVIAG PVCFVCISLMLMVYIGGGGSGGGGSAAGGPGAGSAAPVSSTSSLPLAA LNMRVRRRLSLFLNVRTQVAADWTALAEEMDFEYLEIRQLETQADPT GRLLDAWQGRPGASVGRLLELLTKLGRDDVLLELGPSIEEDCQKYILK QQQEEAEKPLQVAAVDSSVPRTAELAGITTLDDPLGHMPERFDAFICYC PSD | CTSR-1B |
| 92 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRVNRQQKLSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSD | CTSR-2A |
| 93 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRVNRQQKLSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSDAAGGPGAGSAAPVSSTSSLPLAALNMRVRRRLSLFLNVRTQVA ADWTALAEEMDFEYLEIRQLETQADPTGRLLDAWQGRPGASVGRLLE LLTKLGRDDVLLELGPSIEEDCQKYILKQQQEEAEKPLQVAAVDSSVPR TAELAGITTLDDPLGHMPERFDAFICYCPSD | CTSR-2B |
| 94 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRVNRQQKLSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSDGGGGSAAGGPGAGSAAPVSSTSSLPLAALNMRVRRRLSLFLNV | CTSR-2C |
| | RTQVAADWTALAEEMDFEYLEIRQLETQADPTGRLLDAWQGRPGASV GRLLELLTKLGRDDVLLELGPSIEEDCQKYILKQQQEEAEKPLQVAAV DSSVPRTAELAGITTLDDPLGHMPERFDAFICYCPSD | |
| 95 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRV | CTSR-2IN |
| 96 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRVNRQQKLSAAGSGEGRGSLLTCGDVEENP GP | CTSR-2IN-T2A |
| 97 | VTGISLLPPLGVAISVIIIFYCYRVNRQQKLSAAGGPGAGSAAPVSSTSSL PLAALNMRVRRRLSLFLNVRTQVAADWTALAEEMDFEYLEIRQLETQ ADPTGRLLDAWQGRPGASVGRLLELLTKLGRDDVLLELGPSIEEDCQK YILKQQQEEAEKPLQVAAVDSSVPRTAELAGITTLDDPLGHMPERFDAF ICYCPSDGGGGSAAGGPGAGSAAPVSSTSSLPLAALNMRVRRRLSLFLN VRTQVAADWTALAEEMDFEYLEIRQLETQADPTGRLLDAWQGRPGAS VGRLLELLTKLGRDDVLLELGPSIEEDCQKYILKQQQEEAEKPLQVAA VDSSVPRTAELAGITTLDDPLGHMPERFDAFICYCPSD | CTSR-2EN |
| 98 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP KCIMKEKKKPGETFFMCSCSSDECNDNIIFSEEYNTSNPDLLLVIFQVTG ISLLPPLGVAISVIIIFYCYRVNRQQKLSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSDGGGGSAAGGPGAGSAAPVSSTSSLPLAALNMRVRRRLSLFLNV RTQVAADWTALAEEMDFEYLEIRQLETQADPTGRLLDAWQGRPGASV GRLLELLTKLGRDDVLLELGPSIEEDCQKYILKQQQEEAEKPLQVAAV DSSVPRTAELAGITTLDDPLGHMPERFDAFICYCPSDGSGEGRGSLLTC GDVEENPGP | CTSR-2EN-T2A |
| 99 | TIPPHVQKSVNNDMIVTDNNGAVKFPQLCKFCDVRFSTCDNQKSCMSN CSITSICEKPQEVCVAVWRKNDENITLETVCHDPKLPYHDFILEDAASP | CTSR-2A-T2A |
| | KCIMKEKKKPGETFFMCSCSSDECNDNIIFSEEYNTSNPDLLLVIFQVTG ISLLPPLGVAISVIIIFYCYRVNRQQKLSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSDGSGEGRGSLLTCGDVEENPGP | |
| 100 | TIPPHVQKSVNNDVTGISLLPPLGVAISVIIIFYCYRVNRQQKLSAAGGP GAGSAAPVSSTSSLPLAALNMRVRRRLSLFLNVRTQVAADWTALAEE MDFEYLEIRQLETQADPTGRLLDAWQGRPGASVGRLLELLTKLGRDD VLLELGPSIEEDCQKYILKQQQEEAEKPLQVAAVDSSVPRTAELAGITT LDDPLGHMPERFDAFICYCPSDGGGGSAAGGPGAGSAAPVSSTSSLPLA ALNMRVRRRLSLFLNVRTQVAADWTALAEEMDFEYLEIRQLETQADP TGRLLDAWQGRPGASVGRLLELLTKLGRDDVLLELGPSIEEDCQKYIL KQQQEEAEKPLQVAAVDSSVPRTAELAGITTLDDPLGHMPERFDAFIC YCPSDGSGEGRGSLLTCGDVEENPGP | CTSR-2EN-T2A |
| 101 | EQKLISEEDL | Myc tag |
| 102 | MALPVTALLLPLALLLHAARP | CD8a signal peptide |
| 103 | HHHHHH | His tag |
| 104 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGGGGSAAGGPGAGS AAPVSSTSSLPLAALNMRVRRRLSLFLNVRTQVAADWTALAEEMDFE YLEIRQLETQADPTGRLLDAWQGRPGASVGRLLELLTKLGRDDVLLEL GPSIEEDCQKYILKQQQEEAEKPLQVAAVDSSVPRTAELAGITTLDDPL GHMPERFDAFICYCPSDGSGEGRGSLLTCGDVEENPGPMALPVTALLLP LALLLHAARPDIAMTQSQKFMSTSVGDRVSVTCKASQNVDTNVAWYQ QKPGQSPKALIYSASYRYSGVPDRFTGSGSGTDFTLTINNVQSEDLAEY | CTSR-2C-T2A-CAR-A |
| | FCQQYNNYPFTFGSGTKLEIKGGGGSGGGGSGGGGSGGGGSDVQLVES GGGLVQPGGSRKLSCAASGFTFSSFGMHWVRQAPEKGLEWVAYISSDS SAIYYADTVKGRFTISRDNPKNTLFLQMTSLRSEDTAMYYCGRGRENI YYGSRLDYWGQGTTLTVSSTTTPAPRPPTPAPTIASQPLSLRPEACRPAA GGAVHTRGLDFACDIYIWAPLAGTCGVLLLSLVITLYCKRGRKKLLYIF KQPFMRPVQTTQEEDGCSCRFPEEEEGGCELRVKFSRSADAPAYKQGQ NQLYNELNLGRREEYDVLDKRRGRDPEMGGKPRRKNPQEGLYNELQK DKMAEAYSEIGMKGERRRGKGHDGLYQGLSTATKDTYDALHMQALP PR | |
| 105 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGGGGSAAGGPGAGS AAPVSSTSSLPLAALNMRVRRRLSLFLNVRTQVAADWTALAEEMDFE YLEIRQLETQADPTGRLLDAWQGRPGASVGRLLELLTKLGRDDVLLEL GPSIEEDCQKYILKQQQEEAEKPLQVAAVDSSVPRTAELAGITTLDDPL GHMPERFDAFICYCPSDGSGEGRGSLLTCGDVEENPGPMALPVTALLLP LALLLHAARPDIQMTQTTSSLSASLGDRVTISCRASQDISKYLNWYQQK PDGTVKLLIYHTSRLHSGVPSRFSGSGSGTDYSLTISNLEQEDIATYFCQ QGNTLPYTFGGGTKLEITGGGGSGGGGSGGGGSEVKLQESGPGLVAPS QSLSVTCTVSGVSLPDYGVSWIRQPPRKGLEWLGVIWGSETTYYNSAL KSRLTIIKDNSKSQVFLKMNSLQTDDTAIYYCAKHYYYGGSYAMDYW GQGTSVTVSSTTTPAPRPPTPAPTIASQPLSLRPEACRPAAGGAVHTRGL DFACDIYIWAPLAGTCGVLLLSLVITLYCKRGRKKLLYIFKQPFMRPVQ TTQEEDGCSCRFPEEEEGGCELRVKFSRSADAPAYKQGQNQLYNELNL GRREEYDVLDKRRGRDPEMGGKPRRKNPQEGLYNELQKDKMAEAYS EIGMKGERRRGKGHDGLYQGLSTATKDTYDALHMQALPPR | CTSR-2C-T2A-CAR-B |
| 106 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS | CTSR-2A-T2A-CAR-A |
| | CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGSGEGRGSLLTCGDV EENPGPMALPVTALLLPLALLLHAARPDIAMTQSQKFMSTSVGDRVSV TCKASQNVDTNVAWYQQKPGQSPKALIYSASYRYSGVPDRFTGSGSG TDFTLTINNVQSEDLAEYFCQQYNNYPFTFGSGTKLEIKGGGGSGGGGS GGGGSGGGGSDVQLVESGGGLVQPGGSRKLSCAASGFTFSSFGMHWV RQAPEKGLEWVAYISSDSSAIYYADTVKGRFTISRDNPKNTLFLQMTSL RSEDTAMYYCGRGRENIYYGSRLDYWGQGTTLTVSSTTTPAPRPPTPA PTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | |
| 107 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGGPGAGSAAPVSSTSSLPLAALNMRVRRRLSLFLNVRT QVAADWTALAEEMDFEYLEIRQLETQADPTGRLLDAWQGRPGASVGR LLELLTKLGRDDVLLELGPSIEEDCQKYILKQQQEEAEKPLQVAAVDSS VPRTAELAGITTLDDPLGHMPERFDAFICYCPSDGSGEGRGSLLTCGDV EENPGPMALPVTALLLPLALLLHAARPDIQMTQTTSSLSASLGDRVTIS CRASQDISKYLNWYQQKPDGTVKLLIYHTSRLHSGVPSRFSGSGSGTD YSLTISNLEQEDIATYFCQQGNTLPYTFGGGTKLEITGGGGSGGGGSGG GGSEVKLQESGPGLVAPSQSLSVTCTVSGVSLPDYGVSWIRQPPRKGLE WLGVIWGSETTYYNSALKSRLTIIKDNSKSQVFLKMNSLQTDDTAIYY CAKHYYYGGSYAMDYWGQGTSVTVSSTTTPAPRPPTPAPTIASQPLSL RPEACRPAAGGAVHTRGLDFACDIYIWAPLAGTCGVLLLSLVITLYCK RGRKKLLYIFKQPFMRPVQTTQEEDGCSCRFPEEEEGGCELRVKFSRSA DAPAYKQGQNQLYNELNLGRREEYDVLDKRRGRDPEMGGKPRRKNP | CTSR-2C-T2A-CAR-B |
| | QEGLYNELQKDKMAEAYSEIGMKGERRRGKGHDGLYQGLSTATKDT YDALHMQALPPR | |
| 108 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGSGEGRGSLLTCGDVEENPGPMALPVTALLLPLALLLH AARPDIAMTQSQKFMSTSVGDRVSVTCKASQNVDTNVAWYQQKPGQ SPKALIYSASYRYSGVPDRFTGSGSGTDFTLTINNVQSEDLAEYFCQQY NNYPFTFGSGTKLEIKGGGGSGGGGSGGGGSGGGGSDVQLVESGGGL VQPGGSRKLSCAASGFTFSSFGMHWVRQAPEKGLEWVAYISSDSSAIY YADTVKGRFTISRDNPKNTLFLQMTSLRSEDTAMYYCGRGRENIYYGS RLDYWGQGTTLTVSSTTTPAPRPPTPAPTIASQPLSLRPEACRPAAGGA VHTRGLDFACDIYIWAPLAGTCGVLLLSLVITLYCKRGRKKLLYIFKQP FMRPVQTTQEEDGCSCRFPEEEEGGCELRVKFSRSADAPAYKQGQNQL YNELNLGRREEYDVLDKRRGRDPEMGGKPRRKNPQEGLYNELQKDK MAEAYSEIGMKGERRRGKGHDGLYQGLSTATKDTYDALHMQALPPR | CTSR-2IN-T2A-CAR-A |
| 109 | MALPVTALLLPLALLLHAARPEQKLISEEDLTIPPHVQKSVNNDMIVTD NNGAVKFPQLCKFCDVRFSTCDNQKSCMSNCSITSICEKPQEVCVAVW RKNDENITLETVCHDPKLPYHDFILEDAASPKCIMKEKKKPGETFFMCS CSSDECNDNIIFSEEYNTSNPDLLLVIFQVTGISLLPPLGVAISVIIIFYCYR VNRQQKLSAAGSGEGRGSLLTCGDVEENPGPMALPVTALLLPLALLLH AARPDIQMTQTTSSLSASLGDRVTISCRASQDISKYLNWYQQKPDGTV KLLIYHTSRLHSGVPSRFSGSGSGTDYSLTISNLEQEDIATYFCQQGNTL PYTFGGGTKLEITGGGGSGGGGSGGGGSEVKLQESGPGLVAPSQSLSV TCTVSGVSLPDYGVSWIRQPPRKGLEWLGVIWGSETTYYNSALKSRLTI IKDNSKSQVFLKMNSLQTDDTAIYYCAKHYYYGGSYAMDYWGQGTS VTVSSTTTPAPRPPTPAPTIASQPLSLRPEACRPAAGGAVHTRGLDFACD IYIWAPLAGTCGVLLLSLVITLYCKRGRKKLLYIFKQPFMRPVQTTQEE DGCSCRFPEEEEGGCELRVKFSRSADAPAYKQGQNQLYNELNLGRREE YDVLDKRRGRDPEMGGKPRRKNPQEGLYNELQKDKMAEAYSEIGMK GERRRGKGHDGLYQGLSTATKDTYDALHMQALPPR | CTSR-2IN-T2A-CAR-B |
| 110 | MALPVTALLLPLALLLHAARPEQKLISEEDLTIPPHVQKSVNNDVTGISL LPPLGVAISVIIIFYCYRVNRQQKLSAAGGPGAGSAAPVSSTSSLPLAAL | CTSR-2EN-T2A-CAR-A |
| | NMRVRRRLSLFLNVRTQVAADWTALAEEMDFEYLEIRQLETQADPTG RLLDAWQGRPGASVGRLLELLTKLGRDDVLLELGPSIEEDCQKYILKQ QQEEAEKPLQVAAVDSSVPRTAELAGITTLDDPLGHMPERFDAFICYCP SDGGGGSAAGGPGAGSAAPVSSTSSLPLAALNMRVRRRLSLFLNVRTQ VAADWTALAEEMDFEYLEIRQLETQADPTGRLLDAWQGRPGASVGRL LELLTKLGRDDVLLELGPSIEEDCQKYILKQQQEEAEKPLQVAAVDSSV PRTAELAGITTLDDPLGHMPERFDAFICYCPSDGSGEGRGSLLTCGDVE ENPGPMALPVTALLLPLALLLHAARPDIAMTQSQKFMSTSVGDRVSVT CKASQNVDTNVAWYQQKPGQSPKALIYSASYRYSGVPDRFTGSGSGT DFTLTINNVQSEDLAEYFCQQYNNYPFTFGSGTKLEIKGGGGSGGGGS GGGGSGGGGSDVQLVESGGGLVQPGGSRKLSCAASGFTFSSFGMHWV RQAPEKGLEWVAYISSDSSAIYYADTVKGRFTISRDNPKNTLFLQMTSL RSEDTAMYYCGRGRENIYYGSRLDYWGQGTTLTVSSTTTPAPRPPTPA PTIASQPLSLRPEACRPAAGGAVHTRGLDFACDIYIWAPLAGTCGVLLL SLVITLYCKRGRKKLLYIFKQPFMRPVQTTQEEDGCSCRFPEEEEGGCE LRVKFSRSADAPAYKQGQNQLYNELNLGRREEYDVLDKRRGRDPEMG GKPRRKNPQEGLYNELQKDKMAEAYSEIGMKGERRRGKGHDGLYQG LSTATKDTYDALHMQALPPR | |
| 111 | MALPVTALLLPLALLLHAARPEQKLISEEDLTIPPHVQKSVNNDVTGISL LPPLGVAISVIIIFYCYRVNRQQKLSAAGGPGAGSAAPVSSTSSLPLAAL NMRVRRRLSLFLNVRTQVAADWTALAEEMDFEYLEIRQLETQADPTG RLLDAWQGRPGASVGRLLELLTKLGRDDVLLELGPSIEEDCQKYILKQ QQEEAEKPLQVAAVDSSVPRTAELAGITTLDDPLGHMPERFDAFICYCP SDGGGGSAAGGPGAGSAAPVSSTSSLPLAALNMRVRRRLSLFLNVRTQ VAADWTALAEEMDFEYLEIRQLETQADPTGRLLDAWQGRPGASVGRL LELLTKLGRDDVLLELGPSIEEDCQKYILKQQQEEAEKPLQVAAVDSSV PRTAELAGITTLDDPLGHMPERFDAFICYCPSDGSGEGRGSLLTCGDVE ENPGPMALPVTALLLPLALLLHAARPDIQMTQTTSSLSASLGDRVTISC RASQDISKYLNWYQQKPDGTVKLLIYHTSRLHSGVPSRFSGSGSGTDYS LTISNLEQEDIATYFCQQGNTLPYTFGGGTKLEITGGGGSGGGGSGGGG SEVKLQESGPGLVAPSQSLSVTCTVSGVSLPDYGVSWIRQPPRKGLEWL GVIWGSETTYYNSALKSRLTIIKDNSKSQVFLKMNSLQTDDTAIYYCAK HYYYGGSYAMDYWGQGTSVTVSSTTTPAPRPPTPAPTIASQPLSLRPEA CRPAAGGAVHTRGLDFACDIYIWAPLAGTCGVLLLSLVITLYCKRGRK | CTSR-2EN-T2A-CAR-B |
| | KLLYIFKQPFMRPVQTTQEEDGCSCRFPEEEEGGCELRVKFSRSADAPA YKQGQNQLYNELNLGRREEYDVLDKRRGRDPEMGGKPRRKNPQEGL YNELQKDKMAEAYSEIGMKGERRRGKGHDGLYQGLSTATKDTYDAL HMQALPPR | |
| 112 | GGATCTGCGATCGCTCCGGTGCCCGTCAGTGGGCAGAGCGCACATC GCCCACAGTCCCCGAGAAGTTGGGGGGAGGGGTCGGCAATTGAACC GGTGCCTAGAGAAGGTGGCGCGGGGTAAACTGGGAAAGTGATGTC GTGTACTGGCTCCGCCTTTTTCCCGAGGGTGGGGGAGAACCGTATAT AAGTGCAGTAGTCGCCGTGAACGTTCTTTTTCGCAACGGGTTTGCCG CCAGAACACAGCTGAAGCTTCGAGGGGCTCGCATCTCTCCTTCACG CGCCCGCCGCCCTACCTGAGGCCGCCATCCACGCCGGTTGAGTCGC GTTCTGCCGCCTCCCGCCTGTGGTGCCTCCTGAACTGCGTCCGCCGT CTAGGTAAGTTTAAAGCTCAGGTCGAGACCGGGCCTTTGTCCGGCG CTCCCTTGGAGCCTACCTAGACTCAGCCGGCTCTCCACGCTTTGCCT GACCCTGCTTGCTCAACTCTACGTCTTTGTTTCGTTTTCTGTTCTGCG CCGTTACAGATCCAAGCTGTGACCGGCGCCTAC | Ef1alpha promoter with HTLV1 enhancer |
| 113 | GSGEGRGSLLTCGDVEENPGP | T2A |
| 114 | AAGSGEGRGSLLTCGDVEENPGP | T2A |
| 115 | GSTSGSGKPGSGEGSTKG | Linker |
| 116 | SRGGGGSGGGGSGGGGSLEMA | Linker |
| 117 | DIQMTQTTSSLSASLGDRVTISCRASQDISKYLNWYQQKPDGTVKLLIY HTSRLHSGVPSRFSGSGSGTDYSLTISNLEQEDIATYFCQQGNTLPYTFG GGTKLEITGGGGSGGGGSGGGGSEVKLQESGPGLVAPSQSLSVTCTVS GVSLPDYGVSWIRQPPRKGLEWLGVIWGSETTYYNSALKSRLTIIKDNS KSQVFLKMNSLQTDDTAIYYCAKHYYYGGSYAMDYWGQGTSVTVSS TTTPAPRPPTPAPTIASQPLSLRPEACRPAAGGAVHTRGLDFACDIYIWA PLAGTCGVLLLSLVITLYCKRGRKKLLYIFKQPFMRPVQTTQEEDGCSC RFPEEEEGGCELRVKFSRSADAPAYKQGQNQLYNELNLGRREEYDVLD KRRGRDPEMGGKPRRKNPQEGLYNELQKDKMAEAYSEIGMKGERRR GKGHDGLYQGLSTATKDTYDALHMQALPPR | CAR-B (no signal peptide) |
| 118 | DIAMTQSQKFMSTSVGDRVSVTCKASQNVDTNVAWYQQKPGQSPKA LIYSASYRYSGVPDRFTGSGSGTDFTLTINNVQSEDLAEYFCQQYNNYP FTFGSGTKLEIKGGGGSGGGGSGGGGSGGGGSDVQLVESGGGLVQPG GSRKLSCAASGFTFSSFGMHWVRQAPEKGLEWVAYISSDSSAIYYADT VKGRFTISRDNPKNTLFLQMTSLRSEDTAMYYCGRGRENIYYGSRLDY WGQGTTLTVSSTTTPAPRPPTPAPTIASQPLSLRPEACRPAAGGAVHTR GLDFACDIYIWAPLAGTCGVLLLSLVITLYCKRGRKKLLYIFKQPFMRP VQTTQEEDGCSCRFPEEEEGGCELRVKFSRSADAPAYKQGQNQLYNEL NLGRREEYDVLDKRRGRDPEMGGKPRRKNPQEGLYNELQKDKMAEA YSEIGMKGERRRGKGHDGLYQGLSTATKDTYDALHMQALPPR | CAR-A (no signal peptide) |
| 119 | HGTELPSPPSVWFEAEFFHHILHWTPIPNQSESTCYEVALLRYGIESWNS ISNCSQTLSYDLTAVTLDLYHSNGYRARVRAVDGSRHSNWTVTNTRFS VDEVTLTVGSVNLEIHNGFILGKIQLPRPKMAPANDTYESIFSHFREYEI AIRKVPGNFTFTHKKVKHENFSLLTSGEVGEFCVQVKPSVASRSNKGM WSKEECISLTRQYFTVTN | IL10Ra Extracellular domain |
| 120 | VIIFFAFVLLLSGALAYCLAL | IL10Ra Transmembrane domain |
| 121 | QLYVRRRKKLPSVLLFKKPSPFIFISQRPSPETQDTIHPLDEEAFLKVSPE LKNLDLHGSTDSGFGSTKPSLQTEEPQFLLPDPHPQADRTLGNREPPVL GDSCSSGSSNSTDSGICLQEPSLSPSTGPTWEQQVGSNSRGQDDSGIDLV QNSEGRAGDTQGGSALGHHSPPEPEVPGEEDPAAVAFQGYLRQTRCAE EKATKTGCLEEESPLTDGLGPKFGRCLVDEAGLHPPALAKGYLKQDPL EMTLASSGAPTGQWNQPTEEWSLLALSSCSDLGISDWSFAHDLAPLGC VAAPGGLLGSFNSDLVTLPLISSLQSSE | IL10Ra Cytoplasmic domain |
| 122 | MVPPPENVRMNSVNFKNILQWESPAFAKGNLTFTAQYLSYRIFQDKCM NTTLTECDFSSLSKYGDHTLRVRAEFADEHSDWVNITFCPVDDTIIGPP GMQVEVLADSLHMRFLAPKIENEYETWTMKNVYNSWTYNVQYWKN GTDEKFQITPQYDFEVLRNLEPWTTYCVQVRGFLPDRNKAGEWSEPVC EQTTHDETVPS | IL10Rb Extracellular domain |
| 123 | WMVAVILMASVFMVCLALLGCF | IL10Rb Transmembrane domain |
| 124 | ALLWCVYKKTKYAFSPRNSLPQHLKEFLGHPHHNTLLFFSFPLSDEND VFDKLSVIAEDSESGKQNPGDSCSLGTPPGQGPQS | IL10Rb Cytoplasmic domain |
| 125 | MKVLQEPTCVSDYMSISTCEWKMNGPTNCSTELRLLYQLVFLLSEAHT CIPENNGGAGCVCHLLMDDVVSADNYTLDLWAGQQLLWKGSFKPSE HVKPRAPGNLTVHTNVSDTLLLTWSNPYPPDNYLYNHLTYAVNIWSE NDPADFRIYNVTYLEPSLRIAASTLKSGISYRARVRAWAQCYNTTWSE WSPSTKWHNSYREPFEQH | IL4R Extracellular domain |
| 126 | LLLGVSVSCIVILAVCLLCYVSIT | IL4R Transmembrane domain |
| 127 | KIKKEWWDQIPNPARSRLVAIIIQDAQGSQWEKRSRGQEPAKCPHWKN CLTKLLPCFLEHNMKRDEDPHKAAKEMPFQGSGKSAWCPVEISKTVL WPESISVVRCVELFEAPVECEEEEEVEEEKGSFCASPESSRDDFQEGREG IVARLTESLFLDLLGEENGGFCQQDMGESCLLPPSGSTSAHMPWDEFPS AGPKEAPPWGKEQPLHLEPSPPASPTQSPDNLTCTETPLVIAGNPAYRSF SNSLSQSPCPRELGPDPLLARHLEEVEPEMPCVPQLSEPTTVPQPEPETW EQILRRNVLQHGAAAAPVSAPTSGYQEFVHAVEQGGTQASAVVGLGP PGEAGYKAFSSLLASSAVSPEKCGFGASSGEEGYKPFQDLIPGCPGDPA PVPVPLFTFGLDREPPRSPQSSHLPSSSPEHLGLEPGEKVEDMPKPPLPQ EQATDPLVDSLGSGIVYSALTCHLCGHLKQCHGQEDGGQTPVMASPCC GCCCGDRSSPPTTPLRAPDPSPGGVPLEASLCPASLAPSGISEKSKSSSSF HPAPGNAQSSSQTPKIVNFVSVGPTYMRVS | IL4R Cytoplasmic domain |
| 128 | MAAGGPGAGSAAPVSSTSSLPLAALNMRVRRRLSLFLNVRTQVAADW TALAEEMDFEYLEIRQLETQADPTGRLLDAWQGRPGASVGRLLELLTK LGRDDVLLELGPSIEEDCQKYILKQQQEEAEKPLQVAAVDSSVPRTAEL AGITTLDDPLGHMPERFDAFICYCPSDIQFVQEMIRQLEQTNYRLKLCV SDRDVLPGTCVWSIASELIEKRCRRMVVVVSDDYLQSKECDFQTKFAL SLSPGAHQKRLIPIKYKAMKKEFPSILRFITVCDYTNPCTKSWFWTRLA KALSLP | MyD88 |
| 129 | CYRVNRQQKLSSTWETGKTRKLMEFSEHCAIILEDDRSDISSTCANNIN HNTELLPIELDTLVGKGRFAEVYKAKLKQNTSEQFETVAVKIFPYEEYA SWKTEKDIFSDINLKHENILQFLTAEERKTELGKQYWLITAFHAKGNLQ EYLTRHVISWEDLRKLGSSLARGIAHLHSDHTPCGRPKMPIVHRDLKSS NILVKNDLTCCLCDFGLSLRLDPTLSVDDLANSGQVGTARYMAPEVLE SRMNLENVESFKQTDVYSMALVLWEMTSRCNAVGEVKDYEPPFGSKV REHPCVESMKDNVLRDRGRPEIPSFWLNHQGIQMVCETLTECWDHDPE ARLTAQCVAERFSELEHLDRLSGRSCSEEKIPEDGSLNTTK | TGFβR2 Cytoplasmic domain |
| 130 | CHNRTVIHHRVPNEEDPSLDRPFISEGTTLKDLIYDMTTSGSGSGLPLLV QRTIARTIVLQESIGKGRFGEVWRGKWRGEEVAVKIFSSREERSWFREA EIYQTVMLRHENILGFIAADNKDNGTWTQLWLVSDYHEHGSLFDYLN RYTVTVEGMIKLALSTASGLAHLHMEIVGTQGKPAIAHRDLKSKNILV KKNGTCCIADLGLAVRHDSATDTIDIAPNHRVGTKRYMAPEVLDDSIN MKHFESFKRADIYAMGLVFWEIARRCSIGGIHEDYQLPYYDLVPSDPSV EEMRKVVCEQKLRPNIPNRWQSCEALRVMAKIMRECWYANGAARLT ALRIKKTLSQLSQQEGIKM | TGFβR1 Cytoplasmic domain |

**THE INVENTION PROVIDES THE FOLLOWING NUMBERED EMBODIMENTS:**
1. A chimeric signaling receptor comprising:
   (a) an extracellular domain of a TGFβ receptor (TGFβR) or a portion thereof that binds TGFβ;
   (b) a transmembrane domain;
   (c) a first truncated MyD88 polypeptide that lacks the full-length TIR domain of full-length MyD88; and
   (d) a second truncated MyD88 domain that lacks the full-length TIR domain of full-length MyD88.
2. The chimeric signaling receptor of embodiment 1, wherein the TGFβR is a TGFβR2.
3. The chimeric signaling receptor of embodiment 1 or embodiment 2, wherein the extracellular domain or the portion thereof comprises:
   (i) the sequence of amino acids set forth in SEQ ID NO: 20;
   (ii) a sequence of amino acids that exhibits at least or about 85%, at least or about 90%, at least or about 92%, at least or about 95%, at least or about 97% sequence identity to the sequence set forth in SEQ ID NO: 20; or
   (iii) a portion of (i) or (ii) that binds TGFβ.
4. The chimeric signaling receptor of any of embodiments 1-3, wherein the extracellular domain or the portion thereof is set forth in SEQ ID NO:20.
5. The chimeric signaling receptor of any of embodiments 1-4, wherein the transmembrane domain comprises the native transmembrane domain of the TGFβR.
6. The chimeric signaling receptor of any of embodiments 1-5, wherein the transmembrane domain comprises the sequence of amino acids set forth in SEQ ID NO: 22 or a sequence of amino acids that exhibits at least or about 85%, at least or about 90%, at least or about 92%, at least or about 95%, at least or about 97% sequence identity to the sequence set forth in SEQ ID NO: 22.
7. The chimeric signaling receptor of any of embodiments 1-6, wherein the transmembrane domain is set forth in SEQ ID NO:22.
8. The chimeric signaling receptor of any of embodiments 1-4, wherein the transmembrane domain is a heterologous transmembrane domain from a transmembrane protein other than the TGFβR.
9. The chimeric signaling receptor of any of embodiments 1-8, wherein the transmembrane domain and the first truncated MyD88 polypeptide are directly linked.
10. The chimeric signaling receptor of any of embodiments 1-9, wherein the transmembrane domain and the first truncated MyD88 polypeptide are indirectly linked by a linker.
11. The chimeric signaling receptor of embodiment 10, wherein the linker is or comprises a peptide linker.
12. The chimeric signaling receptor of embodiment 10, wherein the linker is or comprises a partial sequence of N-terminal contiguous amino acids of the cytoplasmic domain of the TGFβR that is a non-functional portion, wherein the non-functional portion is not a functional inhibitory signaling domain capable of mediating inhibitory signaling.
13. The chimeric signaling receptor of any of embodiments 1-12, wherein the intracellular domain is not capable of recruiting an inhibitory adaptor molecule, optionally a SMAD.
14. The chimeric signaling receptor of embodiment 10, 12 or 13, wherein the linker is amino acids 1-2, 1-3, 1-4, 1-5, 1-6, 1-7, 1-8, 1-9, 1-10, 1-11, 1-12, 1-13, 1-14, 1-15, 1-16, 1-17, 1-18, 1-19, 1-20, 1-21, 1-22, 1-23, 1-24, or 1-25 of the TGFβR2 cytoplasmic domain set forth in SEQ ID NO:129, optionally wherein the linker is set forth in SEQ ID NO:24.
15. A chimeric signaling receptor comprising (i) a portion of a TGFβ receptor (TGFβR) comprising the extracellular domain and the transmembrane domain, wherein the portion is less than the full-length TGFβR and lacks a functional inhibitory signaling domain of the full-length TGFβR; and (ii) an intracellular domain comprising a first truncated MyD88 polypeptide that lacks the full-length TIR domain of full-length MyD88, and a second truncated MyD88 domain that lacks the full-length TIR domain of full-length MyD88.
16. The chimeric signaling receptor of embodiment 15, wherein the TGFβR is a TGFβR2.
17. The chimeric signaling receptor of embodiment 15 or embodiment 16, wherein the portion of the TGFPR comprises the sequence of amino acids set forth in SEQ ID NO: 3 or a sequence of amino acids that exhibits at least or about 85%, at least or about 90%, at least or about 92%, at least or about 95%, at least or about 97% sequence identity to the sequence set forth in SEQ ID NO: 3.
18. The chimeric signaling receptor of any of embodiments 15-17, wherein the portion of the TGFβR is set forth in SEQ ID NO:3.
19. The chimeric signaling receptor of any of embodiments 15-18, wherein the portion of the TGFβR and the intracellular domain are directly linked.
20. The chimeric signaling receptor of any of embodiments 15-18, wherein the portion of the TGFβR and the intracellular domain are indirectly linked by a peptide linker.
21. The chimeric signaling receptor of any of embodiments 1-20, wherein the first truncated MyD88 polypeptide and the second MyD88 polypeptide each comprise the death domain (DD), the intermediate domain (ID) and a portion of the full-length TIR domain of MyD88.
22. The chimeric signaling receptor of any of embodiments 1-21, wherein the first truncated MyD88 polypeptide and the second MyD88 polypeptide each independently is a sequence of amino acids selected from the group consisting of amino acids 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179 or 2-180 of the full-length MyD88, optionally of SEQ ID NO:128.
23. The chimeric signaling receptor of any of embodiments 1-21, wherein the first truncated MyD88 polypeptide and the second truncated polypeptide each independently is a sequence of amino acids 2-171 or 2-172 of the full-length MyD88, optionally of SEQ ID NO:128.
24. The chimeric signaling receptor of any of embodiments 1-23, wherein the first truncated MyD88 polypeptide is set forth in SEQ ID NO: 2.
25. The chimeric signaling receptor of any of embodiments 1-24, wherein the second truncated MyD88 polypeptide is set forth in SEQ ID NO: 2.
26. The chimeric signaling receptor of any of embodiments 1-25, wherein the first truncated MyD88 polypeptide and the second truncated MyD88 polypeptide are the same.
27. The chimeric signaling receptor of any of embodiments 1-26, wherein the first MyD88 polypeptide and the second MyD88 polypeptide are connected by a peptide linker.
28. The chimeric signaling receptor of embodiment 11, 20 and 27, wherein the peptide linker is (G₄S)ₙ (SEQ ID NO: 47), wherein n is an integer between 1 to 4, inclusive.
29. The chimeric signaling receptor of embodiment 11, 20, 27 and 28, wherein the peptide linker is set forth in SEQ ID NO: 47 ((GGGGS)ₙ), where n is an integer between 1 and 4, inclusive, optionally wherein the peptide linker is selected from the group consisting of SEQ ID NO:48 (GGGGS), SEQ ID NO: 85 (GGGGSGGGGS), SEQ ID NO: 49 (GGGGSGGGGSGGGGS), and SEQ ID NO: 50 (GGGGSGGGGSGGGGSGGGGS).
30. The chimeric signaling receptor of any of embodiments 1-29, wherein the chimeric signaling receptor is set forth in SEQ ID NO:93 or a sequence of amino acids that exhibits at least at or about 85%, at least at or about 90%, at least at or about 92%, at least at or about 95%, or at least at or about97% sequence identity to the sequence set forth in SEQ ID NO:93.
31. The chimeric signaling receptor of any of embodiments 1-30, wherein the chimeric signaling receptor is set forth in SEQ ID NO:93.
32. The chimeric signaling receptor of any of embodiments 1-29, wherein the chimeric signaling receptor is set forth in SEQ ID NO:94 or a sequence of amino acids that exhibits at least at or about 85%, at least at or about 90%, at least at or about 92%, at least at or about 95%, or at least at or about 97% sequence identity to the sequence set forth in SEQ ID NO:94.
33. The chimeric signaling receptor of any of embodiments 1-32, wherein the chimeric signaling receptor is set forth in SEQ ID NO:94.
34. The chimeric signaling receptor of any of embodiments 1-33, wherein, when the chimeric signaling receptor is expressed from a cell, binding of TGFβ to the extracellular domain induces dimerization of the first and second MyD88 polypeptide.
35. The chimeric signaling receptor of any of embodiments 1-34, wherein, when the chimeric signaling receptor is expressed from a cell, binding of TGFβ to the extracellular domain recruits an IRAK4 to the first MyD88 polypeptide and the second MyD88 polypeptide.
36. The chimeric signaling receptor of any of embodiments 1-35, wherein, when the chimeric signaling receptor is expressed from a cell, binding of TGFβ to the extracellular domain results in phosphorylation of IRAK4 .
37. A polynucleotide comprising a sequence of nucleotides encoding the chimeric signaling receptor of any of embodiments 1-36.
38. The polynucleotide of embodiment 37, wherein the sequence of nucleotides is a first sequence of nucleotides and the polynucleotide further comprises a second sequence of nucleotides encoding another recombinant molecule.
39. The polynucleotide of embodiment 38, wherein the recombinant molecule is a recombinant antigen receptor, optionally wherein the recombinant antigen receptor is a recombinant T cell receptor (TCR) or is a chimeric antigen receptor (CAR).
40. The polynucleotide of embodiment 38 or 39, wherein the recombinant antigen receptor is a CAR.
41. The polynucleotide of any of embodiments 38-40, wherein the recombinant antigen receptor binds a tumor antigen, optionally wherein the tumor antigen is a CD19 or B7H3.
42. The polynucleotide of embodiment 41, wherein the recombinant molecule is a cytokine, optionally an interleukin or a functional portion thereof, optionally wherein the interleukin is IL-2, IL-12, or IL-15 or a functional portion thereof.
43. The polynucleotide of embodiment 38, wherein the recombinant molecule is a bispecific antibody, optionally wherein the bispecific antibody is a bispecific T cell engager (BiTE).
44. The polynucleotide of any of embodiments 38-43, wherein the first sequence of nucleotides and the second sequence of nucleotides are separated by a bicistronic element.
45. The polynucleotide of embodiment 44, wherein the bicistronic element is an IRES or is a cleavable peptide.
46. The polynucleotide of embodiment 45, wherein the cleavable peptide is selected from the group consisting of a P2A, a T2A and an F2A.
47. The polynucleotide of embodiment 45 or embodiment 46, wherein the cleavable peptide is a T2A.
48. The polynucleotide of any of embodiments 37-47, wherein the sequence of nucleotides is operably linked to a promoter.
49. The polynucleotide of any of embodiments 38-47, wherein the first sequence of nucleotides and the second sequence of nucleotides are each operably linked to a promoter.
50. The polynucleotide of embodiment 49, wherein the promoter is the same promoter.
51. The polynucleotide of any of embodiments 48-50, wherein the promoter is an EF1 promoter.
52. A vector comprising the polynucleotide of embodiment 37, 48 or 51.
53. A vector comprising the polynucleotide of any of embodiments 37-51.
54. The vector of embodiment 52 or embodiment 53, wherein the vector is a viral vector.
55. The vector of embodiment 54, wherein the viral vector is a retroviral vector.
56. The vector of embodiment 54 or embodiment 55, wherein the viral vector is a lentiviral vector.
57. A method of producing an engineered cell, the method comprising introducing the polynucleotide of any of embodiments 38-51 or the vector of any of embodiments 53-56 into a cell under conditions for expression of the chimeric signaling receptor and the recombinant molecule.
58. A method of producing an engineered cell, the method comprising introducing the polynucleotide of embodiment 37-51 or the vector of any of embodiments 52-56 into a cell under conditions for expression of the chimeric signaling receptor on the surface of the cell.
59. The method of embodiment 58, further comprising introducing a polynucleotide encoding a recombinant antigen receptor into the cell under conditions for expression of the recombinant antigen receptor on the surface of the cell.
60. The method of embodiment 59, wherein the recombinant antigen receptor is a recombinant T cell receptor (TCR) or is a chimeric antigen receptor (CAR).
61. The method of embodiment 59 or embodiment 60, wherein the recombinant antigen receptor is a CAR.
62. The method of embodiment 60 or embodiment 61, wherein the recombinant antigen receptor binds a tumor antigen, optionally wherein the tumor antigen is a CD19 or a B7H3.
63. The method of embodiment 62, further comprising introducing a polynucleotide encoding a cytokine into the cell under conditions for expression, and optionally secretion, of the cytokine, optionally wherein the cytokine is an interleukin or a functional portion thereof, optionally wherein the interleukin is IL-2, IL-12, or IL-15 or a functional portion thereof .
64. The method of embodiment 58, further comprising introducing a polynucleotide encoding a bispecific antibody into the cell under conditions for expression, and optionally secretion, of the bispecific antibody, optionally wherein the bispecific antibody is a BiTE..
65. The method of any of embodiments 57-64, wherein the cell is a primary cell from a subject.
66. The method of any of embodiments 57-65, wherein the cell is a T cell, a tumor-infiltrating cell (TIL), a B cell, a natural killer cell, or a macrophage.
67. The method of any of embodiments 57-66, wherein the cell is a T cell, optionally a CD3+, CD4+, or CD8+ T cells.
68. The method any of embodiments 57-67, wherein the cell is a CD8+ T cell.
69. The method any of embodiments 57-67, wherein the cell is a CD4+ T cell.
70. An engineered cell produced by the method of any of embodiments 57-69.
71. An engineered cell comprising a chimeric signaling receptor of any of embodiments 1-36.
72. The engineered cell of embodiment 71, wherein the engineered cell further comprises a recombinant molecule.
73. An engineered cell comprising a polynucleotide of embodiment 37.
74. The engineered cell of embodiment 73, further comprising a polynucleotide encoding a recombinant molecule.
75. The engineered cell of embodiment 72 or embodiment 74, wherein the recombinant molecule is a recombinant antigen receptor, optionally wherein the recombinant antigen receptor is a recombinant T cell receptor (TCR) or is a chimeric antigen receptor (CAR).
76. The engineered cell of embodiment 75, wherein the recombinant antigen receptor is a CAR.
77. The engineered cell of any of embodiments 75-76, wherein the recombinant antigen receptor binds a tumor antigen, optionally wherein the tumor antigen is CD19 or B7H3.
78. The engineered cell of embodiment 72 or embodiment 74, wherein the recombinant molecule is a molecule that is secretable from the cell.
79. The engineered cell of embodiment 72, embodiment 74 or embodiment 78,
   wherein the recombinant molecule is a cytokine, optionally an interleukin or a functional portion thereof, optionally wherein the interleukin is IL-2, IL-12, or IL-15 or a functional portion thereof.
80. The engineered cell of embodiment 72, embodiment 74 or embodiment 78,
   wherein the recombinant molecule is a bispecific antibody, optionally wherein the bispecific antibody is a BiTE.
81. An engineered cell comprising a polynucleotide of any of embodiments 38-51.
82. The engineered cell of any of embodiments 70-81, wherein the cell is a primary cell from a subject.
83. The engineered cell of any of embodiments 70-82, wherein the engineered cell is a T cell, a tumor infiltrating lymphocyte (TIL), a B cell, a natural killer (NK) cell, or a macrophage.
84. The engineered cell of any of embodiments 70-83, wherein the engineered cell is a T cell, optionally a CD3+, CD4+, and/or CD8+ T cells.
85. The engineered cell of any of embodiments 70-84, wherein the engineered cell is a CD8+ T cell.
86. The engineered cell of any of embodiments 70-85, wherein the engineered cell is a CD4+ T cell.
87. A pharmaceutical composition comprising the engineered cell of any of embodiments 70-86.
88. The pharmaceutical composition of embodiment 87 further comprising a pharmaceutically acceptable excipient.
89. The pharmaceutical composition of embodiment 87 or embodiment 88 wherein the pharmaceutical composition is sterile.
90. A method of treatment comprising administering the engineered cell of any of embodiments 70-86 or the pharmaceutical composition of any of embodiments 87-89 to a subject that has a cancer.
91. The method of embodiment 90, wherein the engineered cells comprise an antigen receptor targeted against an antigen of the cancer.
92. The method of embodiment 90, wherein the engineered cells express a recombinant antigen receptor comprising an antigen-binding domain that binds to a tumor antigen associated with the cancer, optionally where cells of the cancer express the tumor antigen.
93. The method of embodiment 92, wherein the recombinant antigen receptor is a chimeric antigen receptor (CAR) or a T cell receptor (TCR).
94. The method of embodiment 92 or embodiment 93, wherein the recombinant antigen receptor is a CAR.
95. The method of any of embodiments 90-94, wherein the cancer is a hematologic cancer or is a solid tumor.
96. The method of any of embodiments 92-95, wherein the tumor antigen is B7H3.
97. The method of any of embodiments 92-96, wherein the cancer is a prostate cancer, melanoma, Head and neck squamous cell carcinoma (HNSCC), non-small cell lung cancer (NSCLC), urothelial cancer, ovarian cancer, neuroblastoma, rhabdomyosarcoma, osteosarcoma, Ewing sarcoma, Wilms' tumor, optionally wherein the cancer is an ovarian carcinoma or a neuroblastoma.
98. The method of any of embodiments 92-95, wherein the tumor antigen is a CD19.
99. The method of any of embodiments 90-95 and 98, wherein the cancer is a B cell cancer.
100. The method of any of embodiments 90-95, 98 and 99, wherein the cancer is a leukemia or a lymphoma, optionally metastatic lymphoma.
101. The method of any of embodiments 90-100, wherein the engineered cells express a recombinant molecule that is secreted from the cell, optionally wherein the recombinant molecule is a bispecific antibody or a cytokine.
102. Use of pharmaceutical composition of any of embodiments 87-89 for manufacture of a medicament for use in treating a subject that has a cancer.
103. A pharmaceutical composition of any of embodiments 87-89 for use in treating a subject that has a cancer.
104. The use of embodiment 102 or the pharmaceutical composition for use of embodiment 103, wherein the engineered cell of the pharmaceutical composition comprise an antigen receptor targeted against an antigen of the cancer.
105. The use or pharmaceutical composition for use of embodiment 104, wherein the engineered cells express a recombinant antigen receptor comprising an antigen-binding domain that binds to a tumor antigen associated with the cancer, optionally where cells of the cancer express the tumor antigen.
106. The use or pharmaceutical composition for use of embodiment 105, wherein the recombinant antigen receptor is a chimeric antigen receptor (CAR) or a T cell receptor (TCR).
107. The use or pharmaceutical composition for use of embodiment 105 or embodiment 106, wherein the recombinant antigen receptor is a CAR.
108. The use or pharmaceutical composition for use of any of embodiments 103-107, wherein the cancer is a hematologic cancer or is a solid tumor.
109. The use or pharmaceutical composition for use of any of embodiments 103-108, wherein the tumor antigen is B7H3.
110. The use or pharmaceutical composition for use of any of embodiments 103-109, wherein the cancer is a prostate cancer, melanoma, Head and neck squamous cell carcinoma (HNSCC), non-small cell lung cancer (NSCLC), urothelial cancer, ovarian cancer, neuroblastoma, rhabdomyosarcoma, osteosarcoma, Ewing sarcoma, Wilms' tumor, optionally wherein the cancer is an ovarian carcinoma or a neuroblastoma.
111. The use or pharmaceutical composition for use of any of embodiments 103-108, wherein the tumor antigen is a CD19.
112. The use or pharmaceutical composition for use of any of embodiments 103-108 and 111, wherein the cancer is a B cell cancer.
113. The use or pharmaceutical composition for use of any of embodiments 103-108, 111 and 112, wherein the cancer is a leukemia or a lymphoma, optionally metastatic lymphoma.
114. The use or pharmaceutical composition of any of embodiments 103-113, wherein the engineered cells express a recombinant molecule that is secreted from the cell, optionally wherein the recombinant molecule is a bispecific antibody or a cytokine.

## Claims

1. A chimeric signaling receptor comprising, from N to C- terminus:
(a) an extracellular domain that binds to an immunosuppressive cytokine selected from IL10, TGFβ, IL4, and IL1Ra;
(b) a transmembrane domain;
(c) a linker sequence; and
(d) an intracellular signaling domain comprising two MyD88 polypeptides linked in tandem,
wherein the two MyD88 polypeptides lack the full-length TIR domain.

2. The chimeric signaling receptor of claim 1, wherein the extracellular domain is or contains an extracellular domain of a naturally occurring cytokine receptor or cytokine binding fragment thereof that is able to specifically bind the immunosuppressive cytokine.

3. The chimeric signaling receptor of claim 1, wherein the extracellular domain is an antibody or an antigen binding fragment that is able to specifically bind the immunosuppressive cytokine.

4. The chimeric signaling receptor of any of claims 1-3, wherein extracellular domain is:
(i) an extracellular domain of TGFβ receptor (TGFβR) or a portion thereof that binds TGFβ;
(ii) an extracellular domain of an IL10 receptor (IL10R) or a portion thereof that binds IL10;
(iii) an extracellular domain of an IL4 receptor (IL4R) or a portion thereof that binds IL4; or
(iv) an extracellular domain that is an antibody or antigen-binding fragment that binds to IL1Ra, a scFv directed against IL1Ra, or an sdAb directed against IL1Ra.

5. The chimeric signaling receptor of any of claims 1, 2 or 4, wherein the extracellular domain:
(i) is or comprises a portion of an amino acid sequence that exhibits at least or about 85%, 90%, 92%, 95%, or 97% sequence identity to SEQ ID NO: 20 and that binds to TGFβ;
(ii) is or comprises a portion of an amino acid sequence that exhibits at least or about 85%, 90%, 92%, 95%, or 97% sequence identity to SEQ ID NO: 4 and that binds to TGFβ;
(iii) is or comprises a portion of an amino acid sequence that exhibits at least or about 85%, 90%, 92%, 95%, or 97% sequence identity to SEQ ID NO: 119 and that binds to IL10;
(iv) is or comprises a portion of an amino acid sequence that exhibits at least or about 85%, 90%, 92%, 95%, or 97% sequence identity to SEQ ID NO: 122 and that binds to IL10; or
(v) is or comprises a portion of an amino acid sequence that exhibits at least or about 85%, 90%, 92%, 95%, or 97% sequence identity to SEQ ID NO: 125 and that binds to IL4.

6. The chimeric signaling receptor of any of claims 1-5, wherein the transmembrane domain:
(i) comprises the native transmembrane domain of the TGFβR;
(ii) comprises the sequence of amino acids set forth in SEQ ID NO: 22 or a sequence of amino acids that exhibits at least or about 85%, at least or about 90%, at least or about 92%, at least or about 95%, at least or about 97% sequence identity to the sequence set forth in SEQ ID NO: 22; and/or
(iii) is a heterologous transmembrane domain from a transmembrane protein other than the TGFβR.

7. The chimeric signaling receptor of any of claims 1-6, wherein:
(a) the two MyD88 polypeptides each comprise the death domain (DD), the intermediate domain (ID) and a portion of a TIR domain; and/or
(b)
(i) the two MyD88 polypeptides each independently is a sequence of amino acids selected from the group consisting of amino acids 2-155, 2-156, 2-157, 2-158, 2-159, 2-160, 2-161, 2-162, 2-163, 2-164, 2-165, 2-166, 2-167, 2-168, 2-169, 2-170, 2-171, 2-172, 2-173, 2-174, 2-175, 2-176, 2-177, 2-178, 2-179 or 2-180 of the full-length MyD88, optionally of SEQ ID NO:128; or
(ii) wherein the two MyD88 polypeptides each independently is a sequence of amino acids 2-171 or 2-172 of the full-length MyD88, optionally of SEQ ID NO:128.

8. The chimeric signaling receptor of any of claims 1-7, wherein:
(a) the first MyD88 polypeptide is set forth in SEQ ID NO: 2;
(b) the second MyD88 polypeptide is set forth in SEQ ID NO: 2;
(c) the first MyD88 polypeptide and the second MyD88
polypeptide are identical; and/or
(d) the first MyD88 polypeptide and the second MyD88 polypeptide are connected by a peptide linker, optionally wherein the peptide linker is set forth in SEQ ID NO: 47 ((GGGGS)ₙ), where n is an integer between 1 and 4, inclusive, optionally wherein the peptide linker is selected from the group consisting of SEQ ID NO:48 (GGGGS), SEQ ID NO: 85 (GGGGSGGGGS), SEQ ID NO: 49 (GGGGSGGGGSGGGGS), and SEQ ID NO: 50 (GGGGSGGGGSGGGGSGGGGS).

9. A polynucleotide comprising a sequence of nucleotides encoding the chimeric signaling receptor of any of claims 1-8, optionally wherein:
(a) the sequence of nucleotides is a first sequence of nucleotides and the polynucleotide further comprises a second sequence of nucleotides encoding another recombinant molecule, further optionally wherein the recombinant molecule is a recombinant antigen receptor, a cytokine, or a bispecific antibody, optionally wherein:
(i) the recombinant antigen receptor is a recombinant T cell receptor (TCR) or is a chimeric antigen receptor (CAR);
(ii) the cytokine is an interleukin or a functional portion thereof, further optionally wherein the interleukin is IL-2, IL-12, or IL-15 or a functional portion thereof; or
(iii) the bispecific antibody is a bispecific T cell engager (BiTE).

10. A vector comprising the polynucleotide of claim 9.

11. A method of producing an engineered cell, the method comprising introducing the polynucleotide of claim 9 or the vector of any of claim 10 into a cell under conditions for expression of the chimeric signaling receptor on the surface of the cell, wherein the method is not a method for treatment of the human or animal body by surgery or therapy, or a diagnostic method practiced on the human or animal body.

12. An engineered cell comprising a chimeric signaling receptor of any of claims 1-8 or the polynucleotide of claim 9, optionally wherein the engineered cell is a T cell, a tumor infiltrating lymphocyte (TIL), a B cell, a natural killer (NK) cell, or a macrophage.

13. The engineered cell of claim 12, wherein the engineered cell further comprises a recombinant molecule, optionally wherein the recombinant molecule is a recombinant antigen receptor, a cytokine, or a bispecific antibody, further optionally wherein:
(a) the recombinant antigen receptor is a recombinant T cell receptor (TCR) or is a chimeric antigen receptor (CAR);
(b) the cytokine is an interleukin or a functional portion thereof, optionally wherein the interleukin is IL-2, IL-12, or IL-15 or a functional portion thereof; or
(c) the bispecific antibody is a bispecific T cell engager (BiTE).

14. A pharmaceutical composition comprising the engineered cell of claim 12 or claim 13, optionally further comprising a pharmaceutically acceptable excipient.

15. The engineered cell of any of claims 12 to 13 or the pharmaceutical composition of claim 14 for use in a method of treating cancer in a subject, said method comprising administering the engineered cell of any of claims 12 to 13 or the pharmaceutical composition of claim 14 to the subject having cancer, optionally wherein the engineered cell of the pharmaceutical composition comprises an antigen receptor targeted against an antigen of the cancer.
